# EUROPEAN PATENT APPLICATION

(11) **EP 3 556 867 A1**
(43) Date of publication of application: **23.10.2019**
(21) Application number: 19175741.8
(22) Date of filing: 19.11.2010
(51) Int. Cl.: C12Q 1/68

(54) **METHODS TO PREDICT CLINICAL OUTCOME OF CANCER**

(30) Priority: 23.11.2009 US 26376309 P
(62) Divisional of application: 10832289.2
(71) Applicant: Genomic Health, Inc., Redwood City, CA 94063 (US)
(72) Inventor: BAKER, Joffre B., Montara, CA 94037 (US); CRONIN, Maureen T., Los Altos, CA 94024 (US); COLLIN, Francois, Berkeley, CA 94702 (US); LIU, Mei-Lan, San Mateo, CA 94403 (US)
(74) Representative: Gowshall, Jonathan Vallance

(57) **Abstract**

The present invention provides methods to determine the prognosis and appropriate treatment for patients diagnosed with cancer, based on the expression levels of one or more biomarkers. More particularly, the invention relates to the identification of genes, or sets of genes, able to distinguish breast cancer patients with a good clinical prognosis from those with a bad clinical prognosis. The invention further provides methods for providing a personalized genomics report for a cancer patient. The inventions also relates to computer systems and software for data analysis using the prognostic and statistical methods disclosed herein.

## Description

### CROSS REFERENCE

This application claims the benefit of U.S. Provisional Patent Application No. 61/263,763, filed November 23, 2009, which application is incorporated herein by reference in its entirety.

### INTRODUCTION

Oncologists have a number of treatment options available to them, including different combinations of therapeutic regimens that are characterized as "standard of care." The absolute benefit from adjuvant treatment is larger for patients with poor prognostic features, and this has resulted in the policy to select only these so-called 'high-risk' patients for adjuvant chemotherapy. See, e.g., S. Paik, et al., J Clin Oncol. 24(23):3726-34 (2006). Therefore, the best likelihood of good treatment outcome requires that patients be assigned to optimal available cancer treatment, and that this assignment be made as quickly as possible following diagnosis.

Today our healthcare system is riddled with inefficiency and wasteful spending - one example of this is that the efficacy rate of many oncology therapeutics working only about 25% of the time. Many of those cancer patients are experiencing toxic side effects for costly therapies that may not be working. This imbalance between high treatment costs and low therapeutic efficacy is often a result of treating a specific diagnosis one way across a diverse patient population. But with the advent of gene profiling tools, genomic testing, and advanced diagnostics, this is beginning to change.

In particular, once a patient is diagnosed with breast cancer there is a strong need for methods that allow the physician to predict the expected course of disease, including the likelihood of cancer recurrence, long-term survival of the patient, and the like, and select the most appropriate treatment option accordingly. Accepted prognostic and predictive factors in breast cancer include age, tumor size, axillary lymph node status, histological tumor type, pathological grade and hormone receptor status. Molecular diagnostics, however, have been demonstrated to identify more patients with a low risk of breast cancer than was possible with standard prognostic indicators. S. Paik, The Oncologist 12(6):631-635 (2007).

Despite recent advances, the challenge of breast cancer treatment remains to target specific treatment regimens to pathogenically distinct tumor types, and ultimately personalize tumor treatment in order to maximize outcome. Accurate prediction of prognosis and clinical outcome would allow the oncologist to tailor the administration of adjuvant chemotherapy such that women with a higher risk of a recurrence or poor prognosis would receive more aggressive treatment. Furthermore, accurately stratifying patients based on risk would greatly advance the understanding of expected absolute benefit from treatment, thereby increasing success rates for clinical trials for new breast cancer therapies.

Currently, most diagnostic tests used in clinical practice are frequently not quantitative, relying on immunohistochemistry (IHC). This method often yields different results in different laboratories, in part because the reagents are not standardized, and in part because the interpretations are subjective and cannot be easily quantified. Other RNA-based molecular diagnostics require fresh-frozen tissues, which presents a myriad of challenges including incompatibilities with current clinical practices and sample transport regulations. Fixed paraffin-embedded tissue is more readily available and methods have been established to detect RNA in fixed tissue. However, these methods typically do not allow for the study of large numbers of genes (DNA or RNA) from small amounts of material. Thus, traditionally fixed tissue has been rarely used other than for IHC detection of proteins.

### SUMMARY

The present invention provides a set of genes, the expression levels of which are associated with a particular clinical outcome in cancer. For example, the clinical outcome could be a good or bad prognosis assuming the patient receives the standard of care. The clinical outcome may be defined by clinical endpoints, such as disease or recurrence free survival, metastasis free survival, overall survival, etc.

The present invention accommodates the use of archived paraffin-embedded biopsy material for assay of all markers in the set, and therefore is compatible with the most widely available type of biopsy material. It is also compatible with several different methods of tumor tissue harvest, for example, via core biopsy or fine needle aspiration. The tissue sample may comprise cancer cells.

In one aspect, the present invention concerns a method of predicting a clinical outcome of a cancer patient, comprising (a) obtaining an expression level of an expression product (e.g., an RNA transcript) of at least one prognostic gene listed in Tables 1-12 from a tissue sample obtained from a tumor of the patient; (b) normalizing the expression level of the expression product of the at least one prognostic gene, to obtain a normalized expression level; and (c) calculating a risk score based on the normalized expression value, wherein increased expression of prognostic genes in Tables 1, 3, 5, and 7 are positively correlated with good prognosis, and wherein increased expression of prognostic genes in Tables 2, 4, 6, and 8 are negatively associated with good prognosis. In some embodiments, the tumor is estrogen receptor-positive. In other embodiments, the tumor is estrogen receptor negative.

In one aspect, the present disclosure provides a method of predicting a clinical outcome of a cancer patient, comprising (a) obtaining an expression level of an expression product (e.g., an RNA transcript) of at least one prognostic gene from a tissue sample obtained from a tumor of the patient, where the at least one prognostic gene is selected from GSTM2, IL6ST, GSTM3, C8orf4, TNFRSF11B, NAT1, RUNX1, CSF1, ACTR2, LMNB1, TFRC, LAPTM4B, ENO1, CDC20, and IDH2; (b) normalizing the expression level of the expression product of the at least one prognostic gene, to obtain a normalized expression level; and (c) calculating a risk score based on the normalized expression value, wherein increased expression of a prognostic gene selected from GSTM2, IL6ST, GSTM3, C8orf4, TNFRSF11B, NAT1, RUNX1, and CSF1 is positively correlated with good prognosis, and wherein increased expression of a prognostic gene selected from ACTR2, LMNB1, TFRC, LAPTM4B, ENO1, CDC20, and IDH2 is negatively associated with good prognosis. In some embodiments, the tumor is estrogen receptor-positive. In other embodiments, the tumor is estrogen receptor negative.

In various embodiments, the normalized expression level of at least 2, or at least 5, or at least 10, or at least 15, or at least 20, or a least 25 prognostic genes (as determined by assaying a level of an expression product of the gene) is determined. In alternative embodiments, the normalized expression levels of at least one of the genes that co-expresses with prognostic genes in Tables 16-18 is obtained.

In another embodiment, the risk score is determined using normalized expression levels of at least one a stromal or transferrin receptor group gene, or a gene that co-expresses with a stromal or transferrin receptor group gene.

In another embodiment, the cancer is breast cancer. In another embodiment, the patient is a human patient.

In yet another embodiment, the cancer is ER-positive breast cancer.

In yet another embodiment, the cancer is ER-negative breast cancer.

In a further embodiment, the expression product comprises RNA. For example, the RNA could be exonic RNA, intronic RNA, or short RNA (e.g., microRNA, siRNA, promoter-associated small RNA, shRNA, etc.). In various embodiments, the RNA is fragmented RNA.

In a different aspect, the invention concerns an array comprising polynucleotides hybridizing to an RNA transcription of at least one of the prognostic genes listed in Tables 1-12.

In a still further aspect, the invention concerns a method of preparing a personalized genomics profile for a patient, comprising (a) obtaining an expression level of an expression product (e.g., an RNA transcript) of at least one prognostic gene listed in Tables 1-12 from a tissue sample obtained from a tumor of the patient; (b) normalizing the expression level of the expression product of the at least one prognostic gene to obtain a normalized expression level; and (c) calculating a risk score based on the normalized expression value, wherein increased expression of prognostic genes in Tables 1, 3, 5, and 7 are positively correlated with good prognosis, and wherein increased expression of prognostic genes in Tables 2, 4, 6, and 8 are negatively associated with good prognosis. In some embodiments, the tumor is estrogen receptor-positive, and in other embodiments the tumor is estrogen receptor negative.

In various embodiments, a subject method can further include providing a report. The report may include prediction of the likelihood of risk that said patient will have a particular clinical outcome.

The invention further provides a computer-implemented method for classifying a cancer patient based on risk of cancer recurrence, comprising (a) classifying, on a computer, said patient as having a good prognosis or a poor prognosis based on an expression profile comprising measurements of expression levels of expression products of a plurality of prognostic genes in a tumor tissue sample taken from the patient, said plurality of genes comprising at least three different prognostic genes listed in any of Tables 1-12, wherein a good prognosis predicts no recurrence or metastasis within a predetermined period after initial diagnosis, and wherein a poor prognosis predicts recurrence or metastasis within said predetermined period after initial diagnosis; and (b) calculating a risk score based on said expression levels.

### DETAILED DESCRIPTION

### DEFINITIONS

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

"Prognostic factors" are those variables related to the natural history of cancer, which influence the recurrence rates and outcome of patients once they have developed cancer. Clinical parameters that have been associated with a worse prognosis include, for example, lymph node involvement, and high grade tumors. Prognostic factors are frequently used to categorize patients into subgroups with different baseline relapse risks.

The term "prognosis" is used herein to refer to the prediction of the likelihood of cancer-attributable death or progression, including recurrence, metastatic spread, and drug resistance, of a neoplastic disease, such as breast cancer. The term "good prognosis" means a desired or "positive" clinical outcome. For example, in the context of breast cancer, a good prognosis may be an expectation of no recurrences or metastasis within two, three, four, five or more years of the initial diagnosis of breast cancer. The terms "bad prognosis" or "poor prognosis" are used herein interchangeably herein to mean an undesired clinical outcome. For example, in the context of breast cancer, a bad prognosis may be an expectation of a recurrence or metastasis within two, three, four, five or more years of the initial diagnosis of breast cancer.

The term "prognostic gene" is used herein to refer to a gene, the expression of which is correlated, positively or negatively, with a good prognosis for a cancer patient treated with the standard of care. A gene may be both a prognostic and predictive gene, depending on the correlation of the gene expression level with the corresponding endpoint. For example, using a Cox proportional hazards model, if a gene is only prognostic, its hazard ratio (HR) does not change when measured in patients treated with the standard of care or in patients treated with a new intervention.

The term "predictive gene" is used herein to refer to a gene, the expression of which is correlated, positively or negatively, with response to a beneficial response to treatment. For example, treatment could include chemotherapy.

The terms "risk score" or "risk classification" are used interchangeably herein to describe a level of risk (or likelihood) that a patient will experience a particular clinical outcome. A patient may be classified into a risk group or classified at a level of risk based on the methods of the present disclosure, e.g. high, medium, or low risk. A "risk group" is a group of subjects or individuals with a similar level of risk for a particular clinical outcome.

A clinical outcome can be defined using different endpoints. The term "long-term" survival is used herein to refer to survival for a particular time period, e.g., for at least 3 years, more preferably for at least 5 years. The term "Recurrence-Free Survival" (RFS) is used herein to refer to survival for a time period (usually in years) from randomization to first cancer recurrence or death due to recurrence of cancer. The term "Overall Survival" (OS) is used herein to refer to the time (in years) from randomization to death from any cause. The term "Disease-Free Survival" (DFS) is used herein to refer to survival for a time period (usually in years) from randomization to first cancer recurrence or death from any cause.

The calculation of the measures listed above in practice may vary from study to study depending on the definition of events to be either censored or not considered.

The term "biomarker" as used herein refers to a gene, the expression level of which, as measured using a gene product.

The term "microarray" refers to an ordered arrangement of hybridizable array elements, preferably polynucleotide probes, on a substrate.

As used herein, the term "normalized expression level" as applied to a gene refers to the normalized level of a gene product, e.g. the normalized value determined for the RNA expression level of a gene or for the polypeptide expression level of a gene.

The term "Cₜ" as used herein refers to threshold cycle, the cycle number in quantitative polymerase chain reaction (qPCR) at which the fluorescence generated within a reaction well exceeds the defined threshold, i.e. the point during the reaction at which a sufficient number of amplicons have accumulated to meet the defined threshold.

The term "gene product" or "expression product" are used herein to refer to the RNA transcription products (transcripts) of the gene, including mRNA, and the polypeptide translation products of such RNA transcripts. A gene product can be, for example, an unspliced RNA, an mRNA, a splice variant mRNA, a microRNA, a fragmented RNA, a polypeptide, a post-translationally modified polypeptide, a splice variant polypeptide, etc.

The term "RNA transcript" as used herein refers to the RNA transcription products of a gene, including, for example, mRNA, an unspliced RNA, a splice variant mRNA, a microRNA, and a fragmented RNA. "Fragmented RNA" as used herein refers to RNA a mixture of intact RNA and RNA that has been degraded as a result of the sample processing (e.g., fixation, slicing tissue blocks, etc.).

Unless indicated otherwise, each gene name used herein corresponds to the Official Symbol assigned to the gene and provided by Entrez Gene (URL: www.ncbi.nlm.nih.gov/sites/entrez) as of the filing date of this application.

The terms "correlated" and "associated" are used interchangeably herein to refer to a strength of association between two measurements (or measured entities). The disclosure provides genes and gene subsets, the expression levels of which are associated with a particular outcome measure. For example, the increased expression level of a gene may be positively correlated (positively associated) with an increased likelihood of good clinical outcome for the patient, such as an increased likelihood of long-term survival without recurrence of the cancer and/or metastasis-free survival. Such a positive correlation may be demonstrated statistically in various ways, e.g. by a low hazard ratio (e.g. HR < 1.0). In another example, the increased expression level of a gene may be negatively correlated (negatively associated) with an increased likelihood of good clinical outcome for the patient. In that case, for example, the patient may have a decreased likelihood of long-term survival without recurrence of the cancer and/or cancer metastasis, and the like. Such a negative correlation indicates that the patient likely has a poor prognosis, e.g., a high hazard ratio (e.g., HR > 1.0). "Correlated" is also used herein to refer to a strength of association between the expression levels of two different genes, such that expression level of a first gene can be substituted with an expression level of a second gene in a given algorithm in view of their correlation of expression. Such "correlated expression" of two genes that are substitutable in an algorithm usually gene expression levels that are positively correlated with one another, e.g., if increased expression of a first gene is positively correlated with an outcome (e.g., increased likelihood of good clinical outcome), then the second gene that is co-expressed and exhibits correlated expression with the first gene is also positively correlated with the same outcome

The term "recurrence," as used herein, refers to local or distant (metastasis) recurrence of cancer. For example, breast cancer can come back as a local recurrence (in the treated breast or near the tumor surgical site) or as a distant recurrence in the body. The most common sites of breast cancer recurrence include the lymph nodes, bones, liver, or lungs.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including *in vitro* recombinant DNA-mediated techniques and by expression of DNAs in cells and organisms.

The phrase "amplification" refers to a process by which multiple copies of a gene or RNA transcript are formed in a particular sample or cell line. The duplicated region (a stretch of amplified polynucleotide) is often referred to as "amplicon." Usually, the amount of the messenger RNA (mRNA) produced, *i.e.,* the level of gene expression, also increases in the proportion of the number of copies made of the particular gene expressed.

The term "estrogen receptor (ER)" designates the estrogen receptor status of a cancer patient. A tumor is ER-positive if there is a significant number of estrogen receptors present in the cancer cells, while ER-negative indicates that the cells do not have a significant number of receptors present. The definition of "significant" varies amongst testing sites and methods (e.g., immunohistochemistry, PCR). The ER status of a cancer patient can be evaluated by various known means. For example, the ER level of breast cancer is determined by measuring an expression level of a gene encoding the estrogen receptor in a breast tumor sample obtained from a patient.

The term "tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include, but are not limited to, breast cancer, ovarian cancer, colon cancer, lung cancer, prostate cancer, hepatocellular cancer, gastric cancer, pancreatic cancer, cervical cancer, liver cancer, bladder cancer, cancer of the urinary tract, thyroid cancer, renal cancer, carcinoma, melanoma, and brain cancer.

The gene subset identified herein as the "stromal group" includes genes that are synthesized predominantly by stromal cells and are involved in stromal response and genes that co-express with stromal group genes. "Stromal cells" are defined herein as connective tissue cells that make up the support structure of biological tissues. Stromal cells include fibroblasts, immune cells, pericytes, endothelial cells, and inflammatory cells. "Stromal response" refers to a desmoplastic response of the host tissues at the site of a primary tumor or invasion. See, e.g., E. Rubin, J. Farber, Pathology, 985-986 (2nd Ed. 1994). The stromal group includes, for example, CDH11, TAGLN, ITGA4, INHBA, COLIA1, COLIA2, FN1, CXCL14, TNFRSF1, CXCL12, C10ORF116, RUNX1, GSTM2, TGFB3, CAV1, DLC1, TNFRSF10, F3, and DICER1, and co-expressed genes identified in Tables 16-18.

The gene subset identified herein as the "metabolic group" includes genes that are associated with cellular metabolism, including genes associated with carrying proteins for transferring iron, the cellular iron homeostasis pathway, and homeostatic biochemical metabolic pathways, and genes that co-express with metabolic group genes. The metabolic group includes, for example, TFRC, ENO1, IDH2, ARF1, CLDN4, PRDX1, and GBP1, and co-expressed genes identified in Tables 16-18.

The gene subset identified herein as the "immune group" includes genes that are involved in cellular immunoregulatory functions, such as T and B cell trafficking, lymphocyte-associated or lymphocyte markers, and interferon regulation genes, and genes that co-express with immune group genes. The immune group includes, for example, CCL19 and IRF1, and co-expressed genes identified in Tables 16-18.

The gene subset identified herein as the "proliferation group" includes genes that are associated with cellular development and division, cell cycle and mitotic regulation, angiogenesis, cell replication, nuclear transport/stability, wnt signaling, apoptosis, and genes that co-express with proliferation group genes. The proliferation group includes, for example, PGF, SPC25, AURKA, BIRC5, BUB1, CCNB1, CENPA, KPNA, LMNB1, MCM2, MELK, NDC80, TPX2M, and WISP1, and co-expressed genes identified in Tables 16-18.

The term "co-expressed", as used herein, refers to a statistical correlation between the expression level of one gene and the expression level of another gene. Pairwise co-expression may be calculated by various methods known in the art, e.g., by calculating Pearson correlation coefficients or Spearman correlation coefficients. Co-expressed gene cliques may also be identified using a graph theory.

As used herein, the terms "gene clique" and "clique" refer to a subgraph of a graph in which every vertex is connected by an edge to every other vertex of the subgraph.

As used herein, a "maximal clique" is a clique in which no other vertex can be added and still be a clique.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

A "computer-based system" refers to a system of hardware, software, and data storage medium used to analyze information. The minimum hardware of a patient computer-based system comprises a central processing unit (CPU), and hardware for data input, data output (e.g., display), and data storage. An ordinarily skilled artisan can readily appreciate that any currently available computer-based systems and/or components thereof are suitable for use in connection with the methods of the present disclosure. The data storage medium may comprise any manufacture comprising a recording of the present information as described above, or a memory access device that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" or "computing means" references any hardware and/or software combination that will perform the functions required of it. For example, a suitable processor may be a programmable digital microprocessor such as available in the form of an electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

As used herein, "graph theory" refers to a field of study in Computer Science and Mathematics in which situations are represented by a diagram containing a set of points and lines connecting some of those points. The diagram is referred to as a "graph", and the points and lines referred to as "vertices" and "edges" of the graph. In terms of gene co-expression analysis, a gene (or its equivalent identifier, e.g. an array probe) may be represented as a node or vertex in the graph. If the measures of similarity (e.g., correlation coefficient, mutual information, and alternating conditional expectation) between two genes are higher than a significant threshold, the two genes are said to be co-expressed and an edge will be drawn in the graph. When co-expressed edges for all possible gene pairs for a given study have been drawn, all maximal cliques are computed. The resulting maximal clique is defined as a gene clique. A gene clique is a computed co-expressed gene group that meets predefined criteria.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

The term "node negative" cancer, such as "node negative" breast cancer, is used herein to refer to cancer that has not spread to the lymph nodes.

The terms "splicing" and "RNA splicing" are used interchangeably and refer to RNA processing that removes introns and joins exons to produce mature mRNA with continuous coding sequence that moves into the cytoplasm of a eukaryotic cell.

In theory, the term "exon" refers to any segment of an interrupted gene that is represented in the mature RNA product (B. Lewin. Genes IV Cell Press, Cambridge Mass. 1990). In theory the term "intron" refers to any segment of DNA that is transcribed but removed from within the transcript by splicing together the exons on either side of it. Operationally, exon sequences occur in the mRNA sequence of a gene as defined by Ref. SEQ ID numbers. Operationally, intron sequences are the intervening sequences within the genomic DNA of a gene, bracketed by exon sequences and having GT and AG splice consensus sequences at their 5' and 3' boundaries.

### GENE EXPRESSION ASSAY

The present disclosure provides methods that employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

### 1. Gene Expression Profiling

Methods of gene expression profiling include methods based on hybridization analysis of polynucleotides, methods based on sequencing of polynucleotides, and proteomics-based methods. The most commonly used methods known in the art for the quantification of mRNA expression in a sample include northern blotting and in situ hybridization (Parker & Barnes, Methods in Molecular Biology 106:247-283 (1999)); RNAse protection assays (Hod, Biotechniques 13:852-854 (1992)); and PCR-based methods, such as reverse transcription polymerase chain reaction (RT-PCR) (Weis et al., Trends in Genetics 8:263-264 (1992)). Alternatively, antibodies may be employed that can recognize specific duplexes, including DNA duplexes, RNA duplexes, and DNA-RNA hybrid duplexes or DNA-protein duplexes.

### 2. PCR-based Gene Expression Profiling Methods

### a. Reverse Transcriptase PCR (RT-PCR)

Of the techniques listed above, the most sensitive and most flexible quantitative method is RT-PCR, which can be used to compare mRNA levels in different sample populations, in normal and tumor tissues, with or without drug treatment, to characterize patterns of gene expression, to discriminate between closely related mRNAs, and to analyze RNA structure.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, etc., tumor, or tumor cell lines, with pooled DNA from healthy donors. If the source of mRNA is a primary tumor, mRNA can be extracted, for example, from frozen or archived paraffin-embedded and fixed (e.g. formalin-fixed) tissue samples.

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

In some cases, it may be appropriate to amplify RNA prior to initiating expression profiling. It is often the case that only very limited amounts of valuable clinical specimens are available for molecular analysis. This may be due to the fact that the tissues have already be used for other laboratory analyses or may be due to the fact that the original specimen is very small as in the case of needle biopsy or very small primary tumors. When tissue is limiting in quantity it is generally also the case that only small amounts of total RNA can be recovered from the specimen and as a result only a limited number of genomic markers can be analyzed in the specimen. RNA amplification compensates for this limitation by faithfully reproducing the original RNA sample as a much larger amount of RNA of the same relative composition. Using this amplified copy of the original RNA specimen, unlimited genomic analysis can be done to discovery biomarkers associated with the clinical characteristics of the original biological sample. This effectively immortalizes clinical study specimens for the purposes of genomic analysis and biomarker discovery.

As RNA cannot serve as a template for PCR, the first step in gene expression profiling by real-time RT-PCR (RT-PCR) is the reverse transcription of the RNA template into cDNA, followed by its exponential amplification in a PCR reaction. The two most commonly used reverse transcriptases are avian myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction. For further details see, e.g. Held et al., Genome Research 6:986-994 (1996).

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7900® Sequence Detection System™ (Perkin-Elmer-Applied Biosystems, Foster City, CA, USA), or LightCycler® 480 Real-Time PCR System (Roche Diagnostics, GmbH, Penzberg, Germany). In a preferred embodiment, the 5' nuclease procedure is run on a real-time quantitative PCR device such as the ABI PRISM 7900® Sequence Detection System™. The system consists of a thermocycler, laser, charge-coupled device (CCD), camera and computer. The system amplifies samples in a 384-well format on a thermocycler. During amplification, laser-induced fluorescent signal is collected in real-time through fiber optics cables for all 384 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Cₜ).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin.

The steps of a representative protocol for profiling gene expression using fixed, paraffin-embedded tissues as the RNA source, including mRNA isolation, purification, primer extension and amplification are given in various published journal articles. M. Cronin, Am J Pathol 164(1):35-42 (2004). Briefly, a representative process starts with cutting about 10 µm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific primers followed by RT-PCR.

### b. Design of Intron-Based PCR Primers and Probes

PCR primers and probes can be designed based upon exon or intron sequences present in the mRNA transcript of the gene of interest. Prior to carrying out primer/probe design, it is necessary to map the target gene sequence to the human genome assembly in order to identify intron-exon boundaries and overall gene structure. This can be performed using publicly available software, such as Primer3 (Whitehead Inst.) and Primer Express® (Applied Biosystems).

Where necessary or desired, repetitive sequences of the target sequence can be masked to mitigate non-specific signals. Exemplary tools to accomplish this include the Repeat Masker program available on-line through the Baylor College of Medicine, which screens DNA sequences against a library of repetitive elements and returns a query sequence in which the repetitive elements are masked. The masked intron and exon sequences can then be used to design primer and probe sequences for the desired target sites using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Rrawetz S, Misener S (eds) Bioinformatics Methods and Protocols: Methods in Molecular Biology. Humana Press, Totowa, NJ, pp 365-386).

Other factors that can influence PCR primer design include primer length, melting temperature (Tm), and G/C content, specificity, complementary primer sequences, and 3 '-end sequence. In general, optimal PCR primers are generally 17-30 bases in length, and contain about 20-80%, such as, for example, about 50-60% G+C bases, and exhibit Tm's between 50 and 80 °C, e.g. about 50 to 70 °C.

For further guidelines for PCR primer and probe design see, e.g. Dieffenbach, CW. et al, "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997), the entire disclosures of which are hereby expressly incorporated by reference.

Table A provides further information concerning the primer, probe, and amplicon sequences associated with the Examples disclosed herein.

### c. MassARRA Y System

In the MassARRAY-based gene expression profiling method, developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is preloaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

### d. Other PCR-based Methods

Further PCR-based techniques include, for example, differential display (Liang and Pardee, Science 257:967-971 (1992)); amplified fragment length polymorphism (iAFLP) (Kawamoto et al., Genome Res. 12:1305-1312 (1999)); BeadArray™ technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex100 LabMAP system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003)).

### 3. Microarrays

Differential gene expression can also be identified, or confirmed using the microarray technique. Thus, the expression profile of breast cancer-associated genes can be measured in either fresh or paraffin-embedded tumor tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from human tumors or tumor cell lines, and corresponding normal tissues or cell lines. Thus RNA can be isolated from a variety of primary tumors or tumor cell lines. If the source of mRNA is a primary tumor, mRNA can be extracted, for example, from frozen or archived paraffin-embedded and fixed (e.g. formalin-fixed) tissue samples, which are routinely prepared and preserved in everyday clinical practice.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately twofold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Agilent's microarray technology.

The development of microarray methods for large-scale analysis of gene expression makes it possible to search systematically for molecular markers of cancer classification and outcome prediction in a variety of tumor types.

### 4. Gene Expression Analysis by Nucleic Acid Sequencing

Nucleic acid sequencing technologies are suitable methods for analysis of gene expression. The principle underlying these methods is that the number of times a cDNA sequence is detected in a sample is directly related to the relative expression of the mRNA corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). See, e.g., S. Brenner, et al., Nature Biotechnology 18(6):630-634 (2000). More recently, the advent of "next-generation" sequencing technologies has made DGE simpler, higher throughput, and more affordable. As a result, more laboratories are able to utilize DGE to screen the expression of more genes in more individual patient samples than previously possible. See, e.g., J. Marioni, Genome Research 18(9):1509-1517 (2008); R. Morin, Genome Research 18(4):610-621 (2008); A. Mortazavi, Nature Methods 5(7):621-628 (2008); N. Cloonan, Nature Methods 5(7):613-619 (2008).

### 5. Isolating RNA from Body Fluids

Methods of isolating RNA for expression analysis from blood, plasma and serum (See for example, Tsui NB et al. (2002) 48,1647-53 and references cited therein) and from urine (See for example, Boom R et al. (1990) J Clin Microbiol. 28, 495-503 and reference cited therein) have been described.

### 6. Immunohistochemistry

Immunohistochemistry methods are also suitable for detecting the expression levels of the prognostic markers of the present invention. Thus, antibodies or antisera, preferably polyclonal antisera, and most preferably monoclonal antibodies specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

### 7. Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps:
(1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

### 8. General Description of the mRNA Isolation, Purification, and Amplification

The steps of a representative protocol for profiling gene expression using fixed, paraffin-embedded tissues as the RNA source, including mRNA isolation, purification, primer extension and amplification are provided in various published journal articles (for example: T.E. Godfrey et al,. J. Molec. Diagnostics 2: 84-91 [2000]; K. Specht et al., Am. J. Pathol. 158: 419-29 [2001]). Briefly, a representative process starts with cutting about 10 µm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific primers followed by RT-PCR. Finally, the data are analyzed to identify the best treatment option(s) available to the patient on the basis of the characteristic gene expression pattern identified in the tumor sample examined, dependent on the predicted likelihood of cancer recurrence.

### 9. Normalization

The expression data used in the methods disclosed herein can be normalized. Normalization refers to a process to correct for (normalize away), for example, differences in the amount of RNA assayed and variability in the quality of the RNA used, to remove unwanted sources of systematic variation in Cₜ measurements, and the like. With respect to RT-PCR experiments involving archived fixed paraffin embedded tissue samples, sources of systematic variation are known to include the degree of RNA degradation relative to the age of the patient sample and the type of fixative used to preserve the sample. Other sources of systematic variation are attributable to laboratory processing conditions.

Assays can provide for normalization by incorporating the expression of certain normalizing genes, which genes do not significantly differ in expression levels under the relevant conditions. Exemplary normalization genes include housekeeping genes such as PGK1 and UBB. (See, e.g., E. Eisenberg, et al., Trends in Genetics 19(7):362-365 (2003).) Normalization can be based on the mean or median signal (C_{T}) of all of the assayed genes or a large subset thereof (global normalization approach). In general, the normalizing genes, also referred to as reference genes should be genes that are known not to exhibit significantly different expression in colorectal cancer as compared to non-cancerous colorectal tissue, and are not significantly affected by various sample and process conditions, thus provide for normalizing away extraneous effects.

Unless noted otherwise, normalized expression levels for each mRNA/tested tumor/patient will be expressed as a percentage of the expression level measured in the reference set. A reference set of a sufficiently high number (e.g. 40) of tumors yields a distribution of normalized levels of each mRNA species. The level measured in a particular tumor sample to be analyzed falls at some percentile within this range, which can be determined by methods well known in the art.

In exemplary embodiments, one or more of the following genes are used as references by which the expression data is normalized: AAMP, ARF1, EEF1A1, ESD, GPS1, H3F3A, HNRPC, RPL13A, RPL41, RPS23, RPS27, SDHA, TCEA1, UBB, YWHAZ, B-actin, GUS, GAPDH, RPLPO, and TFRC. For example, the calibrated weighted average Cₜ measurements for each of the prognostic genes may be normalized relative to the mean of at least three reference genes, at least four reference genes, or at least five reference genes.

Those skilled in the art will recognize that normalization may be achieved in numerous ways, and the techniques described above are intended only to be exemplary, not exhaustive.

### REPORTING RESULTS

The methods of the present disclosure are suited for the preparation of reports summarizing the expected or predicted clinical outcome resulting from the methods of the present disclosure. A "report," as described herein, is an electronic or tangible document that includes report elements that provide information of interest relating to a likelihood assessment or a risk assessment and its results. A subject report includes at least a likelihood assessment or a risk assessment, e.g., an indication as to the risk of recurrence of breast cancer, including local recurrence and metastasis of breast cancer. A subject report can include an assessment or estimate of one or more of disease-free survival, recurrence-free survival, metastasis-free survival, and overall survival. A subject report can be completely or partially electronically generated, e.g., presented on an electronic display (e.g., computer monitor). A report can further include one or more of: 1) information regarding the testing facility; 2) service provider information; 3) patient data; 4) sample data; 5) an interpretive report, which can include various information including: a) indication; b) test data, where test data can include a normalized level of one or more genes of interest, and 6) other features.

The present disclosure thus provides for methods of creating reports and the reports resulting therefrom. The report may include a summary of the expression levels of the RNA transcripts, or the expression products of such RNA transcripts, for certain genes in the cells obtained from the patient's tumor. The report can include information relating to prognostic covariates of the patient. The report may include an estimate that the patient has an increased risk of recurrence. That estimate may be in the form of a score or patient stratifier scheme (e.g., low, intermediate, or high risk of recurrence). The report may include information relevant to assist with decisions about the appropriate surgery (e.g., partial or total mastectomy) or treatment for the patient.

Thus, in some embodiments, the methods of the present disclosure further include generating a report that includes information regarding the patient's likely clinical outcome, e.g. risk of recurrence. For example, the methods disclosed herein can further include a step of generating or outputting a report providing the results of a subject risk assessment, which report can be provided in the form of an electronic medium (e.g., an electronic display on a computer monitor), or in the form of a tangible medium (e.g., a report printed on paper or other tangible medium).

A report that includes information regarding the patient's likely prognosis (e.g., the likelihood that a patient having breast cancer will have a good prognosis or positive clinical outcome in response to surgery and/or treatment) is provided to a user. An assessment as to the likelihood is referred to below as a "risk report" or, simply, "risk score." A person or entity that prepares a report ("report generator") may also perform the likelihood assessment. The report generator may also perform one or more of sample gathering, sample processing, and data generation, e.g., the report generator may also perform one or more of: a) sample gathering; b) sample processing; c) measuring a level of a risk gene; d) measuring a level of a reference gene; and e) determining a normalized level of a risk gene. Alternatively, an entity other than the report generator can perform one or more sample gathering, sample processing, and data generation.

For clarity, it should be noted that the term "user," which is used interchangeably with "client," is meant to refer to a person or entity to whom a report is transmitted, and may be the same person or entity who does one or more of the following: a) collects a sample; b) processes a sample; c) provides a sample or a processed sample; and d) generates data (e.g., level of a risk gene; level of a reference gene product(s); normalized level of a risk gene ("prognosis gene") for use in the likelihood assessment. In some cases, the person(s) or entity(ies) who provides sample collection and/or sample processing and/or data generation, and the person who receives the results and/or report may be different persons, but are both referred to as "users" or "clients" herein to avoid confusion. In certain embodiments, e.g., where the methods are completely executed on a single computer, the user or client provides for data input and review of data output. A "user" can be a health professional (e.g., a clinician, a laboratory technician, a physician (e.g., an oncologist, surgeon, pathologist), etc.).

In embodiments where the user only executes a portion of the method, the individual who, after computerized data processing according to the methods of the present disclosure, reviews data output (e.g., results prior to release to provide a complete report, a complete, or reviews an "incomplete" report and provides for manual intervention and completion of an interpretive report) is referred to herein as a "reviewer." The reviewer may be located at a location remote to the user (e.g., at a service provided separate from a healthcare facility where a user may be located).

Where government regulations or other restrictions apply (e.g., requirements by health, malpractice, or liability insurance), all results, whether generated wholly or partially electronically, are subjected to a quality control routine prior to release to the user.

### CLINICAL UTILITY

The gene expression assay and information provided by the practice of the methods disclosed herein facilitates physicians in making more well-informed treatment decisions, and to customize the treatment of cancer to the needs of individual patients, thereby maximizing the benefit of treatment and minimizing the exposure of patients to unnecessary treatments which may provide little or no significant benefits and often carry serious risks due to toxic side-effects.

Single or multi-analyte gene expression tests can be used measure the expression level of one or more genes involved in each of several relevant physiologic processes or component cellular characteristics. The expression level(s) may be used to calculate such a quantitative score, and such score may be arranged in subgroups (e.g., tertiles) wherein all patients in a given range are classified as belonging to a risk category (e.g., low, intermediate, or high). The grouping of genes may be performed at least in part based on knowledge of the contribution of the genes according to physiologic functions or component cellular characteristics, such as in the groups discussed above.

The utility of a gene marker in predicting cancer may not be unique to that marker. An alternative marker having an expression pattern that is parallel to that of a selected marker gene may be substituted for, or used in addition to, a test marker. Due to the co-expression of such genes, substitution of expression level values should have little impact on the overall prognostic utility of the test. The closely similar expression patterns of two genes may result from involvement of both genes in the same process and/or being under common regulatory control in colon tumor cells. The present disclosure thus contemplates the use of such co-expressed genes or gene sets as substitutes for, or in addition to, prognostic methods of the present disclosure.

The molecular assay and associated information provided by the methods disclosed herein for predicting the clinical outcome in cancer, e.g. breast cancer, have utility in many areas, including in the development and appropriate use of drugs to treat cancer, to stratify cancer patients for inclusion in (or exclusion from) clinical studies, to assist patients and physicians in making treatment decisions, provide economic benefits by targeting treatment based on personalized genomic profile, and the like. For example, the recurrence score may be used on samples collected from patients in a clinical trial and the results of the test used in conjunction with patient outcomes in order to determine whether subgroups of patients are more or less likely to demonstrate an absolute benefit from a new drug than the whole group or other subgroups. Further, such methods can be used to identify from clinical data subsets of patients who are expected to benefit from adjuvant therapy. Additionally, a patient is more likely to be included in a clinical trial if the results of the test indicate a higher likelihood that the patient will have a poor clinical outcome if treated with surgery alone and a patient is less likely to be included in a clinical trial if the results of the test indicate a lower likelihood that the patient will have a poor clinical outcome if treated with surgery alone.

### STATISTICAL ANALYSIS OF GENE EXPRESSION LEVELS

One skilled in the art will recognize that there are many statistical methods that may be used to determine whether there is a significant relationship between an outcome of interest (e.g., likelihood of survival, likelihood of response to chemotherapy) and expression levels of a marker gene as described here. This relationship can be presented as a continuous recurrence score (RS), or patients may stratified into risk groups (e.g., low, intermediate, high). For example, a Cox proportional hazards regression model may fit to a particular clinical endpoint (e.g., RFS, DFS, OS). One assumption of the Cox proportional hazards regression model is the proportional hazards assumption, i.e. the assumption that effect parameters multiply the underlying hazard.

### Coexpression Analysis

The present disclosure provides genes that co-express with particular prognostic and/or predictive gene that has been identified as having a significant correlation to recurrence and/or treatment benefit. To perform particular biological processes, genes often work together in a concerted way, i.e. they are co-expressed. Co-expressed gene groups identified for a disease process like cancer can serve as biomarkers for disease progression and response to treatment. Such co-expressed genes can be assayed in lieu of, or in addition to, assaying of the prognostic and/or predictive gene with which they are co-expressed.

One skilled in the art will recognize that many co-expression analysis methods now known or later developed will fall within the scope and spirit of the present invention. These methods may incorporate, for example, correlation coefficients, co-expression network analysis, clique analysis, etc., and may be based on expression data from RT-PCR, microarrays, sequencing, and other similar technologies. For example, gene expression clusters can be identified using pair-wise analysis of correlation based on Pearson or Spearman correlation coefficients. (See, e.g., Pearson K. and Lee A., Biometrika 2, 357 (1902); C. Spearman, Amer. J. Psychol 15:72-101 (1904); J. Myers, A. Well, Research Design and Statistical Analysis, p. 508 (2nd Ed., 2003).) In general, a correlation coefficient of equal to or greater than 0.3 is considered to be statistically significant in a sample size of at least 20. (See, e.g., G. Norman, D. Streiner, Biostatistics: The Bare Essentials, 137-138 (3rd Ed. 2007).) In one embodiment disclosed herein, co-expressed genes were identified using a Spearman correlation value of at least 0.7.

### Computer program

The values from the assays described above, such as expression data, recurrence score, treatment score and/or benefit score, can be calculated and stored manually. Alternatively, the above-described steps can be completely or partially performed by a computer program product. The present invention thus provides a computer program product including a computer readable storage medium having a computer program stored on it. The program can, when read by a computer, execute relevant calculations based on values obtained from analysis of one or more biological sample from an individual (e.g., gene expression levels, normalization, thresholding, and conversion of values from assays to a score and/or graphical depiction of likelihood of recurrence/response to chemotherapy, gene co-expression or clique analysis, and the like). The computer program product has stored therein a computer program for performing the calculation.

The present disclosure provides systems for executing the program described above, which system generally includes: a) a central computing environment; b) an input device, operatively connected to the computing environment, to receive patient data, wherein the patient data can include, for example, expression level or other value obtained from an assay using a biological sample from the patient, or microarray data, as described in detail above; c) an output device, connected to the computing environment, to provide information to a user (e.g., medical personnel); and d) an algorithm executed by the central computing environment (e.g., a processor), where the algorithm is executed based on the data received by the input device, and wherein the algorithm calculates a, risk, risk score, or treatment group classification, gene co-expression analysis, thresholding, or other functions described herein. The methods provided by the present invention may also be automated in whole or in part.

### Manual and Computer-Assisted Methods and Products

The methods and systems described herein can be implemented in numerous ways. In one embodiment of particular interest, the methods involve use of a communications infrastructure, for example the Internet. Several embodiments are discussed below. It is also to be understood that the present disclosure may be implemented in various forms of hardware, software, firmware, processors, or a combination thereof. The methods and systems described herein can be implemented as a combination of hardware and software. The software can be implemented as an application program tangibly embodied on a program storage device, or different portions of the software implemented in the user's computing environment (e.g., as an applet) and on the reviewer's computing environment, where the reviewer may be located at a remote site associated (e.g., at a service provider's facility).

For example, during or after data input by the user, portions of the data processing can be performed in the user-side computing environment. For example, the user-side computing environment can be programmed to provide for defined test codes to denote a likelihood "risk score," where the score is transmitted as processed or partially processed responses to the reviewer's computing environment in the form of test code for subsequent execution of one or more algorithms to provide a results and/or generate a report in the reviewer's computing environment. The risk score can be a numerical score (representative of a numerical value, e.g. likelihood of recurrence based on validation study population) or a non-numerical score representative of a numerical value or range of numerical values (e.g., low, intermediate, or high).

The application program for executing the algorithms described herein may be uploaded to, and executed by, a machine comprising any suitable architecture. In general, the machine involves a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof) that is executed via the operating system. In addition, various other peripheral devices may be connected to the computer platform such as an additional data storage device and a printing device.

As a computer system, the system generally includes a processor unit. The processor unit operates to receive information, which can include test data (e.g., level of a risk gene, level of a reference gene product(s); normalized level of a gene; and may also include other data such as patient data. This information received can be stored at least temporarily in a database, and data analyzed to generate a report as described above.

Part or all of the input and output data can also be sent electronically; certain output data (e.g., reports) can be sent electronically or telephonically (e.g., by facsimile, e.g., using devices such as fax back). Exemplary output receiving devices can include a display element, a printer, a facsimile device and the like. Electronic forms of transmission and/or display can include email, interactive television, and the like. In an embodiment of particular interest, all or a portion of the input data and/or all or a portion of the output data (e.g., usually at least the final report) are maintained on a web server for access, preferably confidential access, with typical browsers. The data may be accessed or sent to health professionals as desired. The input and output data, including all or a portion of the final report, can be used to populate a patient's medical record which may exist in a confidential database at the healthcare facility.

A system for use in the methods described herein generally includes at least one computer processor (e.g., where the method is carried out in its entirety at a single site) or at least two networked computer processors (e.g., where data is to be input by a user (also referred to herein as a "client") and transmitted to a remote site to a second computer processor for analysis, where the first and second computer processors are connected by a network, e.g., via an intranet or internet). The system can also include a user component(s) for input; and a reviewer component(s) for review of data, generated reports, and manual intervention. Additional components of the system can include a server component(s); and a database(s) for storing data (e.g., as in a database of report elements, e.g., interpretive report elements, or a relational database (RDB) which can include data input by the user and data output. The computer processors can be processors that are typically found in personal desktop computers (e.g., IBM, Dell, Macintosh), portable computers, mainframes, minicomputers, or other computing devices.

The networked client/server architecture can be selected as desired, and can be, for example, a classic two or three tier client server model. A relational database management system (RDMS), either as part of an application server component or as a separate component (RDB machine) provides the interface to the database.

In one example, the architecture is provided as a database-centric client/server architecture, in which the client application generally requests services from the application server which makes requests to the database (or the database server) to populate the report with the various report elements as required, particularly the interpretive report elements, especially the interpretation text and alerts. The server(s) (e.g., either as part of the application server machine or a separate RDB/relational database machine) responds to the client's requests.

The input client components can be complete, stand-alone personal computers offering a full range of power and features to run applications. The client component usually operates under any desired operating system and includes a communication element (e.g., a modem or other hardware for connecting to a network), one or more input devices (e.g., a keyboard, mouse, keypad, or other device used to transfer information or commands), a storage element (e.g., a hard drive or other computer-readable, computer-writable storage medium), and a display element (e.g., a monitor, television, LCD, LED, or other display device that conveys information to the user). The user enters input commands into the computer processor through an input device. Generally, the user interface is a graphical user interface (GUI) written for web browser applications.

The server component(s) can be a personal computer, a minicomputer, or a mainframe and offers data management, information sharing between clients, network administration and security. The application and any databases used can be on the same or different servers.

Other computing arrangements for the client and server(s), including processing on a single machine such as a mainframe, a collection of machines, or other suitable configuration are contemplated. In general, the client and server machines work together to accomplish the processing of the present disclosure.

Where used, the database(s) is usually connected to the database server component and can be any device that will hold data. For example, the database can be a any magnetic or optical storing device for a computer (e.g., CDROM, internal hard drive, tape drive). The database can be located remote to the server component (with access via a network, modem, etc.) or locally to the server component.

Where used in the system and methods, the database can be a relational database that is organized and accessed according to relationships between data items. The relational database is generally composed of a plurality of tables (entities). The rows of a table represent records (collections of information about separate items) and the columns represent fields (particular attributes of a record). In its simplest conception, the relational database is a collection of data entries that "relate" to each other through at least one common field.

Additional workstations equipped with computers and printers may be used at point of service to enter data and, in some embodiments, generate appropriate reports, if desired. The computer(s) can have a shortcut (e.g., on the desktop) to launch the application to facilitate initiation of data entry, transmission, analysis, report receipt, etc. as desired.

### Computer-readable storage media

The present disclosure also contemplates a computer-readable storage medium (e.g. CD-ROM, memory key, flash memory card, diskette, etc.) having stored thereon a program which, when executed in a computing environment, provides for implementation of algorithms to carry out all or a portion of the results of a response likelihood assessment as described herein. Where the computer-readable medium contains a complete program for carrying out the methods described herein, the program includes program instructions for collecting, analyzing and generating output, and generally includes computer readable code devices for interacting with a user as described herein, processing that data in conjunction with analytical information, and generating unique printed or electronic media for that user.

Where the storage medium provides a program that provides for implementation of a portion of the methods described herein (e.g., the user-side aspect of the methods (e.g., data input, report receipt capabilities, etc.)), the program provides for transmission of data input by the user (e.g., via the internet, via an intranet, etc.) to a computing environment at a remote site. Processing or completion of processing of the data is carried out at the remote site to generate a report. After review of the report, and completion of any needed manual intervention, to provide a complete report, the complete report is then transmitted back to the user as an electronic document or printed document (e.g., fax or mailed paper report). The storage medium containing a program according to the present disclosure can be packaged with instructions (e.g., for program installation, use, etc.) recorded on a suitable substrate or a web address where such instructions may be obtained. The computer-readable storage medium can also be provided in combination with one or more reagents for carrying out response likelihood assessment (e.g., primers, probes, arrays, or other such kit components).

All aspects of the present invention may also be practiced such that a limited number of additional genes that are co-expressed with the disclosed genes, for example as evidenced by statistically meaningful Pearson and/or Spearman correlation coefficients, are included in a prognostic or predictive test in addition to and/or in place of disclosed genes.

Having described the invention, the same will be more readily understood through reference to the following Examples, which are provided by way of illustration, and are not intended to limit the invention in any way.

### EXAMPLE 1:

The study included breast cancer tumor samples obtained from 136 patients diagnosed with breast cancer ("Providence study"). Biostatistical modeling studies of prototypical data sets demonstrated that amplified RNA is a useful substrate for biomarker identification studies. This was verified in this study by including known breast cancer biomarkers along with candidate prognostic genesin the tissues samples. The known biomarkers were shown to be associated with clinical outcome in amplified RNA based on the criteria outlined in this protocol.

### Study design

Refer to the original Providence Phase II study protocol for biopsy specimen information. The study looked at the statistical association between clinical outcome and 384candidate biomarkers tested in amplified samples derived from 25 ng of mRNA that was extracted from fixed, paraffin-embedded tissue samples obtained from 136 of the original Providence Phase II study samples. The expression level of the candidate genes was normalized using reference genes. Several reference genes were analyzed in this study: AAMP, ARF1, EEF1A1, ESD, GPS1, H3F3A, HNRPC, RPL13A, RPL41, RPS23, RPS27, SDHA, TCEA1, UBB, YWHAZ, B-actin, GUS, GAPDH, RPLPO, and TFRC.

The 136 samples were split into 3 automated RT plates each with 2X 48 samples and 40 samples and 3 RT positive and negative controls. Quantitative PCR assays were performed in 384 wells without replicate using the QuantiTect Probe PCR Master Mix® (Qiagen). Plates were analyzed on the Light Cycler® 480 and, after data quality control, all samples from the RT plate 3 were repeated and new RT-PCR data was generated. The data was normalized by subtracting the median crossing point (Cp) (point at which detection rises above background signal) for five reference genes from the C_{P} value for each individual candidate gene. This normalization is performed on each sample resulting in final data that has been adjusted for differences in overall sample Cp. This data set was used for the final data analysis.

### Data Analysis

For each gene, a standard z test was run.. (S. Darby, J. Reissland, Journal of the Royal Statistical Society 144(3):298-331 (1981)). This returns a z score (measure of distance in standard deviations of a sample from the mean), p value, and residuals along with other statistics and parameters from the model. If the z score is negative, expression is positively correlated with a good prognosis; if positive, expression is negatively correlated to a good prognosis. Using the p values, a q value was created using a library q value. The poorly correlated and weakly expressed genes were excluded from the calculation of the distribution used for the q values. For each gene, Cox Proportional Hazard Model test was run checking survival time matched with the event vector against gene expression. This returned a hazard ratio (HR) estimating the effect of expression of each gene (individually) on the risk of a cancer-related event. The resulting data is provided in Tables 1-6. A HR < 1 indicates that expression of that gene is positively associated with a good prognosis, while a HR > 1 indicates that expression of that gene is negatively associated with a good prognosis.

### EXAMPLE 2:

### Study design

Amplified samples were derived from 25 ng of mRNA that was extracted from fixed, paraffin-embedded tissue samples obtained from 78 evaluable cases from a Phase II breast cancer study conducted at Rush University Medical Center. Three of the samples failed to provide sufficient amplified RNA at 25 ng, so amplification was repeated a second time with 50 ng of RNA. The study also analyzed several reference genes for use in normalization: AAMP, ARF1, EEF1A1, ESD, GPS1, H3F3A, HNRPC, RPL13A, RPL41, RPS23, RPS27, SDHA, TCEA1, UBB, YWHAZ, Beta-actin, RPLPO, TFRC, GUS, and GAPDH.

Assays were performed in 384 wells without replicate using the QuantiTect Probe PCR Master Mix. Plates were analyzed on the Light Cycler 480 instruments. This data set was used for the final data analysis. The data was normalized by subtracting the median C_{P} for five reference genes from the C_{P} value for each individual candidate gene. This normalization was performed on each sample resulting in final data that was adjusted for differences in overall sample Cp.

### Data analysis

There were 34 samples with average CP values above 35. However, none of the samples were excluded from analysis because they were deemed to have sufficient valuable information to remain in the study. Principal Component Analysis (PCA) was used to determine whether there was a plate effect causing variation across the different RT plates. The first principal component correlated well with the median expression values, indicating that expression level accounted for most of the variation between samples. Also, there were no unexpected variations between plates.

### Data for Other Variables

Group - The patients were divided into two groups (cancer/non-cancer). There was little difference between the two in overall gene expression as the difference between median CP value in each group was minimal (0.7).

Sample Age - The samples varied widely in their overall gene expression but there was a trend toward lower Cp values as they decreased in age.

Instrument - The overall sample gene expression from instrument to instrument was consistent. One instrument showed a slightly higher median Cp compared to the other three, but it was well within the acceptable variation.

RT Plate - The overall sample gene expression between RT plates was also very consistent. The median Cp for each of the 3 RT plates (2 automated RT plates and 1 manual plate containing repeated samples) were all within 1 C_{P} of each other.

### Univariate Analyses for Genes Significantly Different Between Study Groups

The genes were analyzed using the z-test and Cox Proportional Hazard Model, as described in Example 1. The resulting data can be seen in Tables 7-12.

### EXAMPLE 3:

The statistical correlations between clinical outcome and expression levels of the genes identified in Examples 1 and 2 were validated in breast cancer gene expression datasets maintained by the Swiss Institute of Bioinformatics (SIB). Further information concerning the SIB database, study datasets, and processing methods, is providing in P. Wirapati, et al., Breast Cancer Research 10(4):R65 (2008). Univariate Cox proportional hazards analyses were performed to confirm the relationship between clinical outcome (DFS, MFS, OS) of breast cancer patients and expression levels of the genes identified as significant in the amplified RNA studies described above. The meta-analysis included both fixed-effect and random-effect models, which are further described in L. Hedges and J. Vevea, Psychological Methods 3 (4): 486-504 (1998) and K. Sidik and J. Jonkman, Statistics in Medicine 26:1964-1981 (2006) (the contents of which are incorporated herein by reference). The results of the validation for all genes identified as having a stastistically significant association with breast cancer clinical outcome are described in Table 13. In those tables, "Est" designates an estimated coefficient of a covariate (gene expression); "SE" is standard error; "t" is the t-score for this estimate (i.e., Est/SE); and "fe" is the fixed estimate of effect from the meta analysis. Several of gene families with significant statistical association with clinical outcome (including metabolic, proliferation, immune, and stromal group genes) in breast cancer were confirmed using the SIB dataset. For example, Table 14 contains analysis of genes included in the metabolic group and Table 15 the stromal group.

### EXAMPLE 4:

A co-expression analysis was conducted using microarray data from six (6) breast cancer data sets. The "processed" expression values are taken from the GEO website, however, further processing was necessary. If the expression values are RMA, they are median normalized on the sample level. If the expression values are MAS5.0, they are: (1) changed to 10 if they are <10; (2) log base e transformed; and (3) median normalized on the sample level.

Generating Correlation Pairs: A rank matrix was generated by arranging the expression values for each sample in decreasing order. Then a correlation matrix was created by calculating the Spearman correlation values for every pair of probe IDs. Pairs of probes which had a Spearman value ≥ 0.7 were considered co-expressed. Redundant or overlapping correlation pairs in multiple datasets were identified. For each correlation matrix generated from an array dataset, pairs of significant probes that occur in >1 dataset were identified. This served to filter "non-significant" pairs from the analysis as well as provide extra evidence for "significant" pairs with their presence in multiple datasets. Depending on the number of datasets included in each tissue specific analysis, only pairs which occur in a minimum # or % of datasets were included.

Co-expression cliques were generated using the Bron-Kerbosch algorithm for maximal clique finding in an undirected graph. The algorithm generates three sets of nodes: *compsub, candidates,* and *not. Compsub* contains the set of nodes to be extended or shrunk by one depending on its traversal direction on the tree search. *Candidates* consists of all the nodes eligible to be added to *compsub. Not* contains the set of nodes that have been added to *compsub* and are now excluded from extension. The algorithm consists of five steps: selection of a candidate; adding the candidate node to *compsub;* creating new sets *candidates* and *not* from the old sets by removing all points not connected to the candidate node; recursively calling the extension operator on the new *candidates* and *not* sets; and upon return, remove the candidate node from *compsub* and place in the old *not* set.

There was a depth-first search with pruning, and the selection of candidate nodes had an effect on the run time of the algorithm. By selecting nodes in decreasing order of frequency in the pairs, the run time was optimized. Also, recursive algorithms generally cannot be implemented in a multi-threaded manner, but was multi-threaded the extension operator of the first recursive level. Since the data between the threads were independent because they were at the top-level of the recursive tree, they were run in parallel.

Clique Mapping and Normalization: Since the members of the co-expression pairs and cliques are at the probe level, one must map the probe IDs to genes (or Refseqs) before they can be analyzed. The Affymetrix gene map information was used to map every probe ID to a gene name. Probes may map to multiple genes, and genes may be represented by multiple probes. The data for each clique is validated by manually calculating the correlation values for each pair from a single clique.

The results of this co-expression analysis are set forth in Tables 16-18.

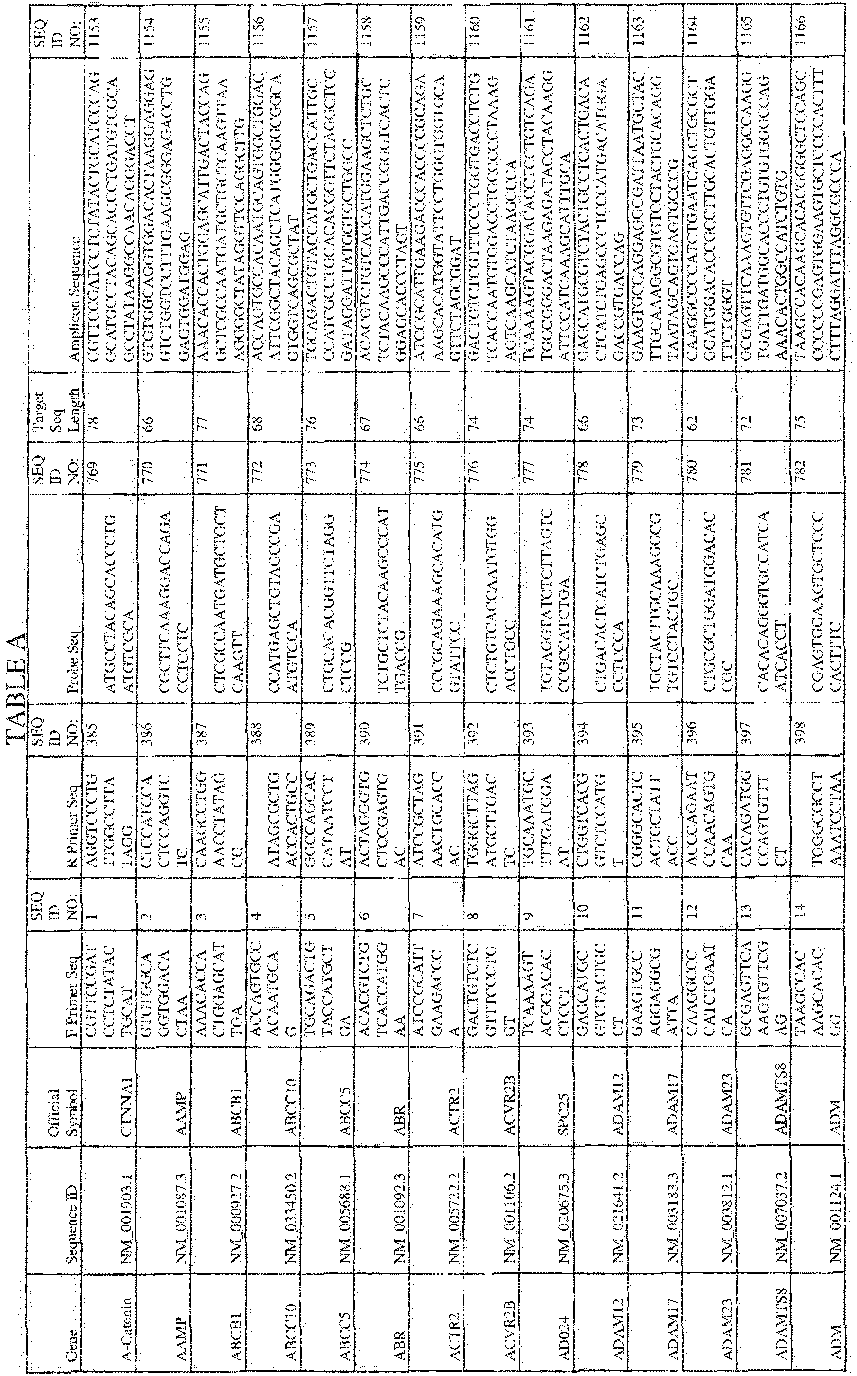

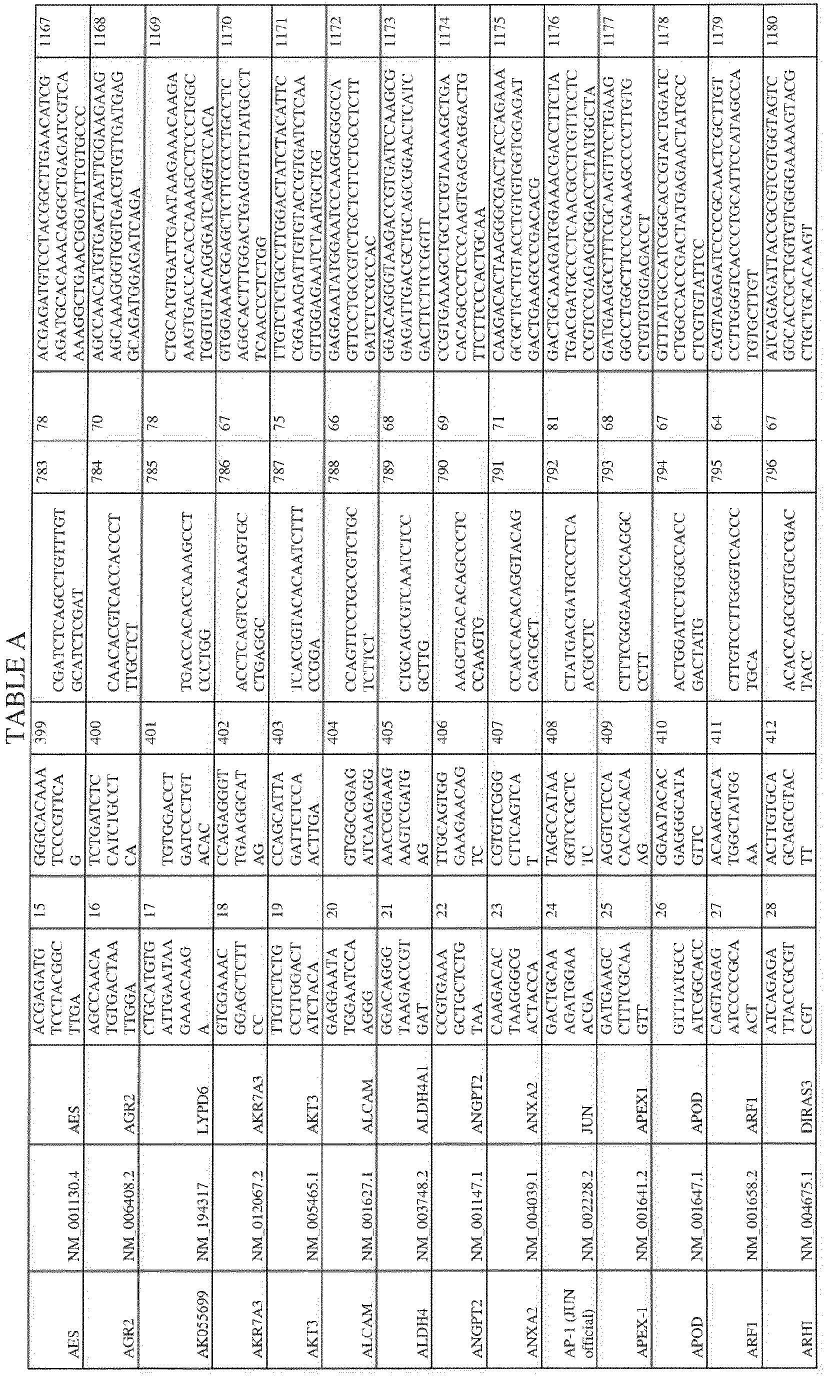

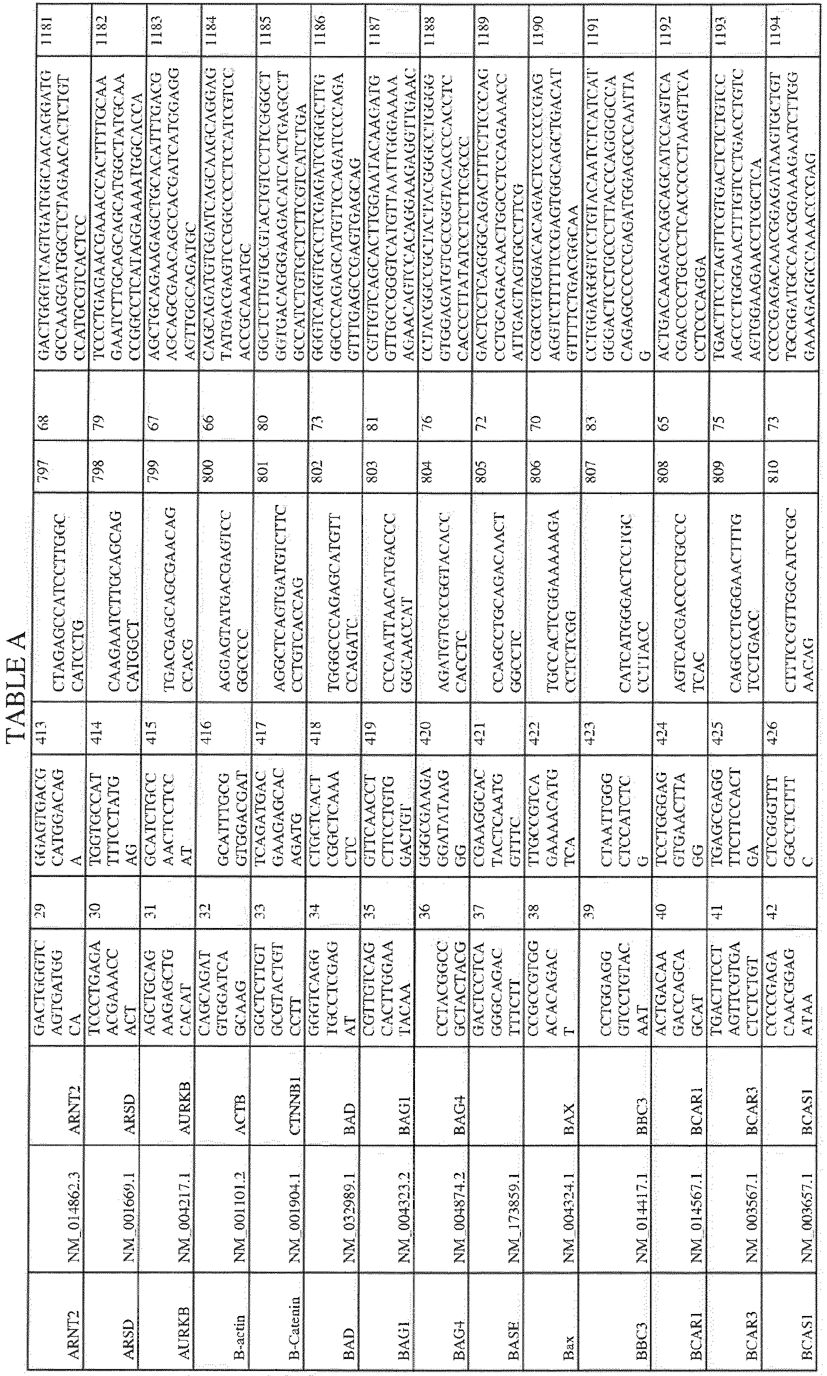

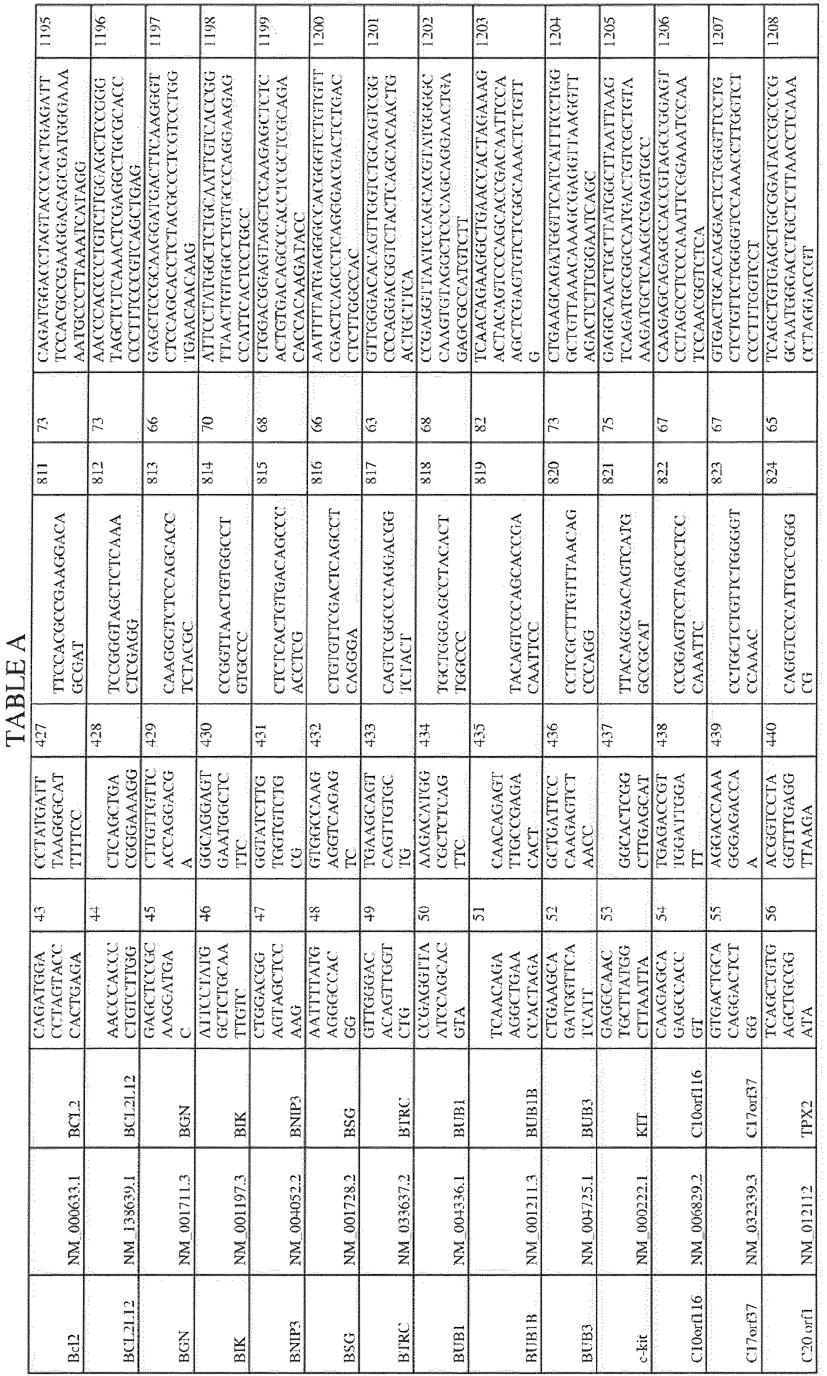

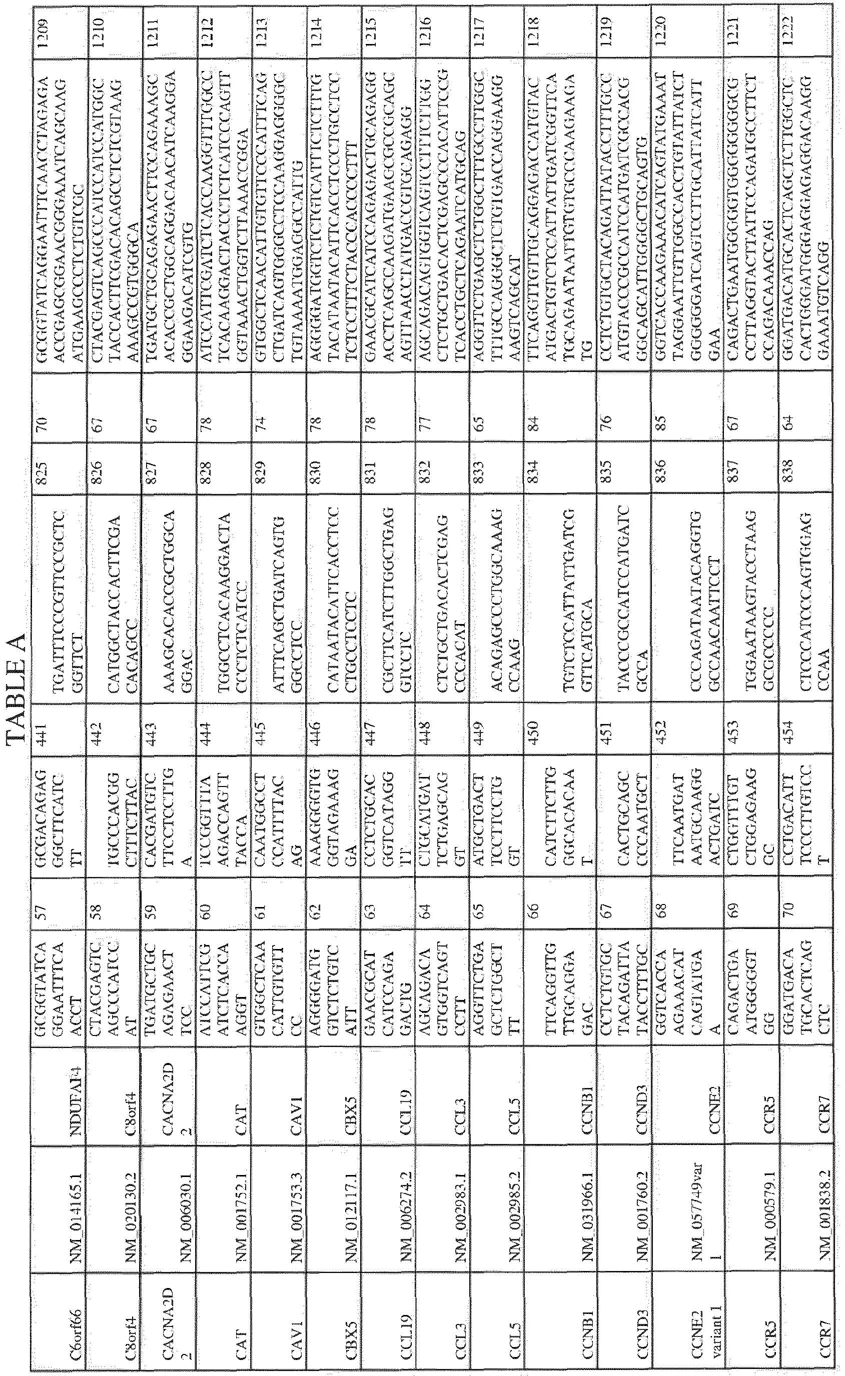

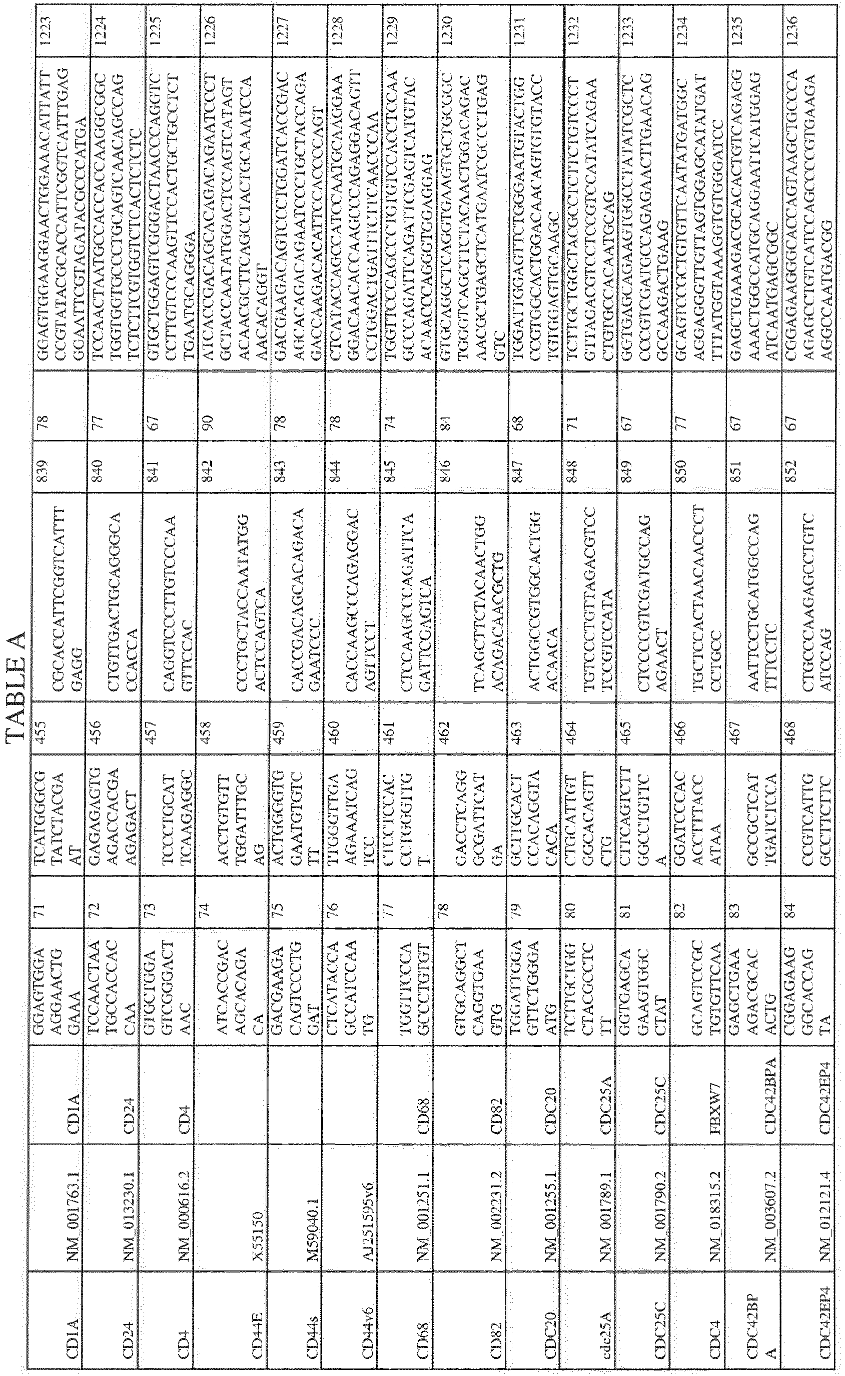

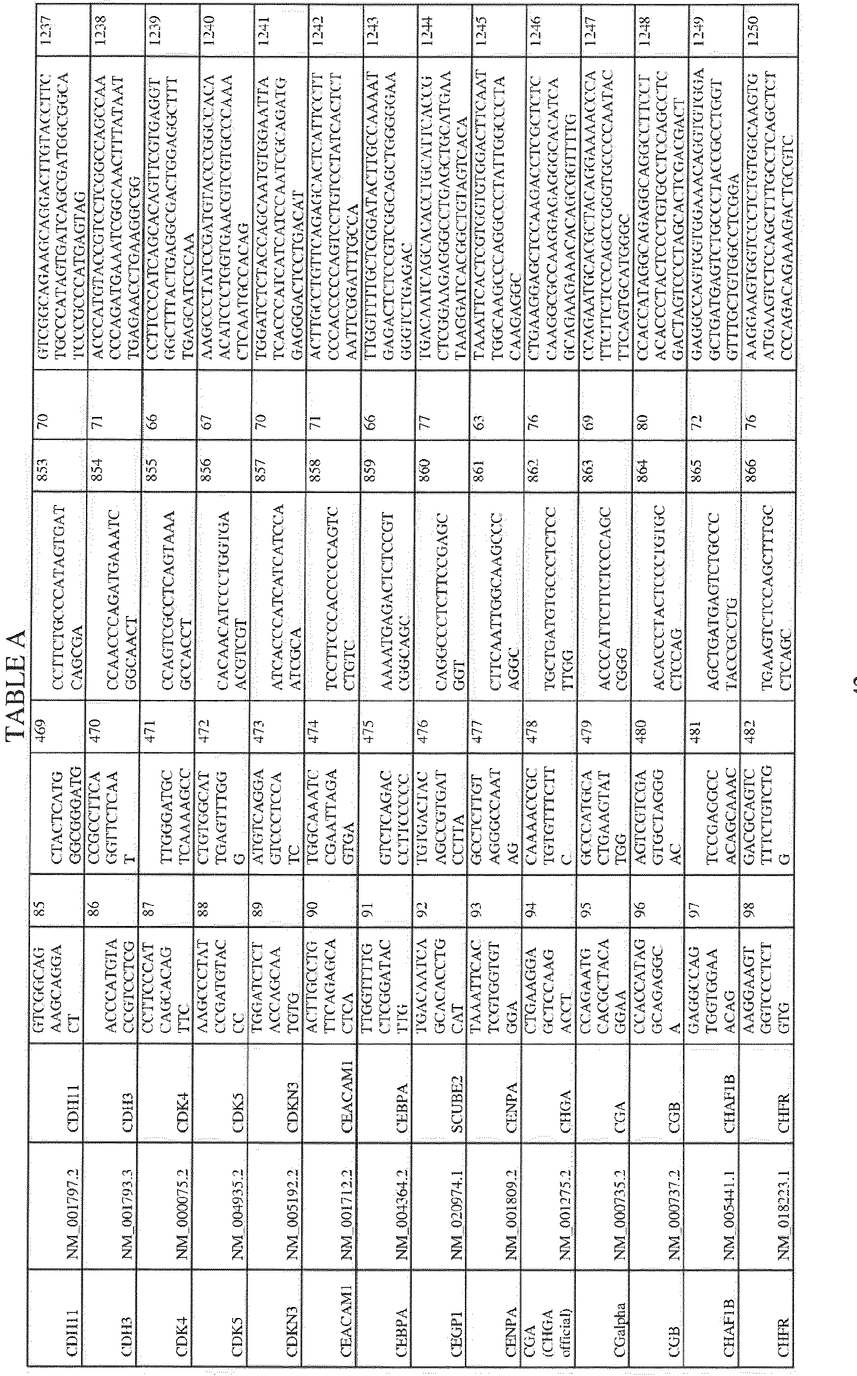

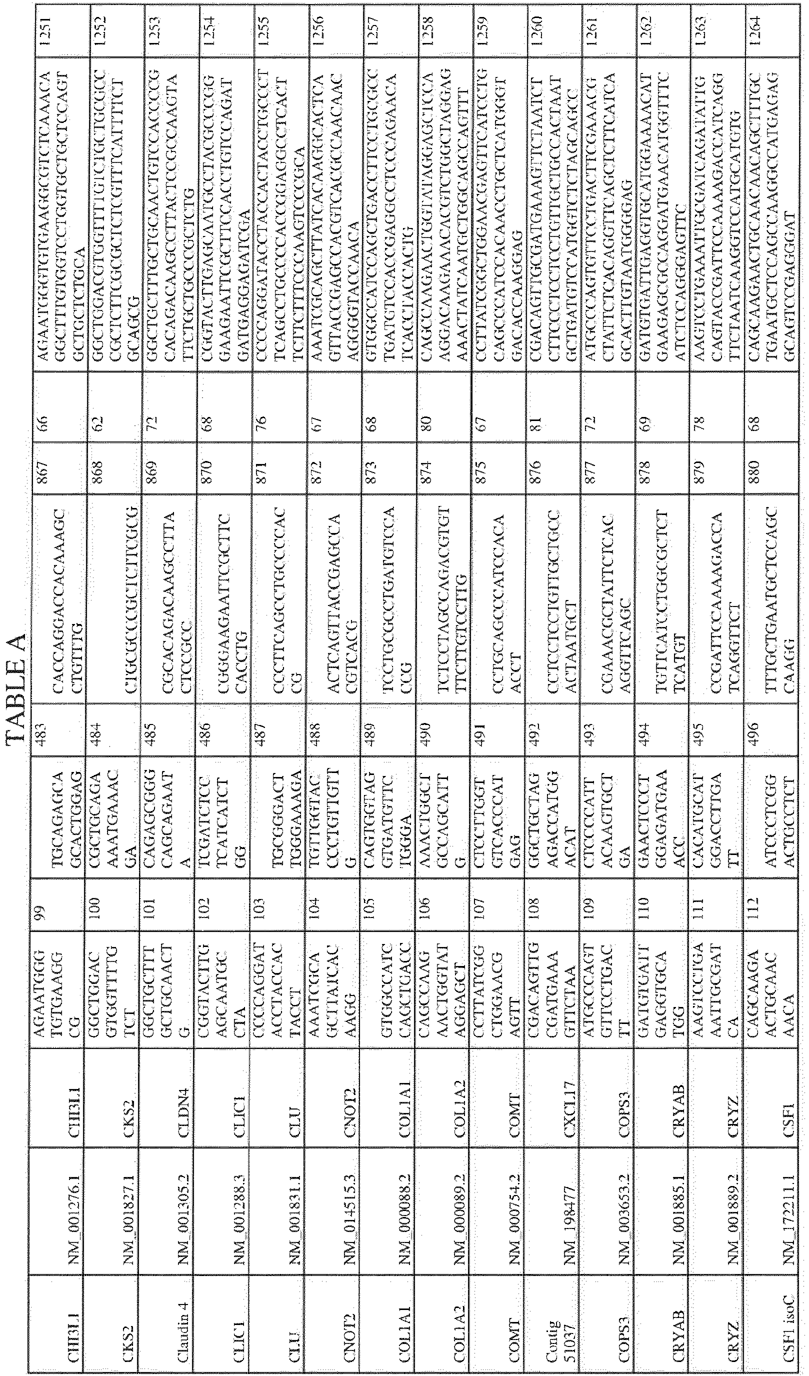

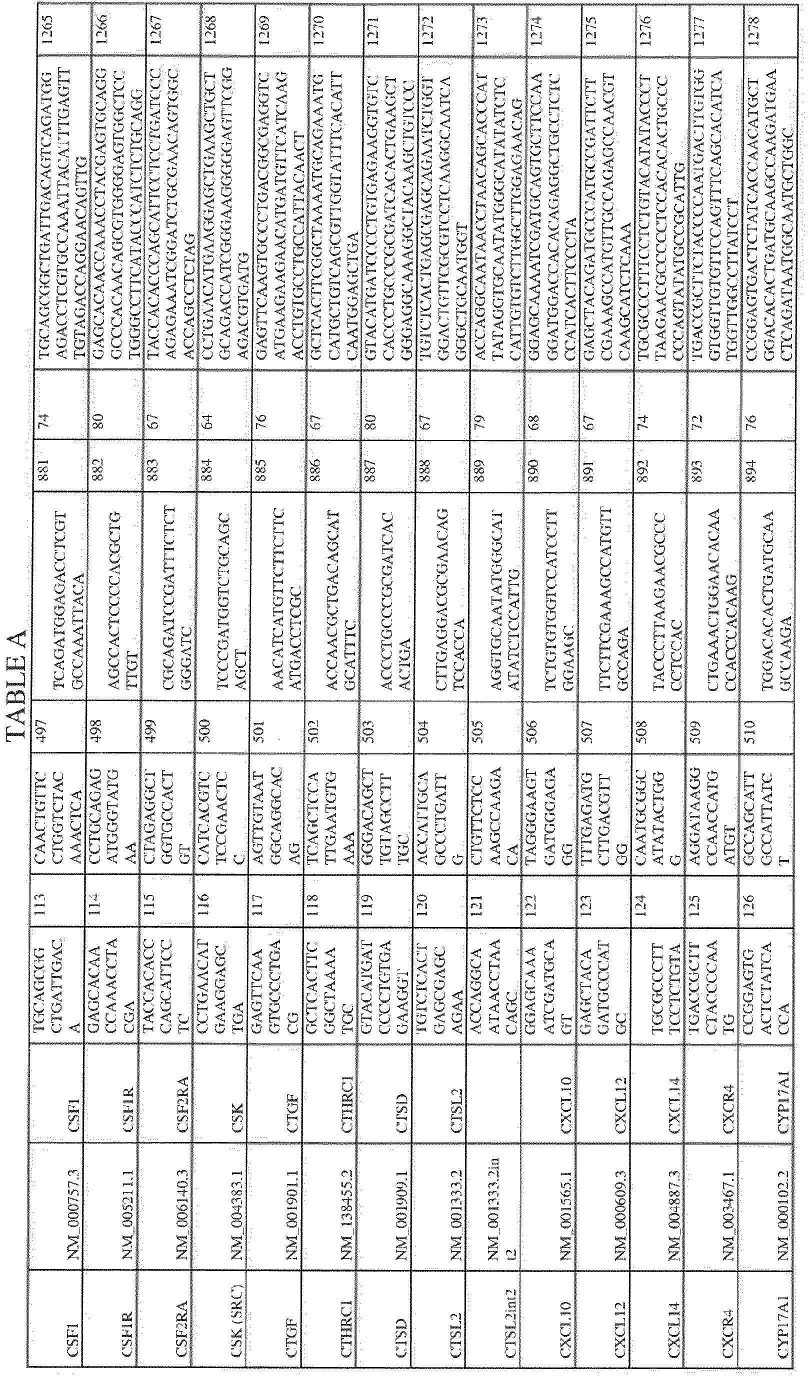

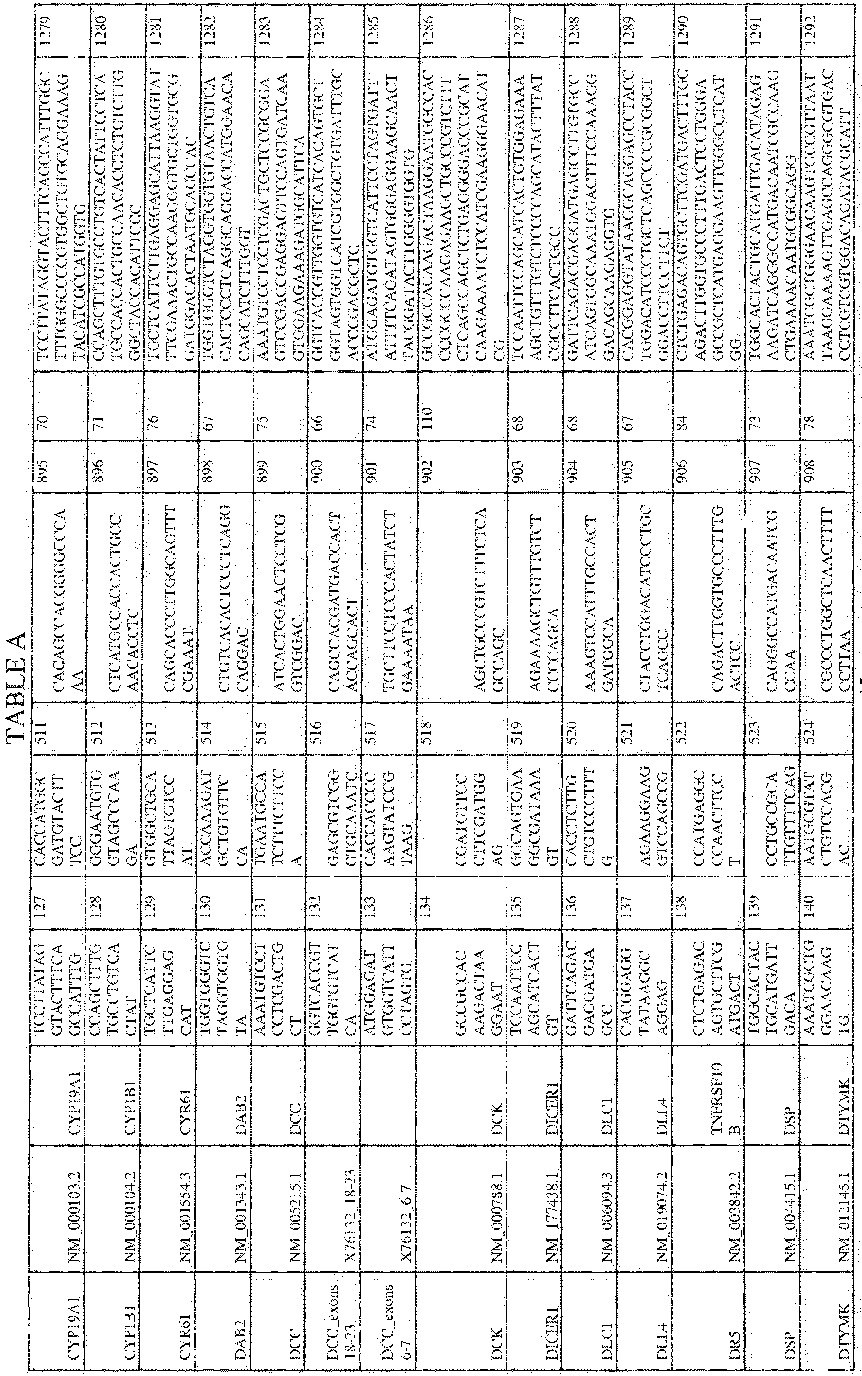

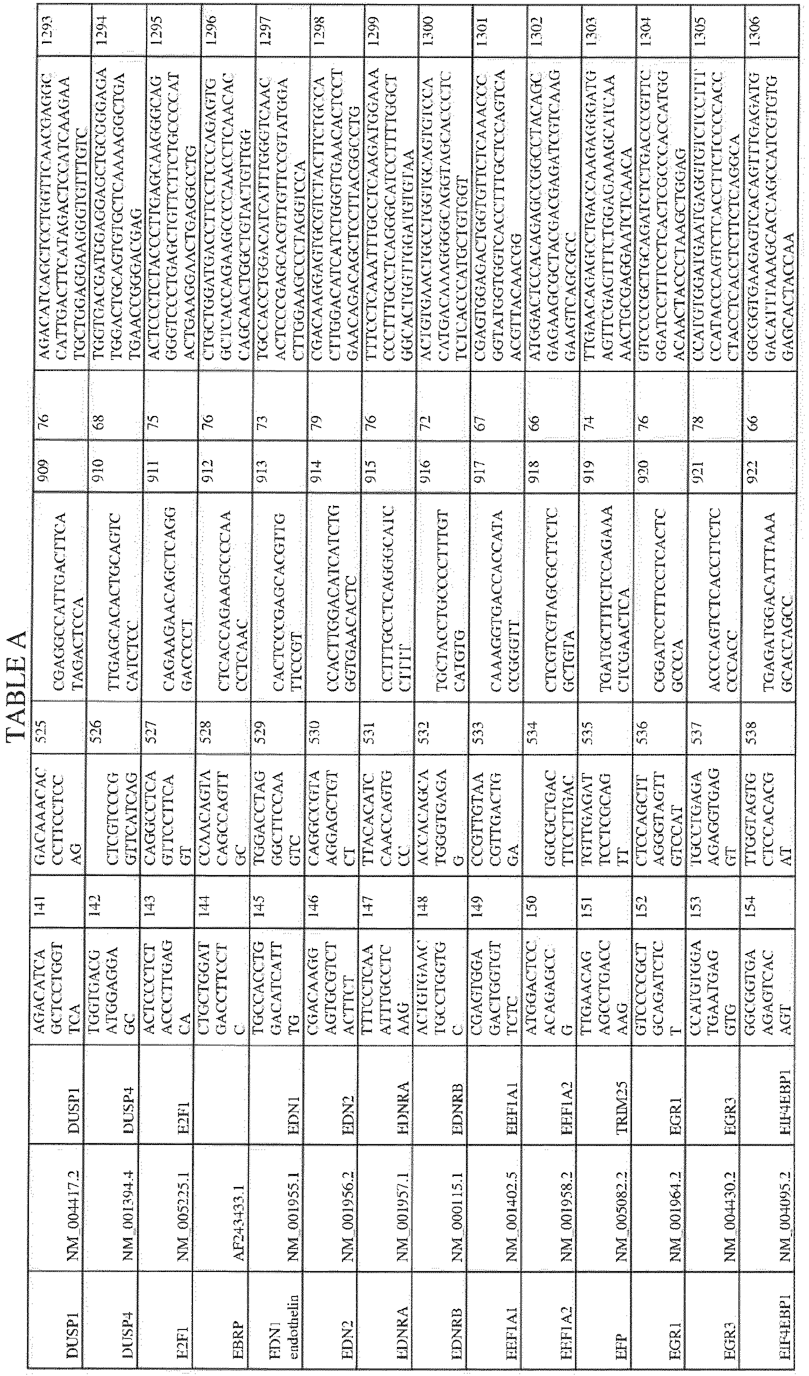

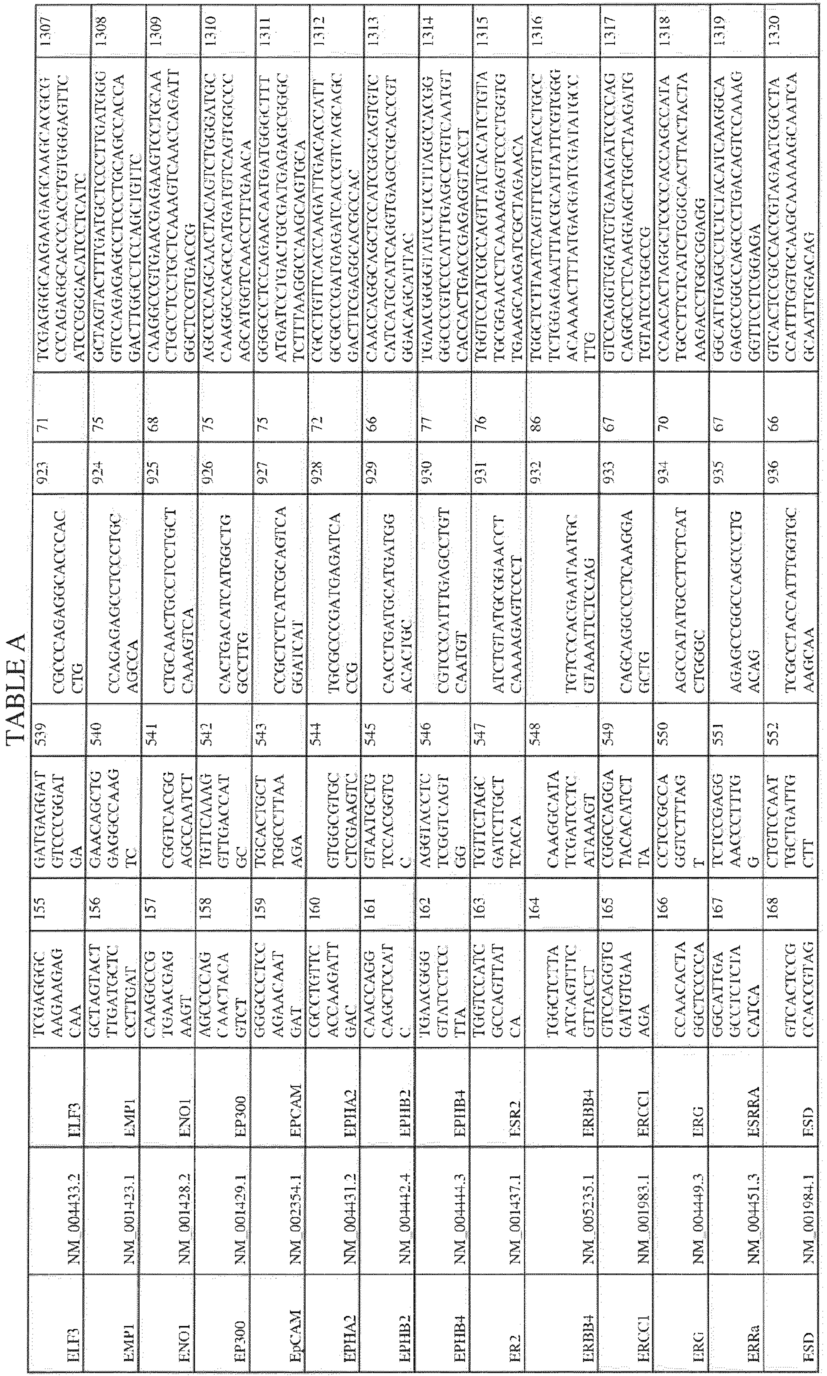

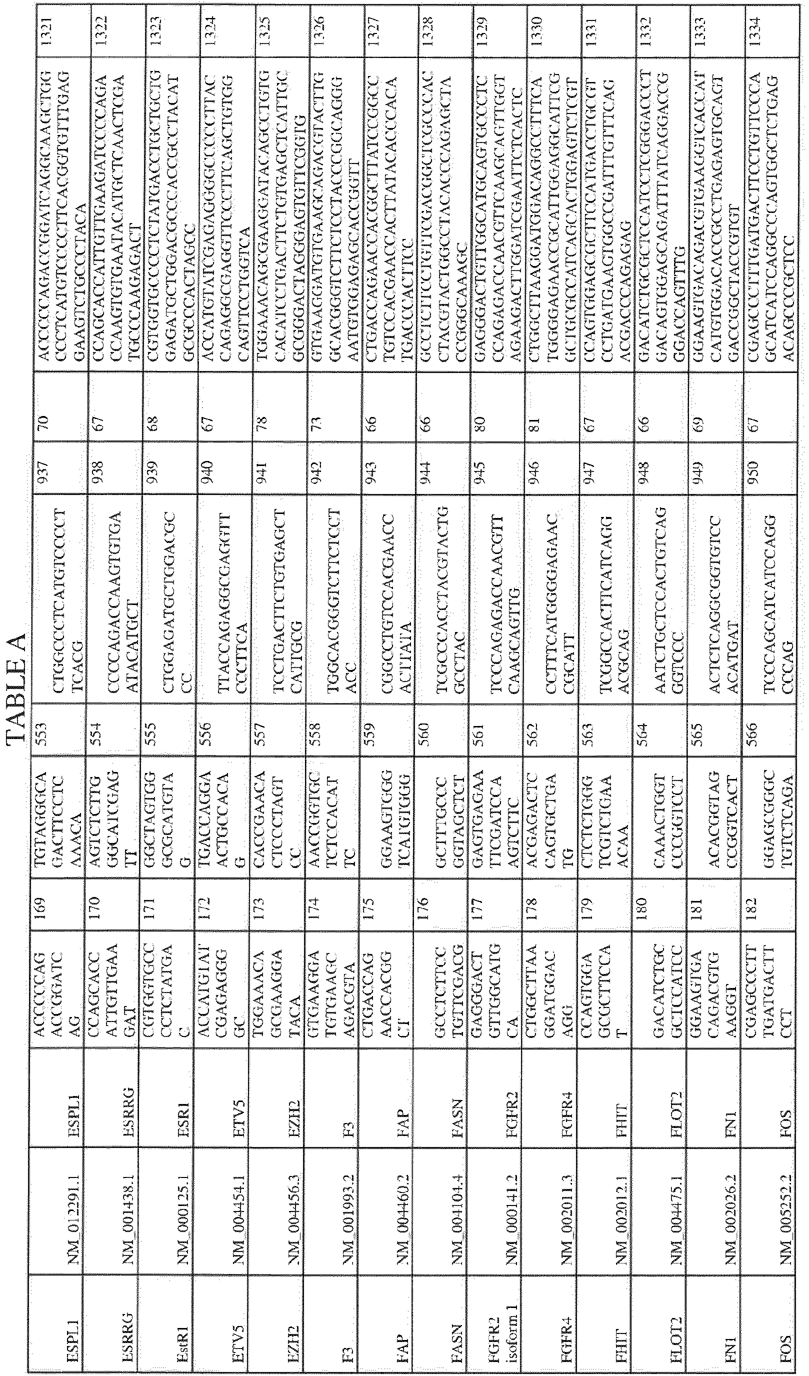

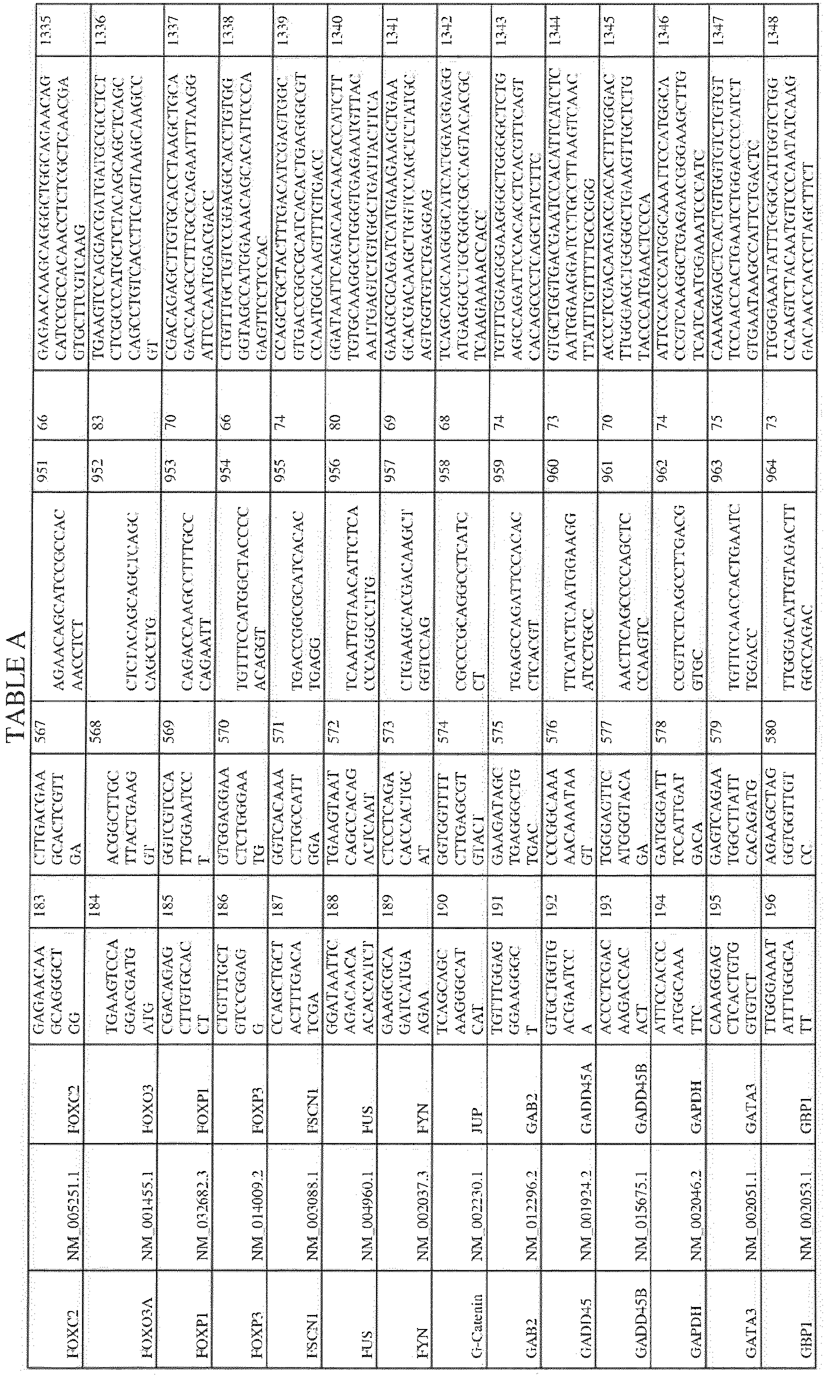

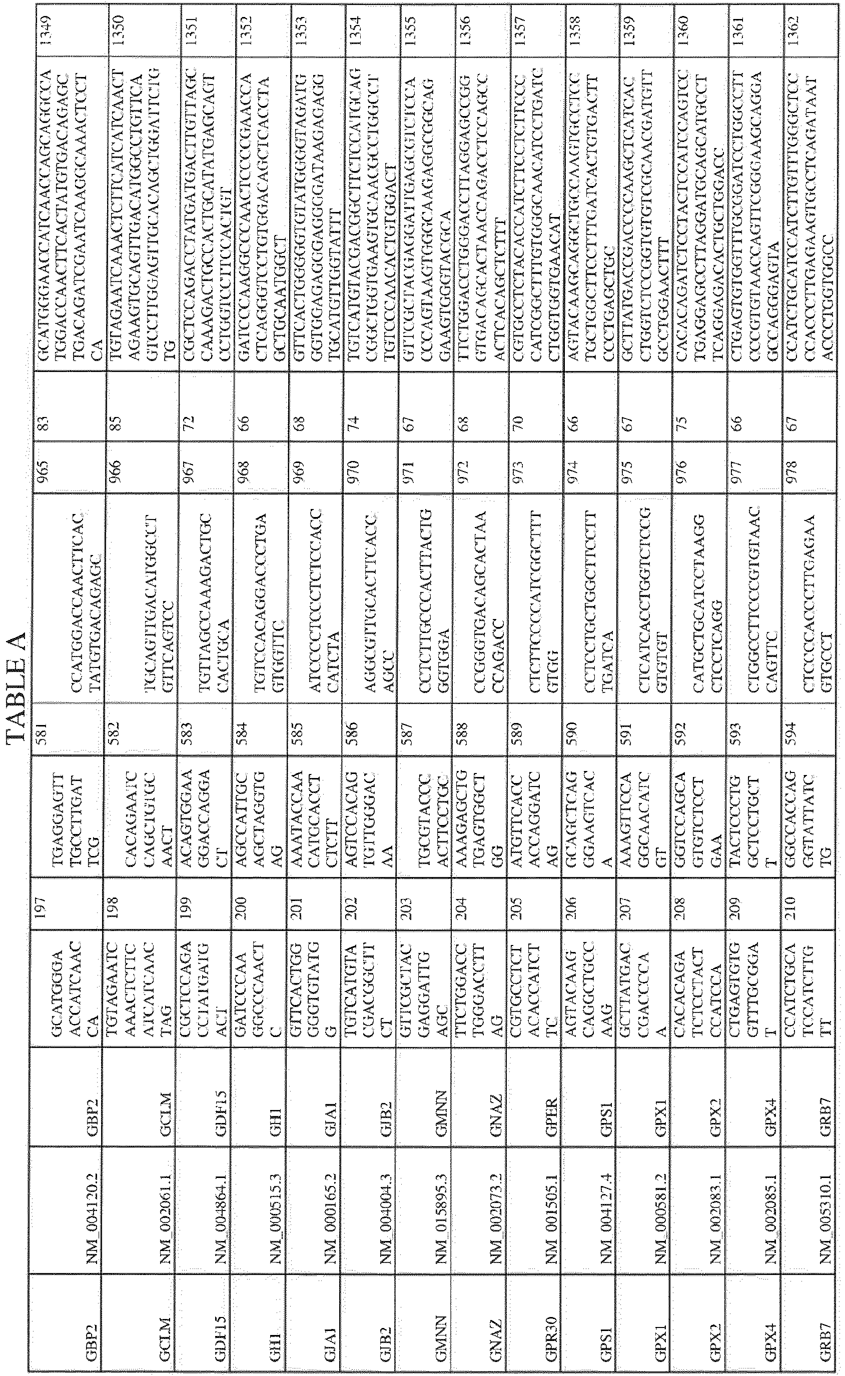

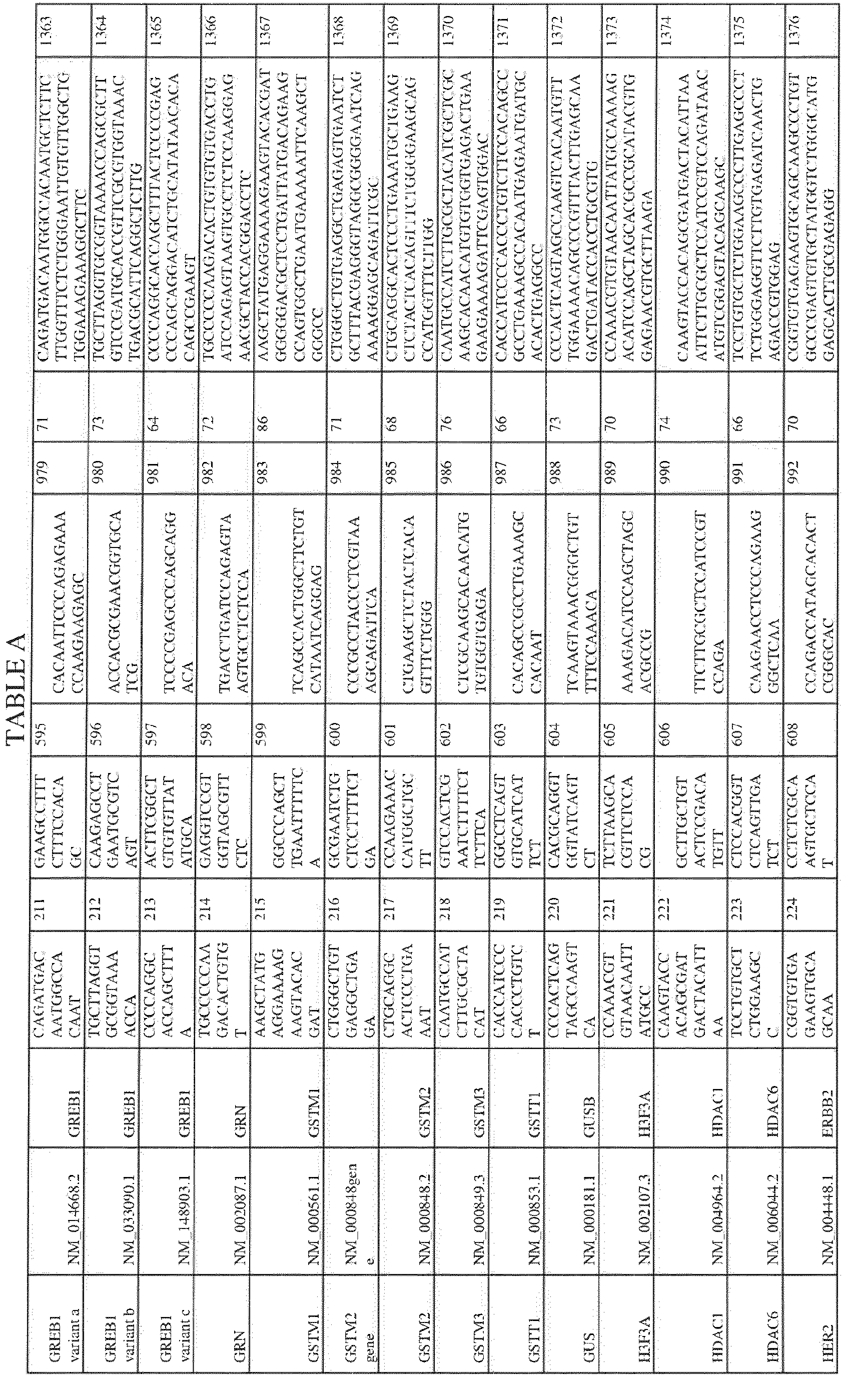

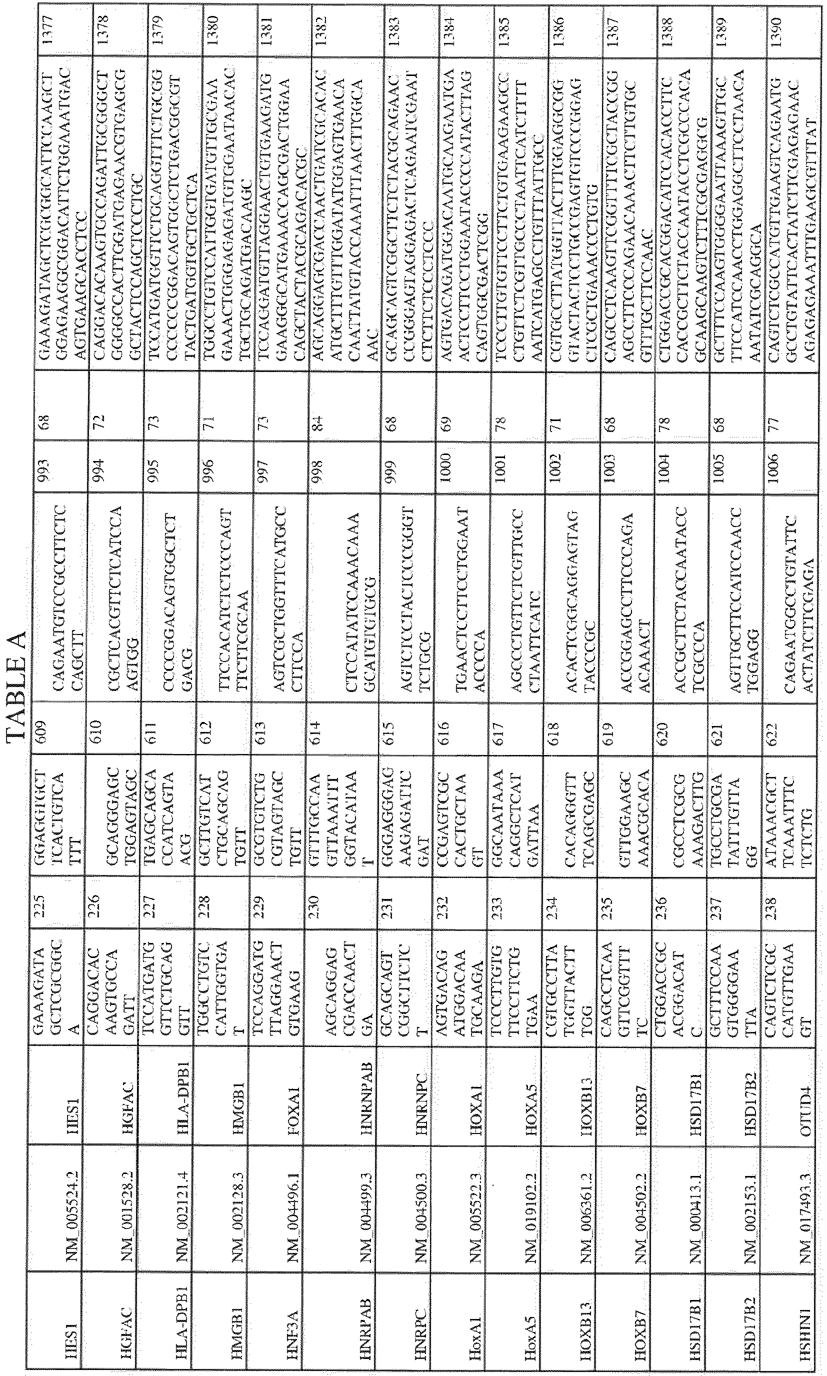

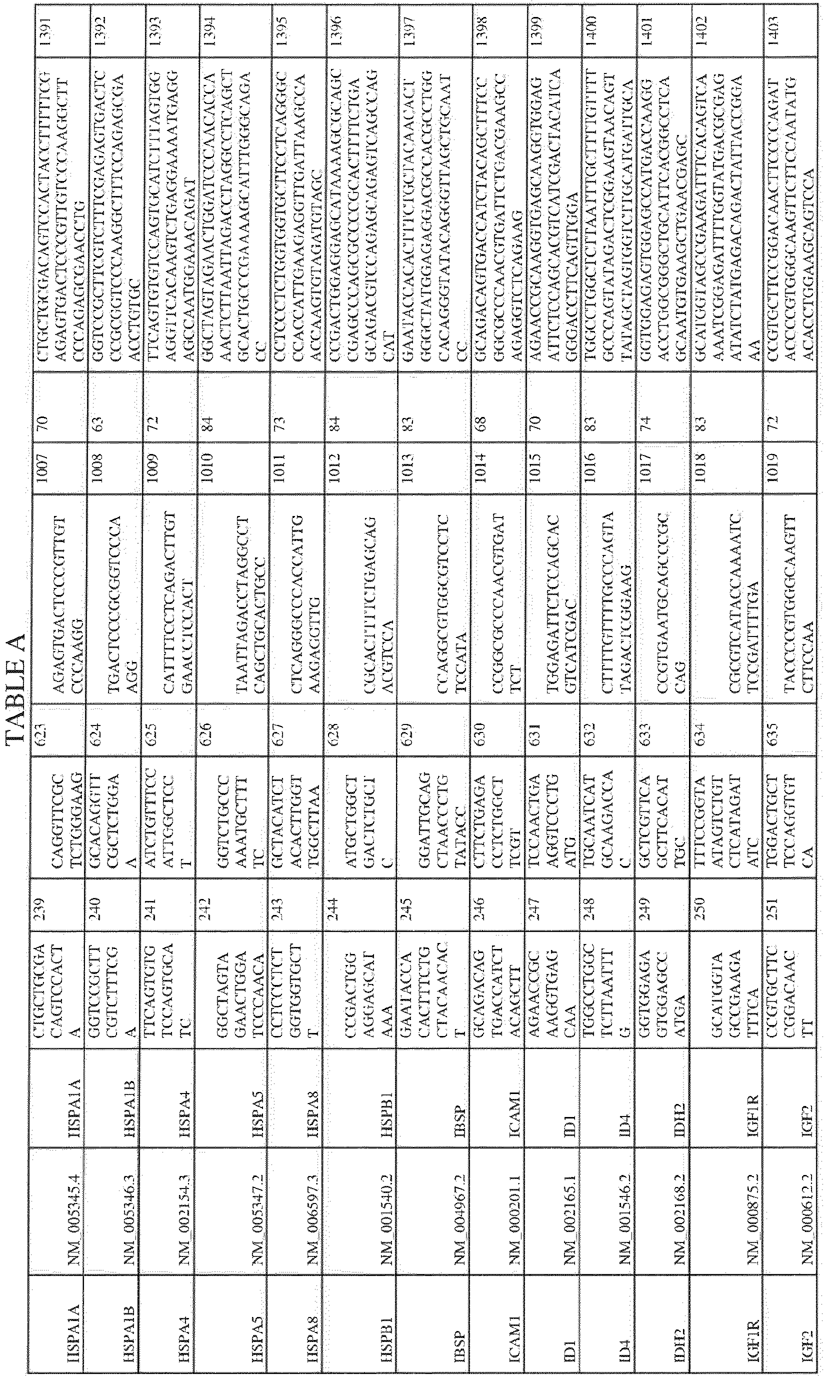

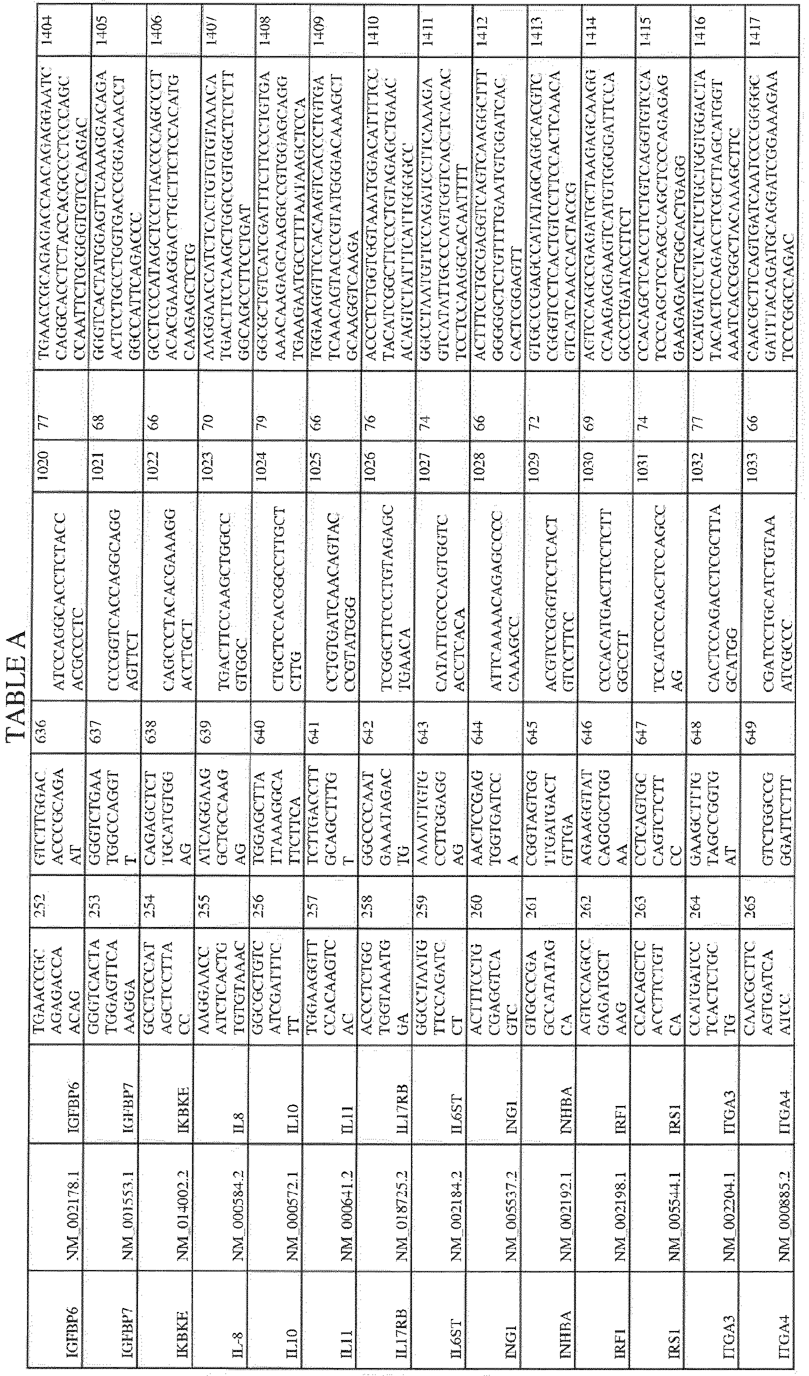

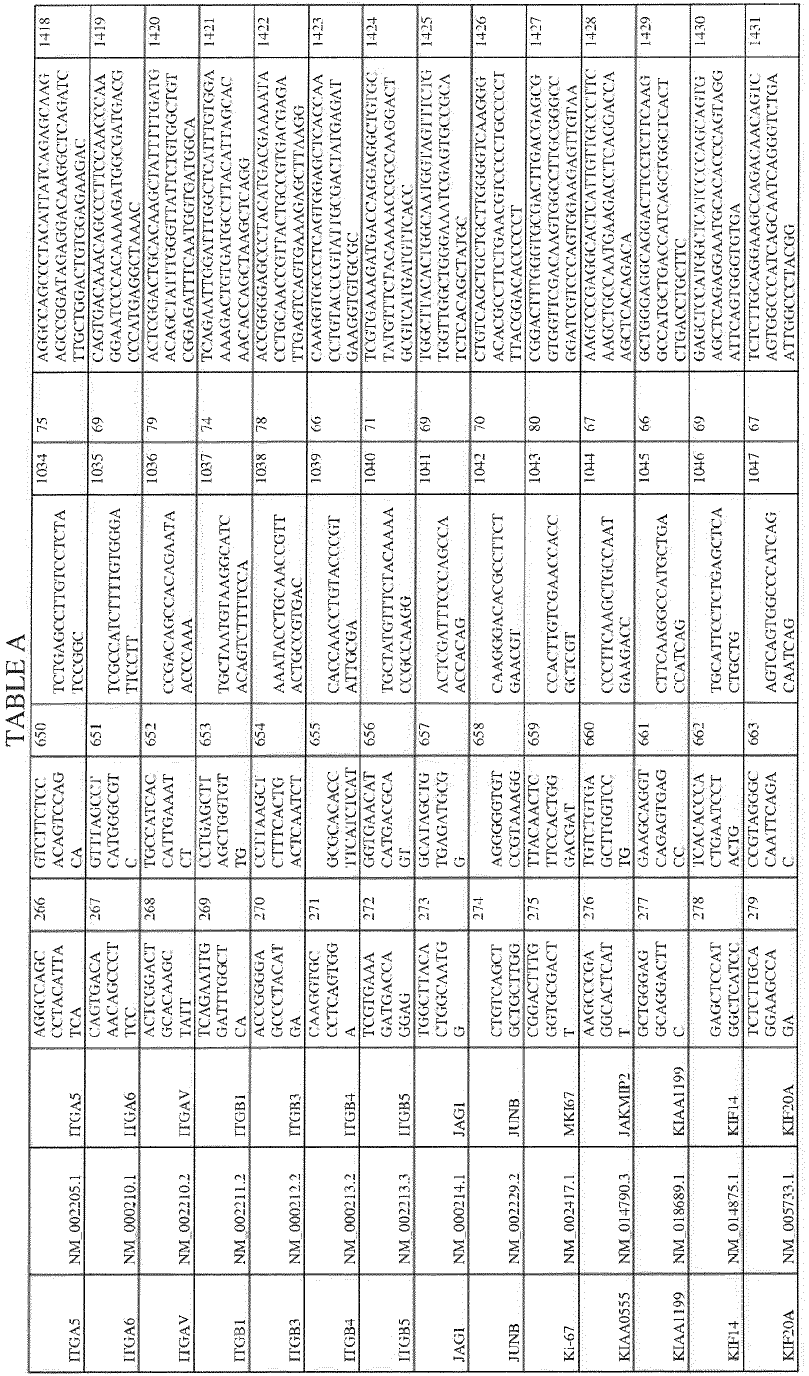

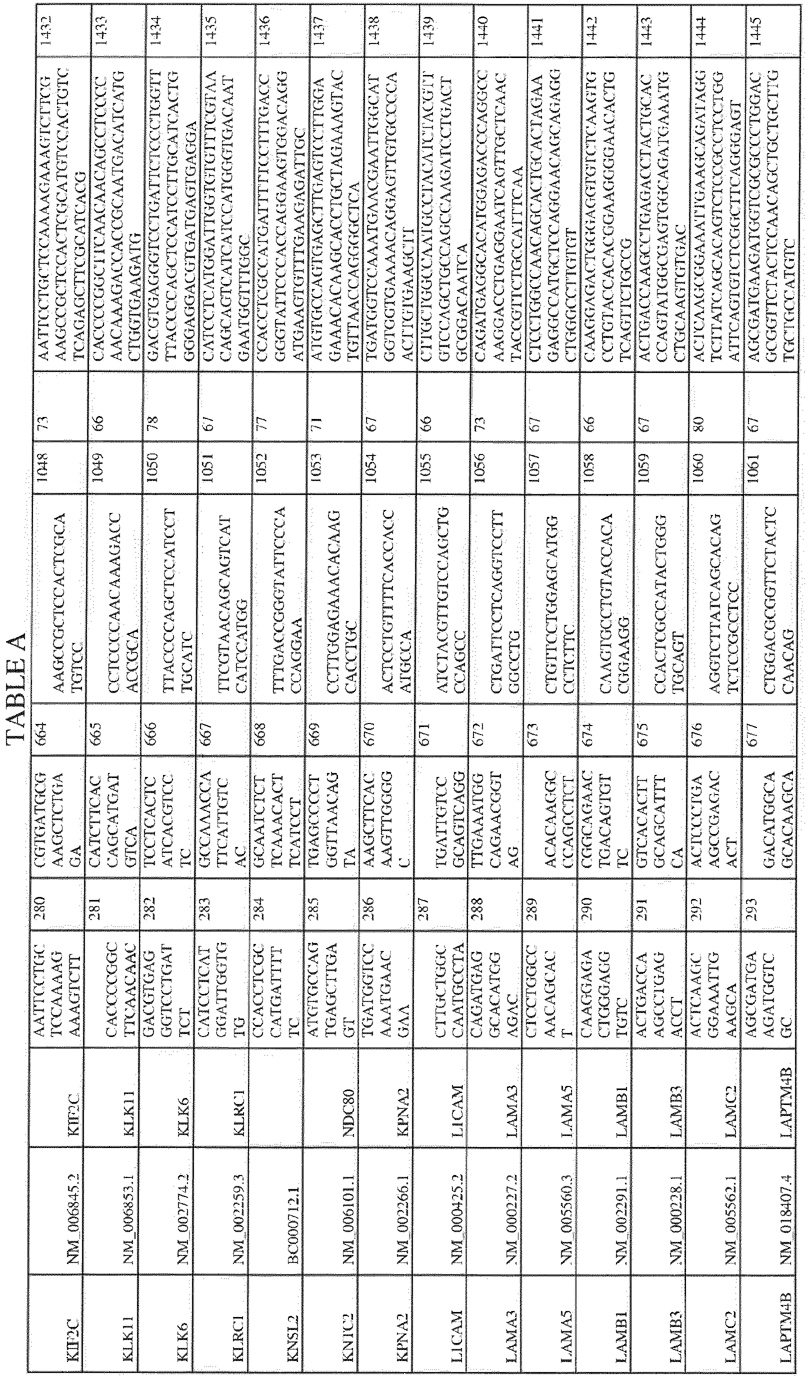

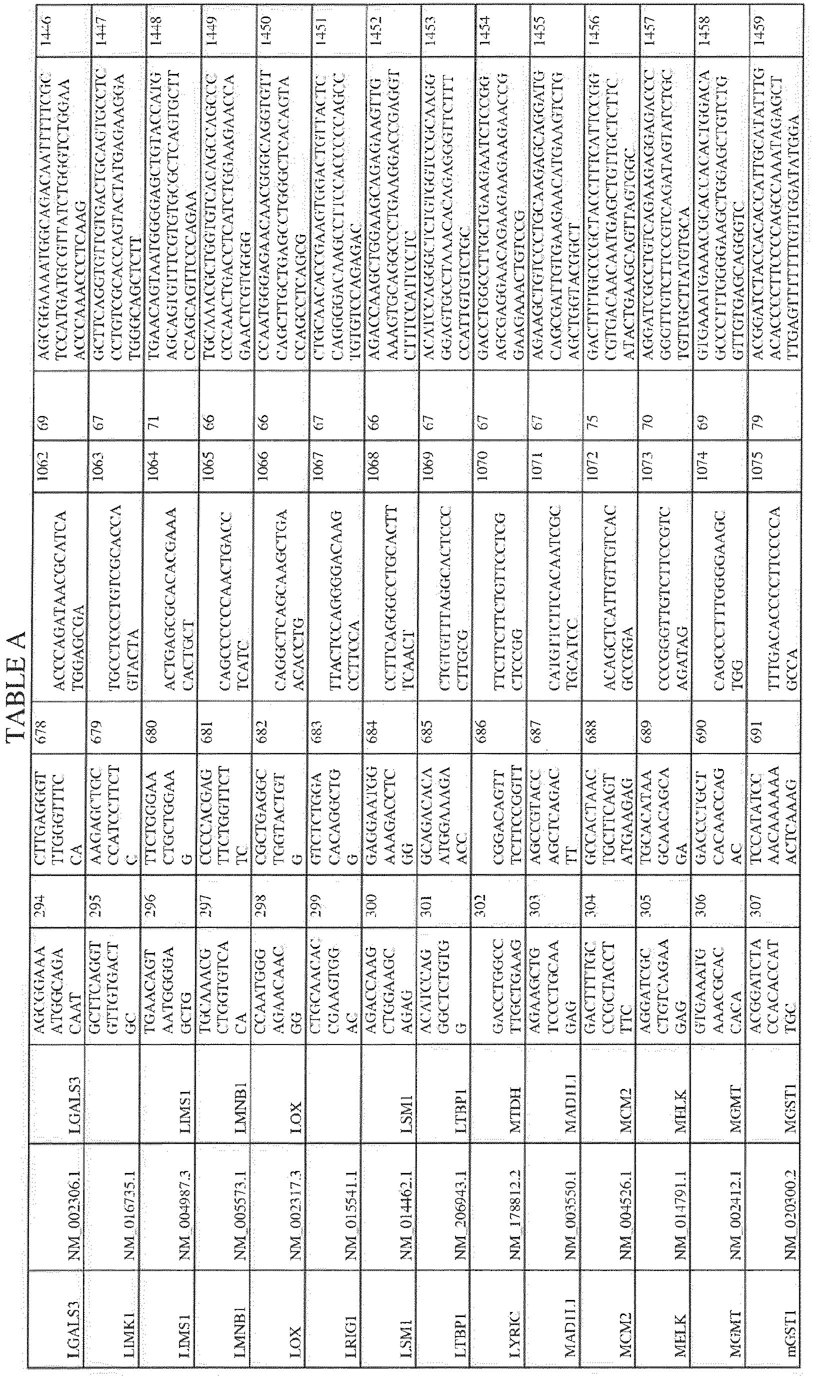

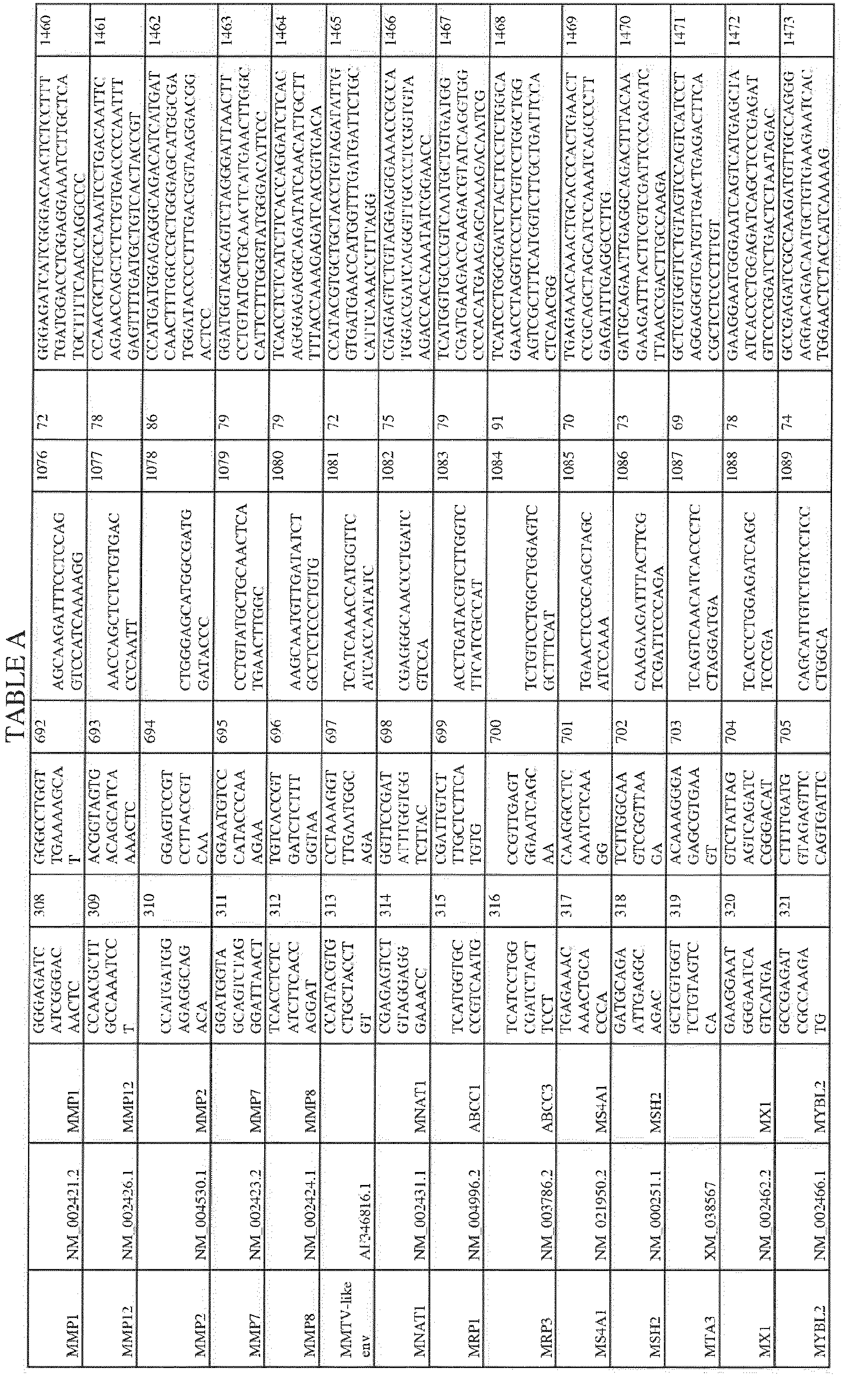

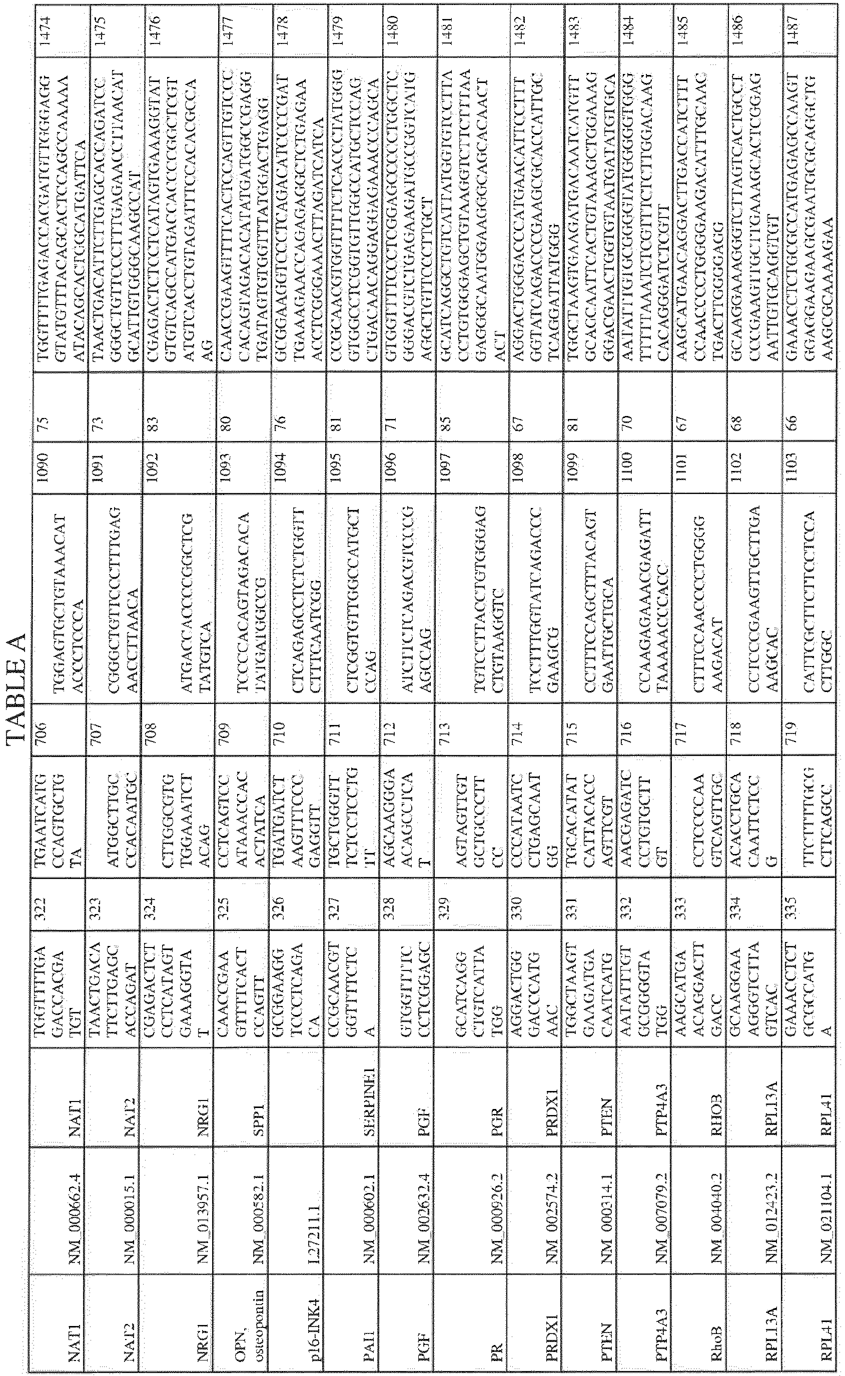

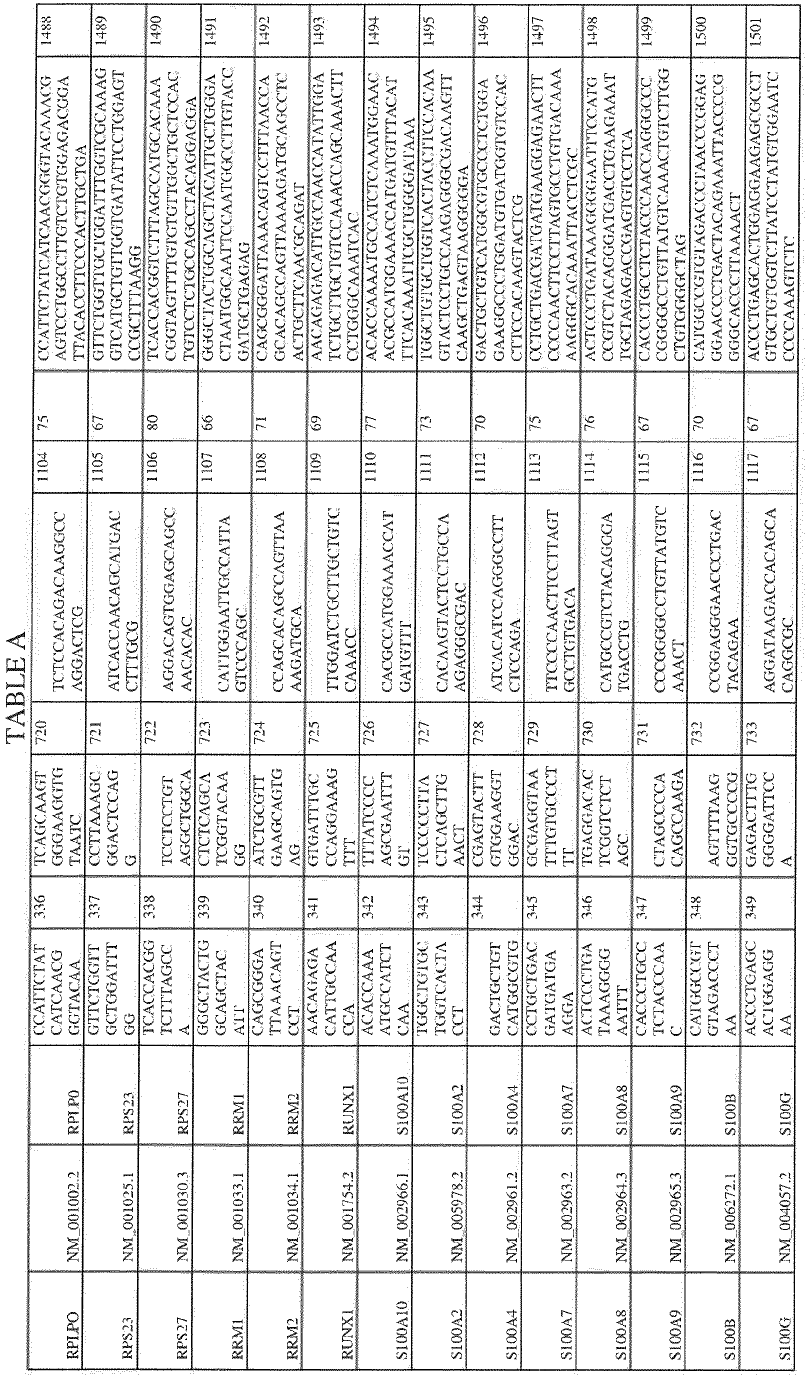

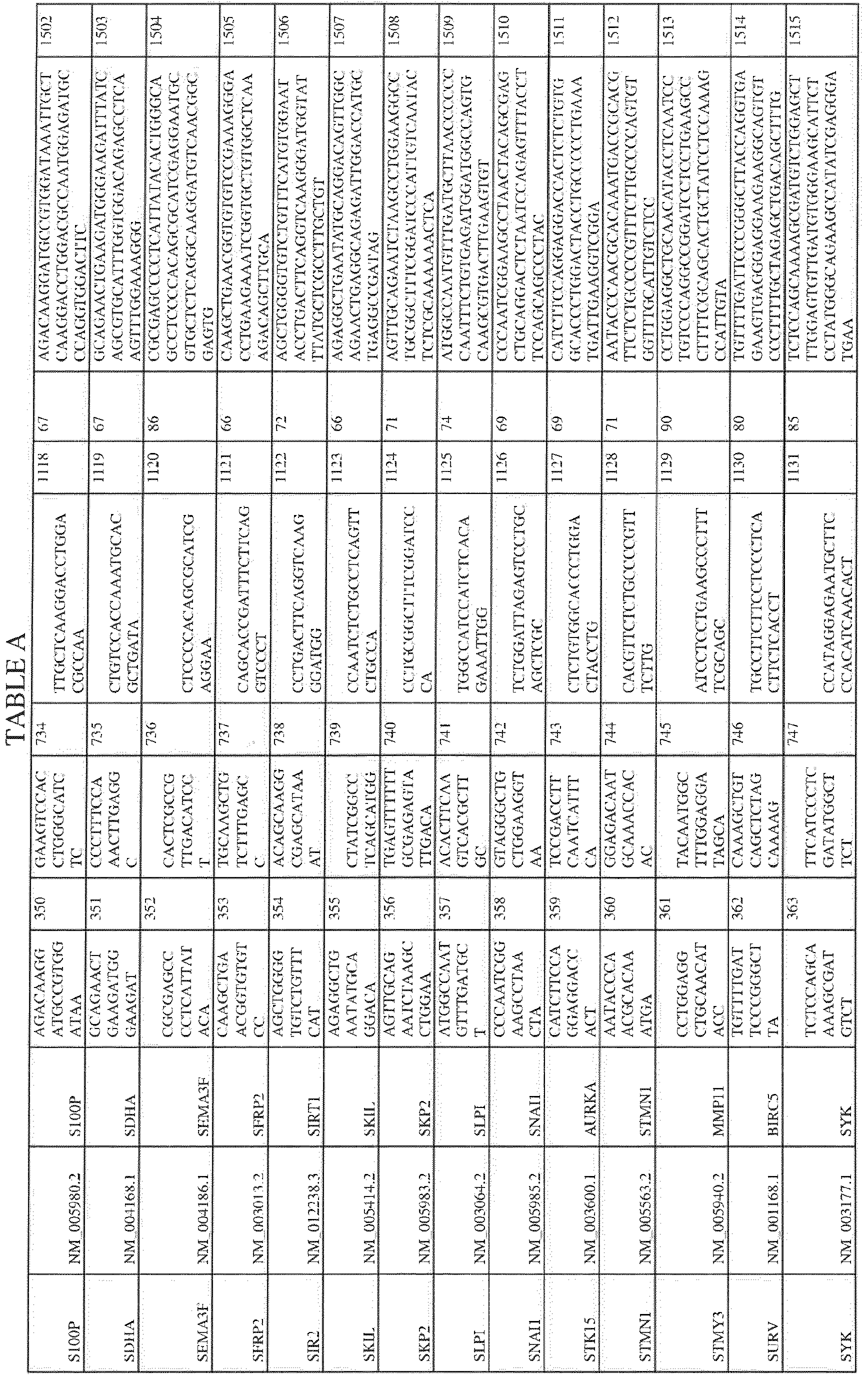

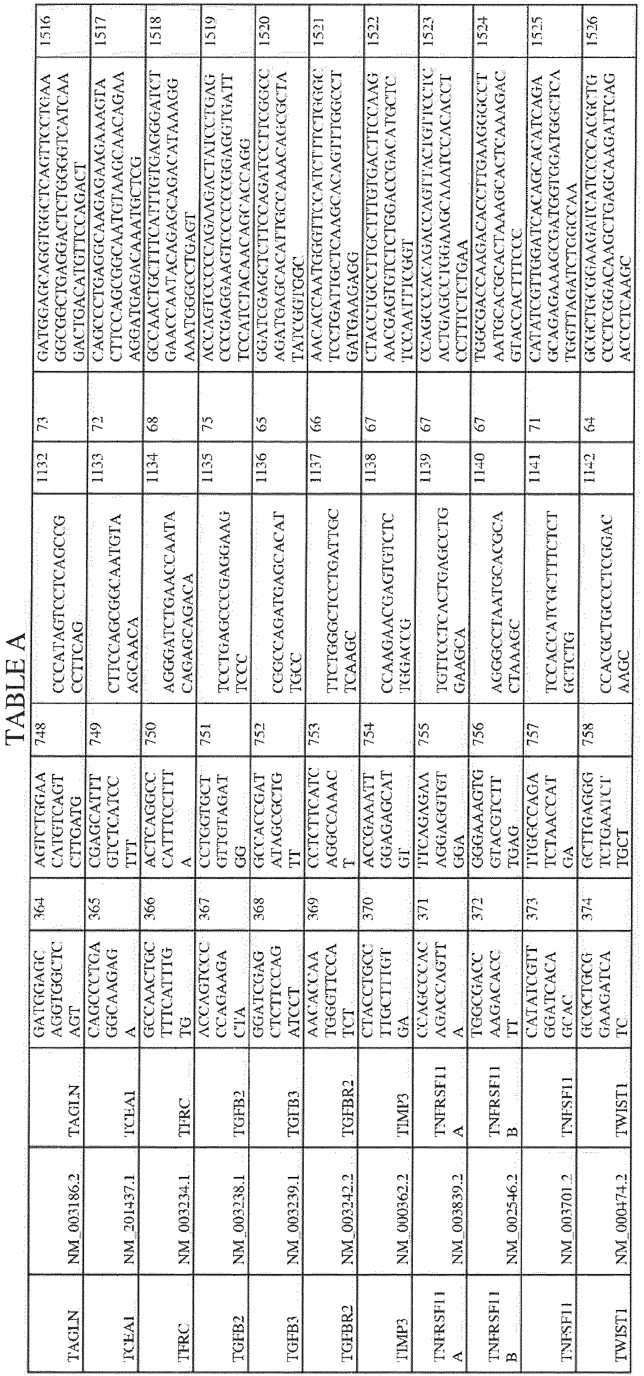

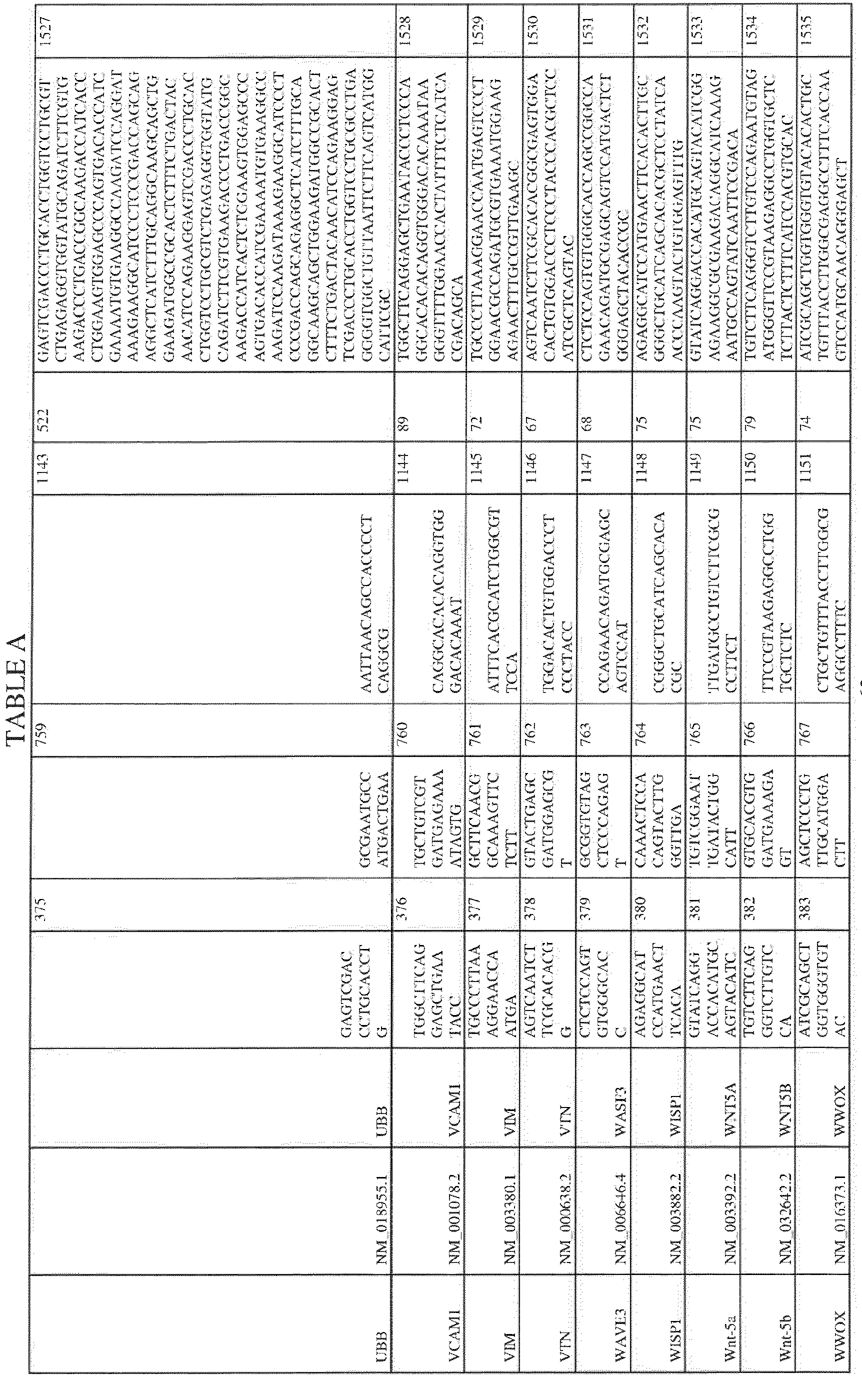

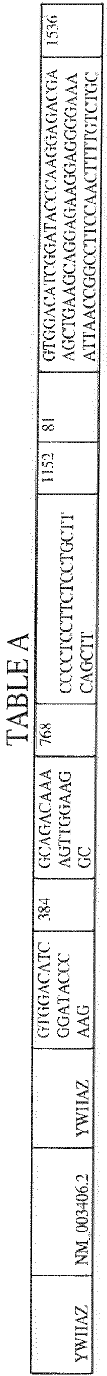

**Table 1: Cox proportional hazards for Prognostic Genes that are positively associated with good prognosis for breast cancer (Providence study)**

| **Table 1** | | | |
|---|---|---|---|
| **Gene_all** | **z (Coef)** | **HR** | **p (Wald)** |
| GSTM2 | -4.306 | 0.525 | 0.000 |
| IL6ST | -3.730 | 0.522 | 0.000 |
| CEGP1 | -3.712 | 0.756 | 0.000 |
| Bcl2 | -3.664 | 0.555 | 0.000 |
| GSTM1 | -3.573 | 0.679 | 0.000 |
| ERBB4 | -3.504 | 0.767 | 0.000 |
| GADD45 | -3.495 | 0.601 | 0.000 |
| PR | -3.474 | 0.759 | 0.001 |
| GPR30 | -3.348 | 0.660 | 0.001 |
| CAV1 | -3.344 | 0.649 | 0.001 |
| C10orf116 | -3.194 | 0.681 | 0.001 |
| DR5 | -3.102 | 0.543 | 0.002 |
| DICER1 | -3.097 | 0.296 | 0.002 |
| EstR1 | -2.983 | 0.825 | 0.003 |
| BTRC | -2.976 | 0.639 | 0.003 |
| GSTM3 | -2.931 | 0.722 | 0.003 |
| GATA3 | -2.874 | 0.745 | 0.004 |
| DLC1 | -2.858 | 0.564 | 0.004 |
| CXCL14 | -2.804 | 0.693 | 0.005 |
| IL17RB | -2.796 | 0.744 | 0.005 |
| C8orf4 | -2.786 | 0.699 | 0.005 |
| FOXO3A | -2.786 | 0.617 | 0.005 |
| TNFRSF11B | -2.690 | 0.739 | 0.007 |
| BAG1 | -2.675 | 0.451 | 0.008 |
| SNAI1 | -2.632 | 0.692 | 0.009 |
| TGFB3 | -2.617 | 0.623 | 0.009 |
| NAT1 | -2.576 | 0.820 | 0.010 |
| FUS | -2.543 | 0.376 | 0.011 |
| F3 | -2.527 | 0.705 | 0.012 |
| GSTM2 gene | -2.461 | 0.668 | 0.014 |
| EPHB2 | -2.451 | 0.708 | 0.014 |
| LAMA3 | -2.448 | 0.778 | 0.014 |
| BAD | -2.425 | 0.506 | 0.015 |
| IGF1R | -2.378 | 0.712 | 0.017 |
| RUNX1 | -2.356 | 0.511 | 0.018 |
| ESRRG | -2.289 | 0.825 | 0.022 |
| HSHIN1 | -2.275 | 0.371 | 0.023 |
| CXCL12 | -2.151 | 0.623 | 0.031 |
| IGFBP7 | -2.137 | 0.489 | 0.033 |
| SKIL | -2.121 | 0.593 | 0.034 |
| PTEN | -2.110 | 0.449 | 0.035 |
| AKT3 | -2.104 | 0.665 | 0.035 |
| MGMT | -2.060 | 0.571 | 0.039 |
| LRIG1 | -2.054 | 0.649 | 0.040 |
| S100B | -2.024 | 0.798 | 0.043 |
| GREB1 variant a | -1.996 | 0.833 | 0.046 |
| CSF1 | -1.976 | 0.624 | 0.048 |
| ABR | -1.973 | 0.575 | 0.048 |
| AK055699 | -1.972 | 0.790 | 0.049 |

**Table 2: Cox proportional hazards for Prognostic Genes that are negatively associated with good prognosis for breast cancer (Providence study)**

| **Table 2** | | | |
|---|---|---|---|
| **Gene_all** | **z (Coef)** | **HR** | **p (Wald)** |
| S100A7 | 1.965 | 1.100 | 0.049 |
| MCM2 | 1.999 | 1.424 | 0.046 |
| Contig 51037 | 2.063 | 1.185 | 0.039 |
| S100P | 2.066 | 1.170 | 0.039 |
| ACTR2 | 2.119 | 2.553 | 0.034 |
| MYBL2 | 2.158 | 1.295 | 0.031 |
| DUSP1 | 2.166 | 1.330 | 0.030 |
| HOXB13 | 2.192 | 1.206 | 0.028 |
| SURV | 2.216 | 1.329 | 0.027 |
| MELK | 2.234 | 1.336 | 0.026 |
| HSPA8 | 2.240 | 2.651 | 0.025 |
| cdc25A | 2.314 | 1.478 | 0.021 |
| C20_orf1 | 2.336 | 1.497 | 0.019 |
| LMNB1 | 2.387 | 1.682 | 0.017 |
| S100A9 | 2.412 | 1.185 | 0.016 |
| CENPA | 2.419 | 1.366 | 0.016 |
| CDC25C | 2.437 | 1.384 | 0.015 |
| GAPDH | 2.498 | 1.936 | 0.012 |
| KNTC2 | 2.512 | 1.450 | 0.012 |
| PRDX1 | 2.540 | 2.131 | 0.011 |
| RRM2 | 2.547 | 1.439 | 0.011 |
| ADM | 2.590 | 1.445 | 0.010 |
| ARF1 | 2.634 | 2.973 | 0.008 |
| E2F1 | 2.716 | 1.486 | 0.007 |
| TFRC | 2.720 | 1.915 | 0.007 |
| STK15 | 2.870 | 1.860 | 0.004 |
| LAPTM4B | 2.880 | 1.538 | 0.004 |
| EpCAM | 2.909 | 1.919 | 0.004 |
| ENO1 | 2.958 | 2.232 | 0.003 |
| CCNB1 | 3.003 | 1.738 | 0.003 |
| BUB1 | 3.018 | 1.590 | 0.003 |
| Claudin 4 | 3.034 | 2.151 | 0.002 |
| CDC20 | 3.056 | 1.555 | 0.002 |
| Ki-67 | 3.329 | 1.717 | 0.001 |
| KPNA2 | 3.523 | 1.722 | 0.000 |
| IDH2 | 3.994 | 1.638 | 0.000 |

**Table 3: Cox proportional hazards for Prognostic Genes that are positively associated with good prognosis for ER-negative (ERO) breast cancer (Providence study)**

| **Gene_ER0** | **HR** | **z (Coef)** | **p (Wald)** |
|---|---|---|---|
| SYK | 0.185 | -2.991 | 0.003 |
| Wnt-5a | 0.443 | -2.842 | 0.005 |
| WISP1 | 0.455 | -2.659 | 0.008 |
| CYR61 | 0.405 | -2.484 | 0.013 |
| GADD45 | 0.520 | -2.474 | 0.013 |
| TAGLN | 0.364 | -2.376 | 0.018 |
| TGFB3 | 0.465 | -2.356 | 0.018 |
| INHBA | 0.610 | -2.255 | 0.024 |
| CDH11 | 0.584 | -2.253 | 0.024 |
| CHAF1B | 0.551 | -2.113 | 0.035 |
| ITGAV | 0.192 | -2.101 | 0.036 |
| SNAI1 | 0.655 | -2.077 | 0.038 |
| IL11 | 0.624 | -2.026 | 0.043 |
| KIAA1199 | 0.692 | -2.005 | 0.045 |
| TNFRSF 11B | 0.659 | -1.989 | 0.047 |

**Table 4: Cox proportional hazards for Prognostic Genes that are negatively associated with good prognosis for ER-negative (ERO) breast cancer (Providence study)**

| **Gene_ER0** | **HR** | **z (Coef)** | **p (Wald)** |
|---|---|---|---|
| RPL41 | 3.547 | 2.062 | 0.039 |
| Claudin 4 | 2.883 | 2.117 | 0.034 |
| LYRIC | 4.029 | 2.364 | 0.018 |
| TFRC | 3.223 | 2.596 | 0.009 |
| VTN | 2.484 | 3.205 | 0.001 |

**Table 5: Cox proportional hazards for Prognostic Genes that are positively associated with good prognosis for ER-positive (ER1) breast cancer (Providence study)**

| **Gene_ER1** | **HR** | **z (Coef)** | **p (Wald)** |
|---|---|---|---|
| DR5 | 0.428 | -3.478 | 0.001 |
| GSTM2 | 0.526 | -3.173 | 0.002 |
| HSHIN1 | 0.175 | -3.031 | 0.002 |
| ESRRG | 0.736 | -3.028 | 0.003 |
| VTN | 0.622 | -2.935 | 0.003 |
| Bcl2 | 0.469 | -2.833 | 0.005 |
| ERBB4 | 0.705 | -2.802 | 0.005 |
| GPR30 | 0.625 | -2.794 | 0.005 |
| BAG1 | 0.339 | -2.733 | 0.006 |
| CAV1 | 0.635 | -2.644 | 0.008 |
| IL6ST | 0.503 | -2.551 | 0.011 |
| C10orf116 | 0.679 | -2.497 | 0.013 |
| FOXO3A | 0.607 | -2.473 | 0.013 |
| DICER1 | 0.311 | -2.354 | 0.019 |
| GADD45 | 0.645 | -2.338 | 0.019 |
| CSF1 | 0.500 | -2.312 | 0.021 |
| F3 | 0.677 | -2.300 | 0.021 |
| GBP2 | 0.604 | -2.294 | 0.022 |
| APEX-1 | 0.234 | -2.253 | 0.024 |
| FUS | 0.322 | -2.252 | 0.024 |
| BBC3 | 0.581 | -2.248 | 0.025 |
| GSTM3 | 0.737 | -2.203 | 0.028 |
| ITGA4 | 0.620 | -2.161 | 0.031 |
| EPHB2 | 0.685 | -2.128 | 0.033 |
| IRF1 | 0.708 | -2.105 | 0.035 |
| CRYZ | 0.593 | -2.103 | 0.035 |
| CCL19 | 0.773 | -2.076 | 0.038 |
| SKIL | 0.540 | -2.019 | 0.043 |
| MRP1 | 0.515 | -1.964 | 0.050 |

**Table 6: Cox proportional hazards for Prognostic Genes that are negatively associated with good prognosis for ER-positive (ER1) breast cancer (Providence study)**

| **Gene_ER1** | **HR** | **z (Coef)** | **p (Wald)** |
|---|---|---|---|
| CTHRC1 | 2.083 | 1.958 | 0.050 |
| RRM2 | 1.450 | 1.978 | 0.048 |
| BUB1 | 1.467 | 1.988 | 0.047 |
| LMNB1 | 1.764 | 2.009 | 0.045 |
| SURV | 1.380 | 2.013 | 0.044 |
| EpCAM | 1.966 | 2.076 | 0.038 |
| CDC20 | 1.504 | 2.081 | 0.037 |
| GAPDH | 2.405 | 2.126 | 0.033 |
| STK15 | 1.796 | 2.178 | 0.029 |
| HSPA8 | 3.095 | 2.215 | 0.027 |
| LAPTM4B | 1.503 | 2.278 | 0.023 |
| MCM2 | 1.872 | 2.370 | 0.018 |
| CDC25C | 1.485 | 2.423 | 0.015 |
| ADM | 1.695 | 2.486 | 0.013 |
| MMP1 | 1.365 | 2.522 | 0.012 |
| CCNB1 | 1.893 | 2.646 | 0.008 |
| Ki-67 | 1.697 | 2.649 | 0.008 |
| E2F1 | 1.662 | 2.689 | 0.007 |
| KPNA2 | 1.683 | 2.701 | 0.007 |
| DUSP1 | 1.573 | 2.824 | 0.005 |
| GDF15 | 1.440 | 2.896 | 0.004 |

**Table 7: Cox proportional hazards for Prognostic Genes that are positively associated with good prognosis for breast cancer (Rush study)**

| **Gene_all** | **z (Coef)** | **HR** | **p (Wald)** |
|---|---|---|---|
| GSTM2 | -3.275 | 0.752 | 0.001 |
| GSTM1 | -2.946 | 0.772 | 0.003 |
| C8orf4 | -2.639 | 0.793 | 0.008 |
| ELF3 | -2.478 | 0.769 | 0.013 |
| RUNX1 | -2.388 | 0.609 | 0.017 |
| IL6ST | -2.350 | 0.738 | 0.019 |
| AAMP | -2.325 | 0.715 | 0.020 |
| PR | -2.266 | 0.887 | 0.023 |
| FHIT | -2.193 | 0.790 | 0.028 |
| CD44v6 | -2.191 | 0.754 | 0.028 |
| GREB1 variant c | -2.120 | 0.874 | 0.034 |
| ADAM17 | -2.101 | 0.686 | 0.036 |
| EstR1 | -2.084 | 0.919 | 0.037 |
| NAT1 | -2.081 | 0.878 | 0.037 |
| TNFRSF11B | -2.074 | 0.843 | 0.038 |
| ITGB4 | -2.006 | 0.740 | 0.045 |
| CSF1 | -1.963 | 0.750 | 0.050 |

**Table 8: Cox proportional hazards for Prognostic Genes that are negatively associated with good prognosis for breast cancer (Rush study)**

| **Gene_all** | **z (Coef)** | **HR** | **p (Wald)** |
|---|---|---|---|
| STK15 | 1.968 | 1.298 | 0.049 |
| TFRC | 2.049 | 1.399 | 0.040 |
| ITGB1 | 2.071 | 1.812 | 0.038 |
| ITGAV | 2.081 | 1.922 | 0.037 |
| MYBL2 | 2.089 | 1.205 | 0.037 |
| MRP3 | 2.092 | 1.165 | 0.036 |
| SKP2 | 2.143 | 1.379 | 0.032 |
| LMNB1 | 2.155 | 1.357 | 0.031 |
| ALCAM | 2.234 | 1.282 | 0.025 |
| COMT | 2.271 | 1.412 | 0.023 |
| CDC20 | 2.300 | 1.253 | 0.021 |
| GAPDH | 2.307 | 1.572 | 0.021 |
| GRB7 | 2.340 | 1.205 | 0.019 |
| S100A9 | 2.374 | 1.120 | 0.018 |
| S100A7 | 2.374 | 1.092 | 0.018 |
| HER2 | 2.425 | 1.210 | 0.015 |
| ACTR2 | 2.499 | 1.788 | 0.012 |
| S100A8 | 2.745 | 1.144 | 0.006 |
| ENO1 | 2.752 | 1.687 | 0.006 |
| MMP1 | 2.758 | 1.212 | 0.006 |
| LAPTM4B | 2.775 | 1.375 | 0.006 |
| FGFR4 | 3.005 | 1.215 | 0.003 |
| C17orf37 | 3.260 | 1.387 | 0.001 |

**Table 9: Cox proportional hazards for Prognostic Genes that are positively associated with good prognosis for ER-negative (ERO) breast cancer (Rush study)**

| **Gene_ER0** | **z (Coef)** | **HR** | **p (Wald)** |
|---|---|---|---|
| SEMA3F | -2.465 | 0.503 | 0.014 |
| LAMA3 | -2.461 | 0.519 | 0.014 |
| CD44E | -2.418 | 0.719 | 0.016 |
| AD024 | -2.256 | 0.617 | 0.024 |
| LAMB3 | -2.237 | 0.690 | 0.025 |
| Ki-67 | -2.209 | 0.650 | 0.027 |
| MMP7 | -2.208 | 0.768 | 0.027 |
| GREB1 variant c | -2.019 | 0.693 | 0.044 |
| ITGB4 | -1.996 | 0.657 | 0.046 |
| CRYZ | -1.976 | 0.662 | 0.048 |
| CD44s | -1.967 | 0.650 | 0.049 |

**Table 10: Cox proportional hazards for Prognostic Genes that are negatively associated with good prognosis for ER-negative (ERO) breast cancer (Rush study)**

| **Gene_ER0** | **z (Coef)** | **HR** | **p(Wald)** |
|---|---|---|---|
| S100A8 | 1.972 | 1.212 | 0.049 |
| EEF1A2 | 2.031 | 1.195 | 0.042 |
| TAGLN | 2.072 | 2.027 | 0.038 |
| GRB7 | 2.086 | 1.231 | 0.037 |
| HER2 | 2.124 | 1.232 | 0.034 |
| ITGAV | 2.217 | 3.258 | 0.027 |
| CDH11 | 2.237 | 2.728 | 0.025 |
| COL1A1 | 2.279 | 2.141 | 0.023 |
| C17orf37 | 2.319 | 1.329 | 0.020 |
| COL1A2 | 2.336 | 2.577 | 0.020 |
| ITGB5 | 2.375 | 3.236 | 0.018 |
| ITGA5 | 2.422 | 2.680 | 0.015 |
| RPL41 | 2.428 | 6.665 | 0.015 |
| ALCAM | 2.470 | 1.414 | 0.013 |
| CTHRC1 | 2.687 | 3.454 | 0.007 |
| PTEN | 2.692 | 8.706 | 0.007 |
| FN1 | 2.833 | 2.206 | 0.005 |

**Table 11: Cox proportional hazards for Prognostic Genes that are positively associated with good prognosis for ER-positive (ER1) breast cancer (Rush study)**

| **Gene_ER1** | **z (Coef)** | **HR** | **p (Wald)** |
|---|---|---|---|
| GSTM1 | -3.938 | 0.628 | 0.000 |
| HNF3A | -3.220 | 0.500 | 0.001 |
| EstR1 | -3.165 | 0.643 | 0.002 |
| Bcl2 | -2.964 | 0.583 | 0.003 |
| GATA3 | -2.641 | 0.624 | 0.008 |
| ELF3 | -2.579 | 0.741 | 0.010 |
| C8orf4 | -2.451 | 0.730 | 0.014 |
| GSTM2 | -2.416 | 0.774 | 0.016 |
| PR | -2.416 | 0.833 | 0.016 |
| RUNX1 | -2.355 | 0.537 | 0.019 |
| CSF1 | -2.261 | 0.662 | 0.024 |
| IL6ST | -2.239 | 0.627 | 0.025 |
| AAMP | -2.046 | 0.704 | 0.041 |
| TNFRSF11B | -2.028 | 0.806 | 0.043 |
| NAT1 | -2.025 | 0.833 | 0.043 |
| ADAM17 | -1.981 | 0.642 | 0.048 |

**Table 12: Cox proportional hazards for Prognostic Genes that are negatively associated with good prognosis for ER-positive (ER1) breast cancer (Rush study)**

| **Gene_ER1** | **z (Coef)** | **HR** | **p (Wald)** |
|---|---|---|---|
| HSPA1B | 1.966 | 1.382 | 0.049 |
| AD024 | 1.967 | 1.266 | 0.049 |
| FGFR4 | 1.991 | 1.175 | 0.047 |
| CDK4 | 2.014 | 1.576 | 0.044 |
| ITGB1 | 2.021 | 2.163 | 0.043 |
| EPHB2 | 2.121 | 1.342 | 0.034 |
| LYRIC | 2.139 | 1.583 | 0.032 |
| MYBL2 | 2.174 | 1.273 | 0.030 |
| PGF | 2.176 | 1.439 | 0.030 |
| EZH2 | 2.199 | 1.390 | 0.028 |
| HSPA1A | 2.209 | 1.452 | 0.027 |
| RPLPO | 2.273 | 2.824 | 0.023 |
| LMNB1 | 2.322 | 1.529 | 0.020 |
| IL-8 | 2.404 | 1.166 | 0.016 |
| C6orf66 | 2.468 | 1.803 | 0.014 |
| GAPDH | 2.489 | 1.950 | 0.013 |
| P16-INK4 | 2.490 | 1.541 | 0.013 |
| CLIC1 | 2.557 | 2.745 | 0.011 |
| ENO1 | 2.719 | 2.455 | 0.007 |
| ACTR2 | 2.878 | 2.543 | 0.004 |
| CDC20 | 2.931 | 1.452 | 0.003 |
| SKP2 | 2.952 | 1.916 | 0.003 |
| LAPTM4B | 3.124 | 1.558 | 0.002 |

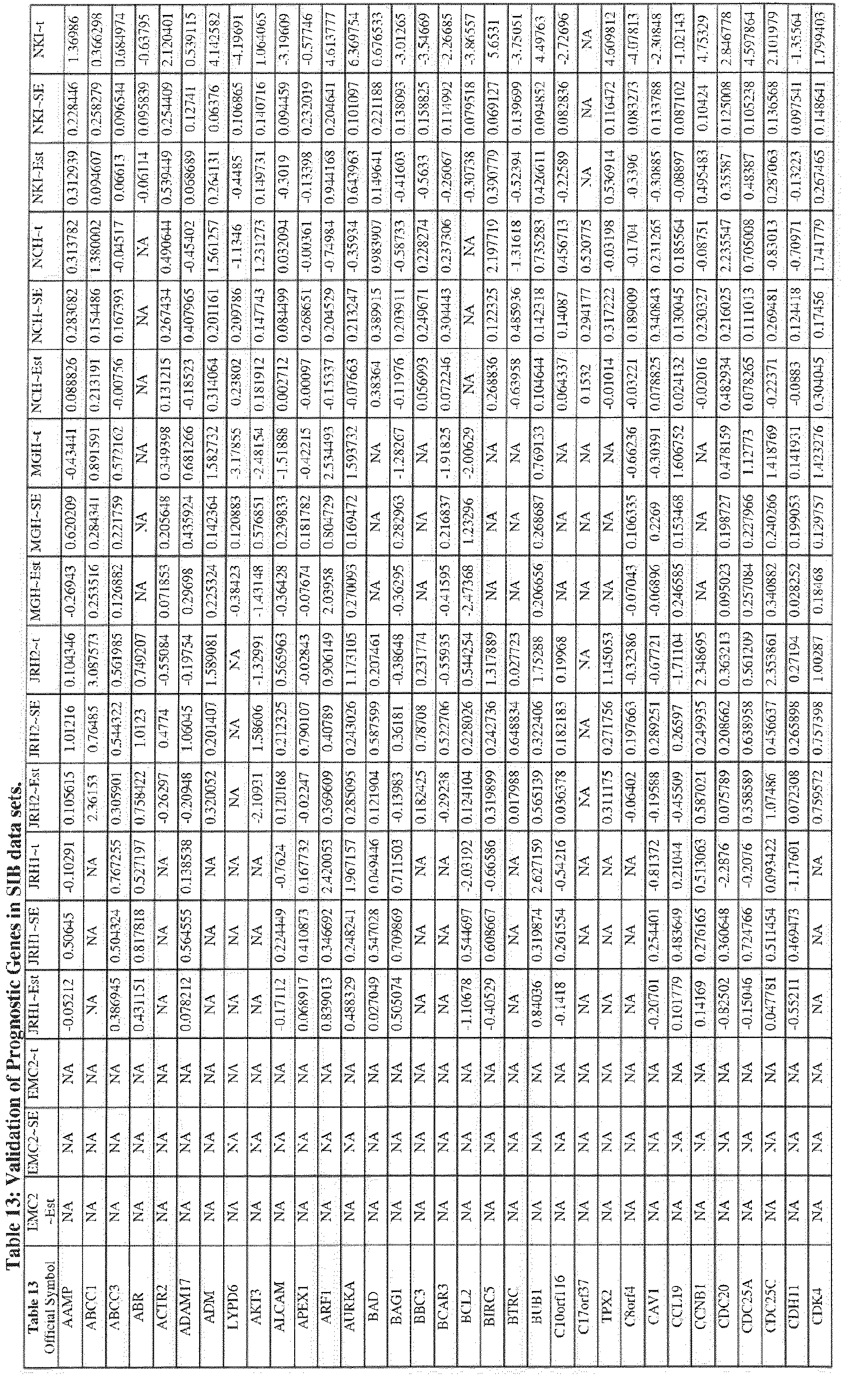

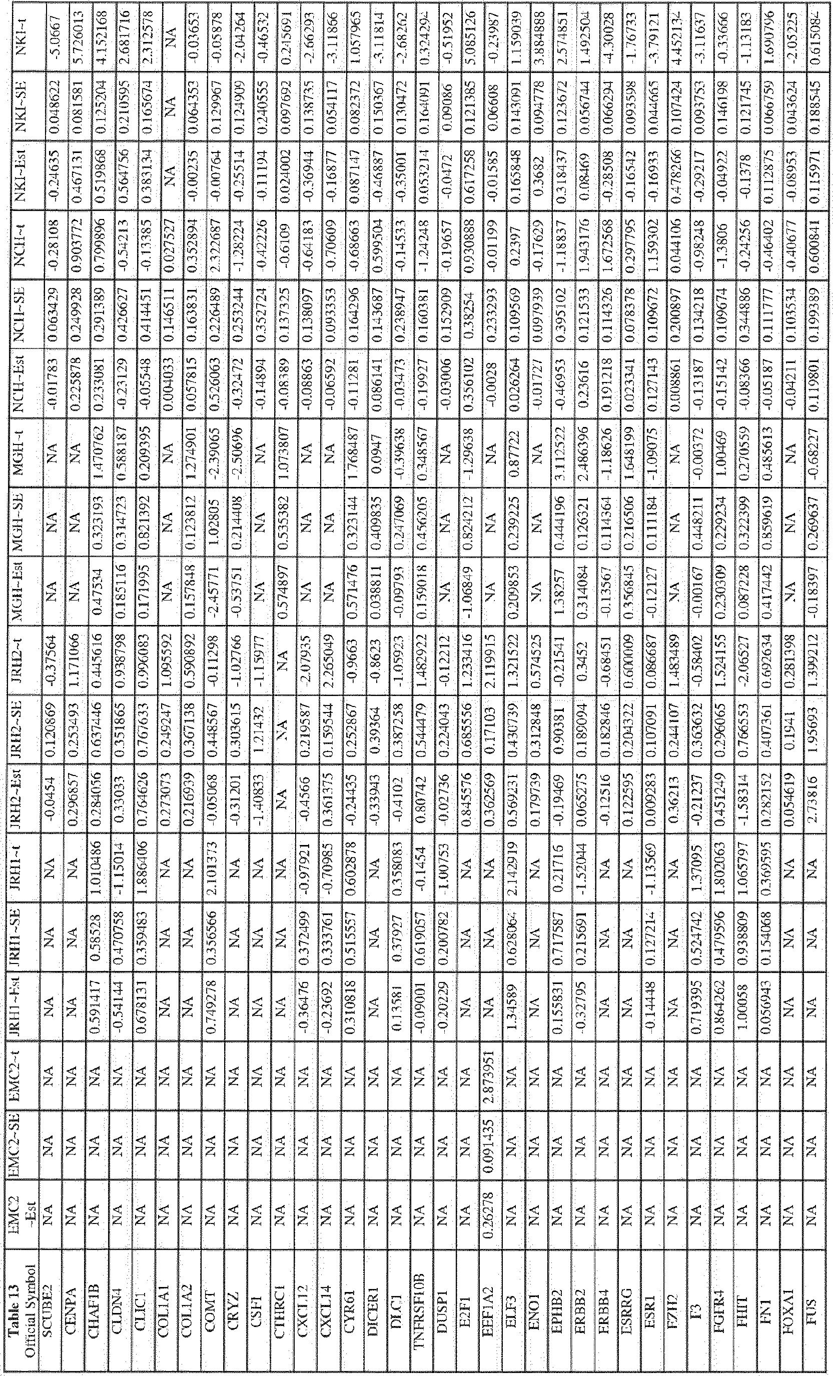

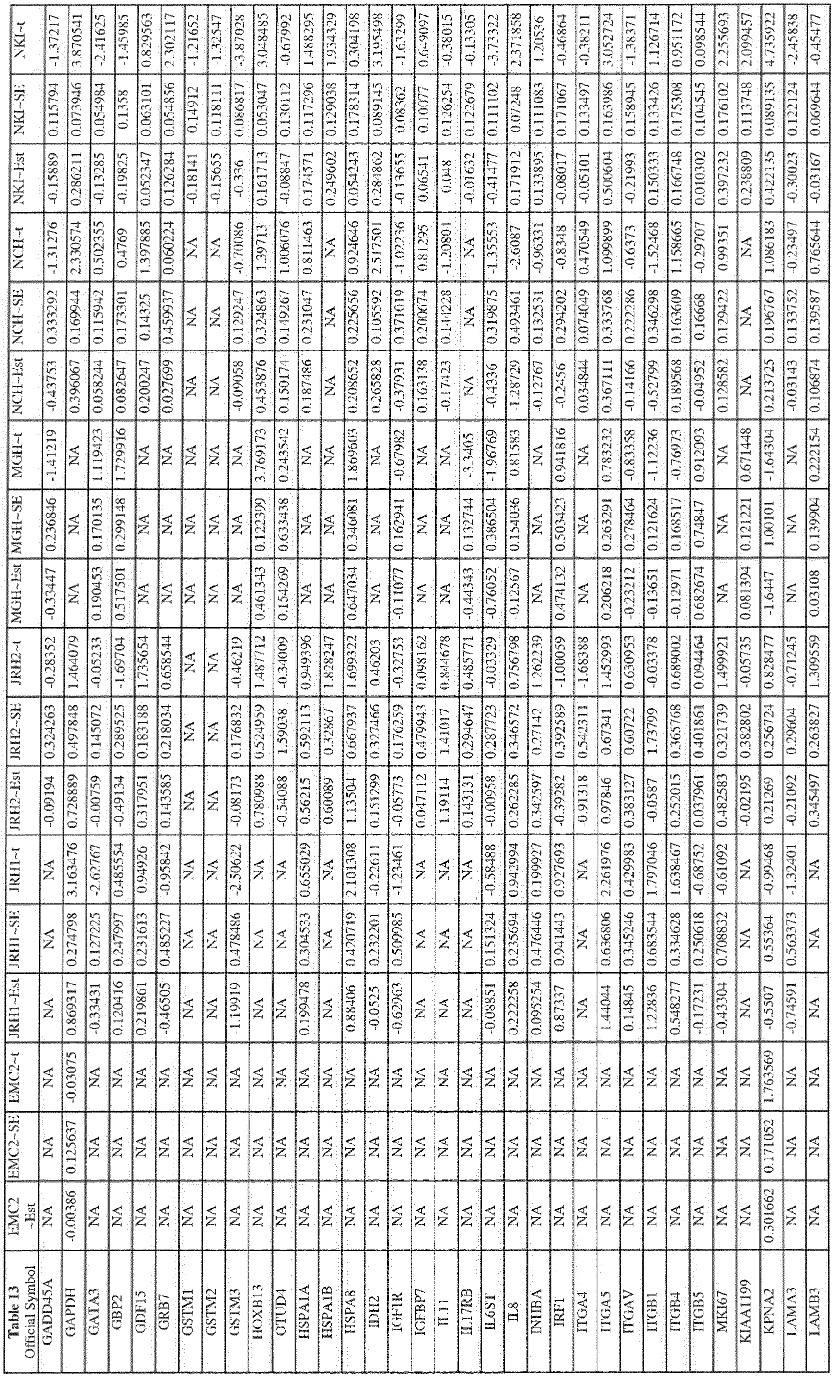

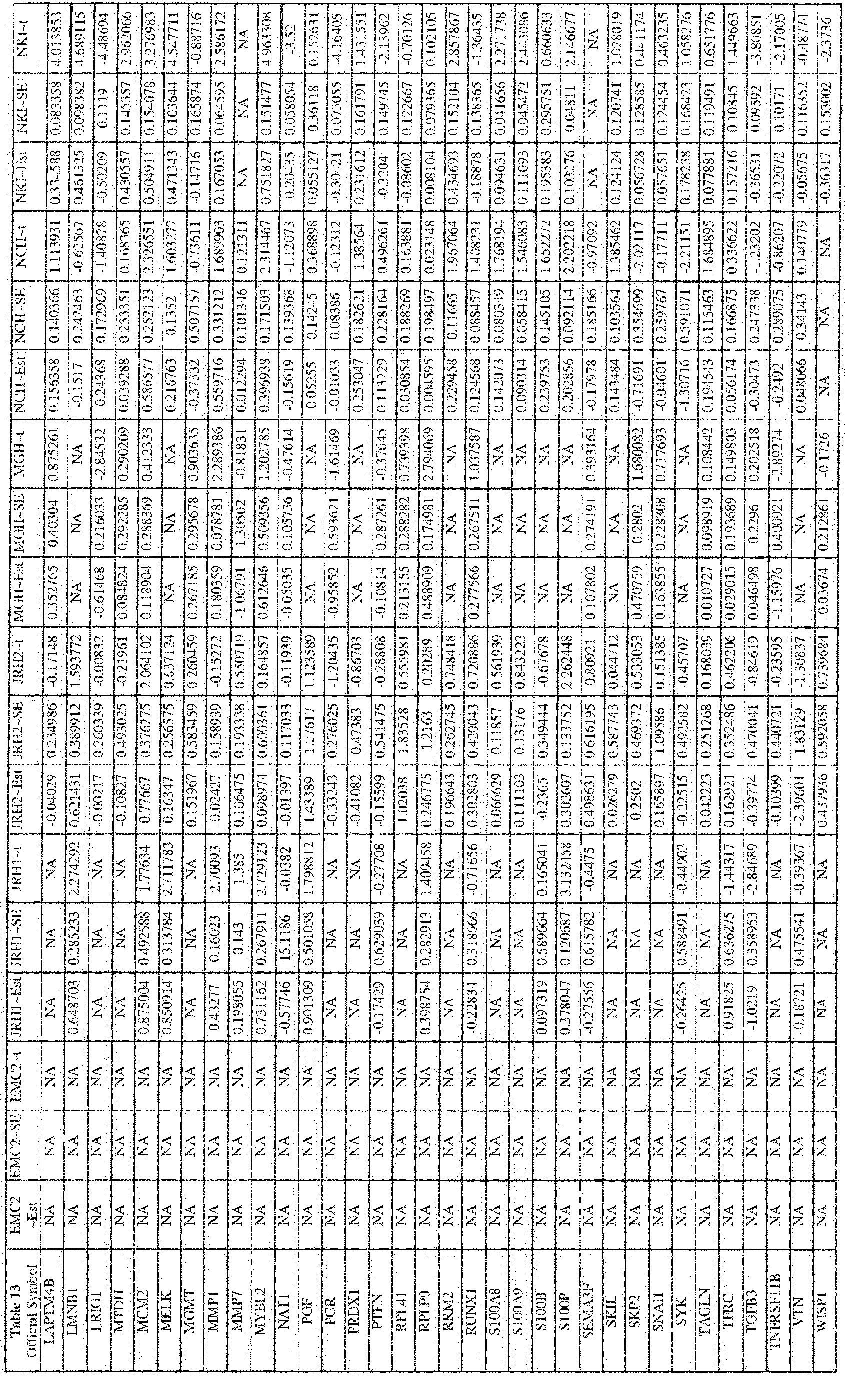

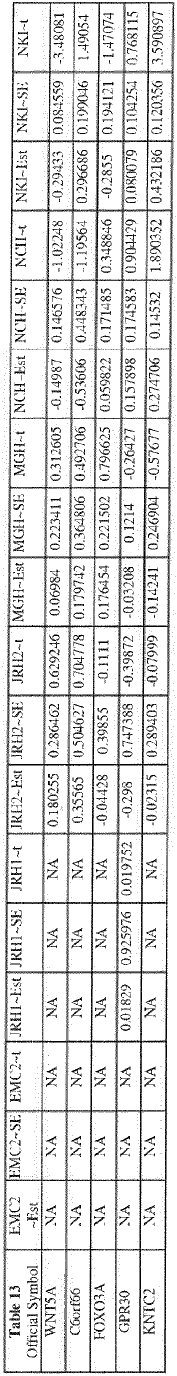

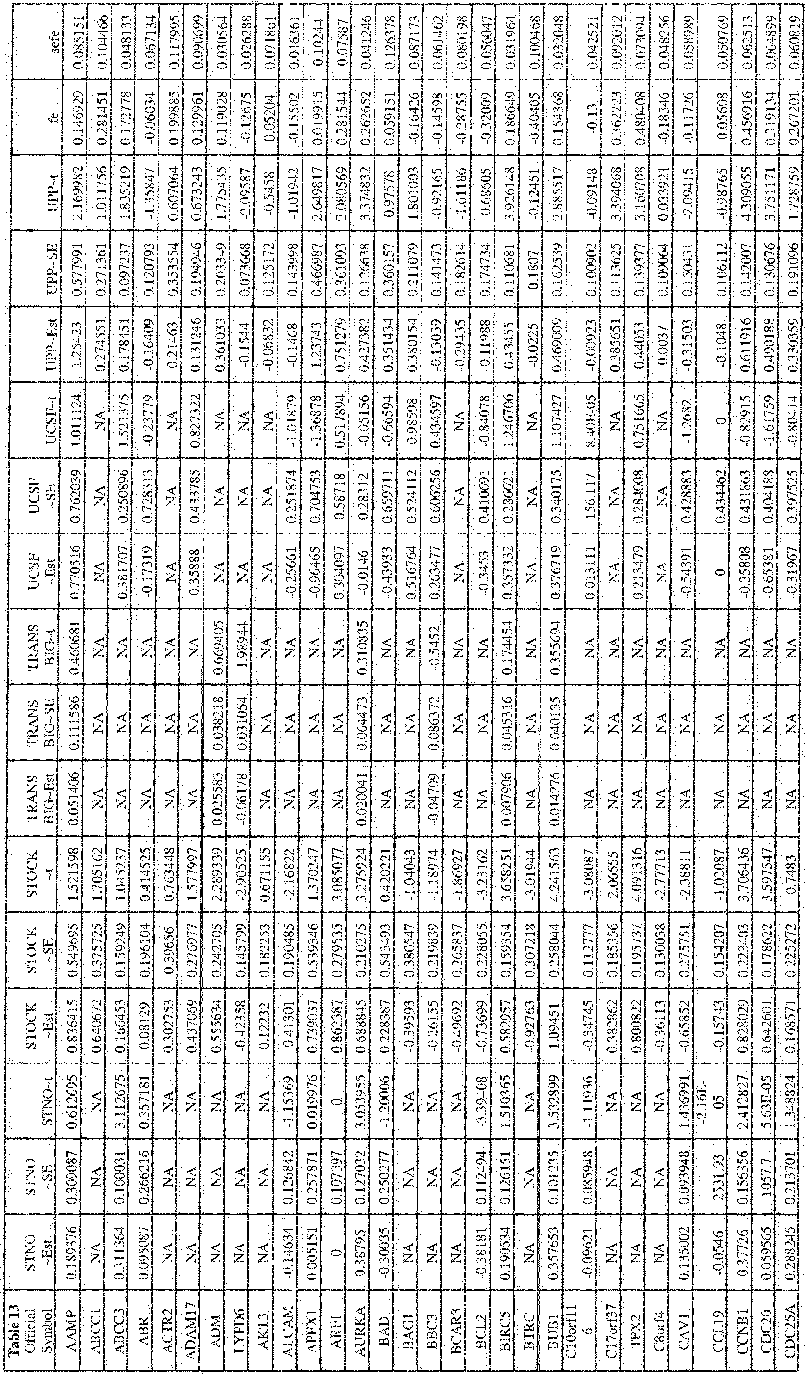

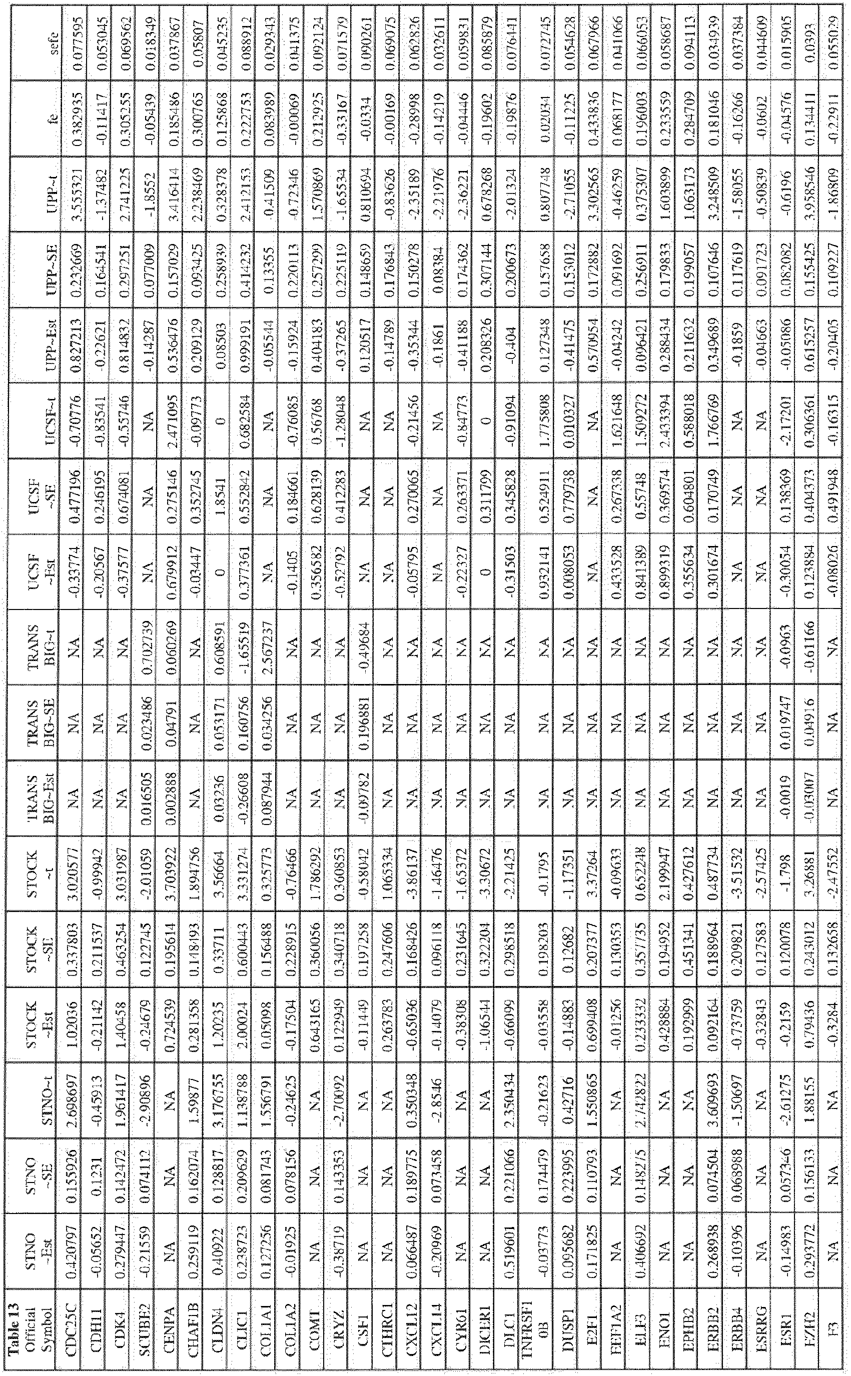

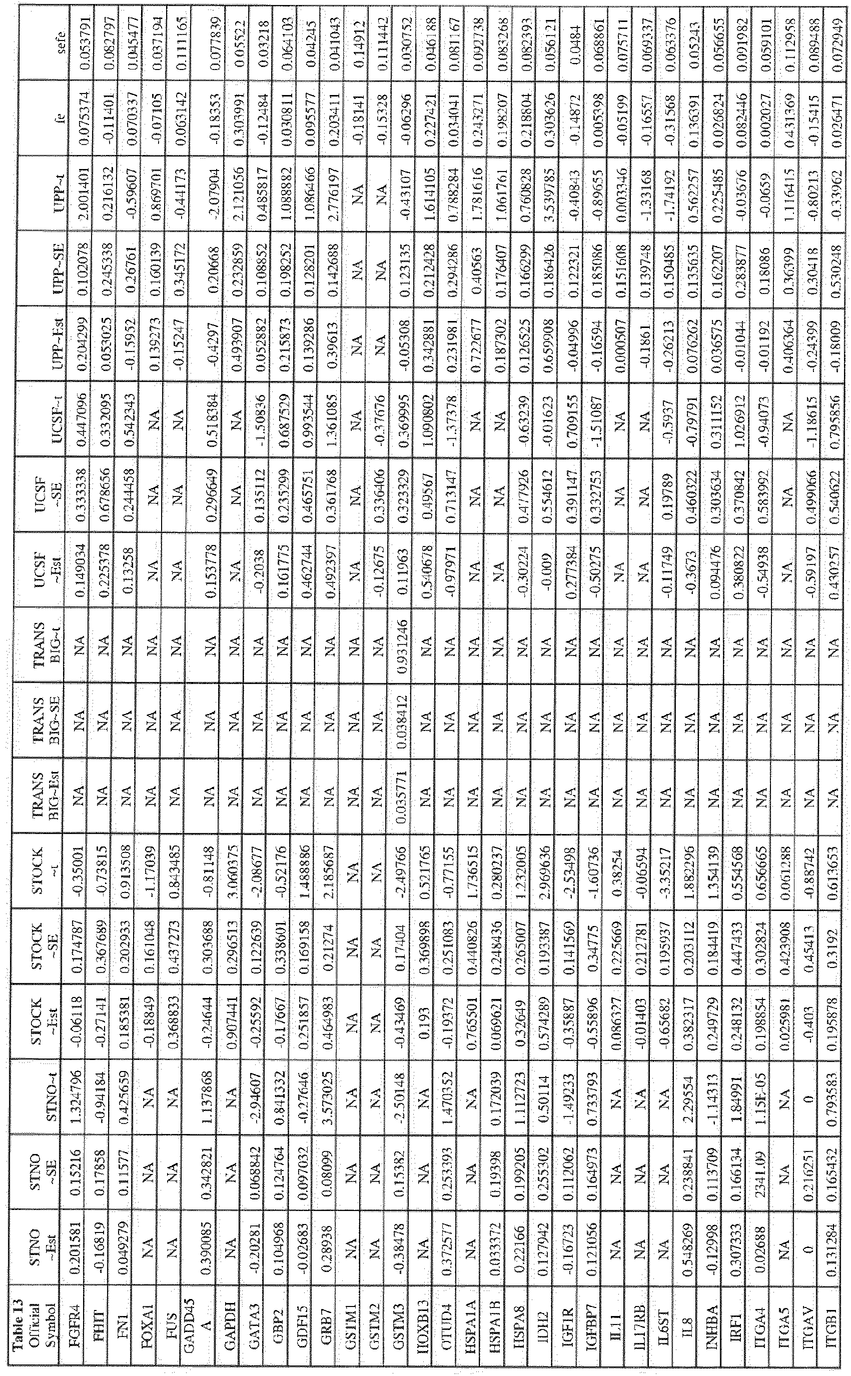

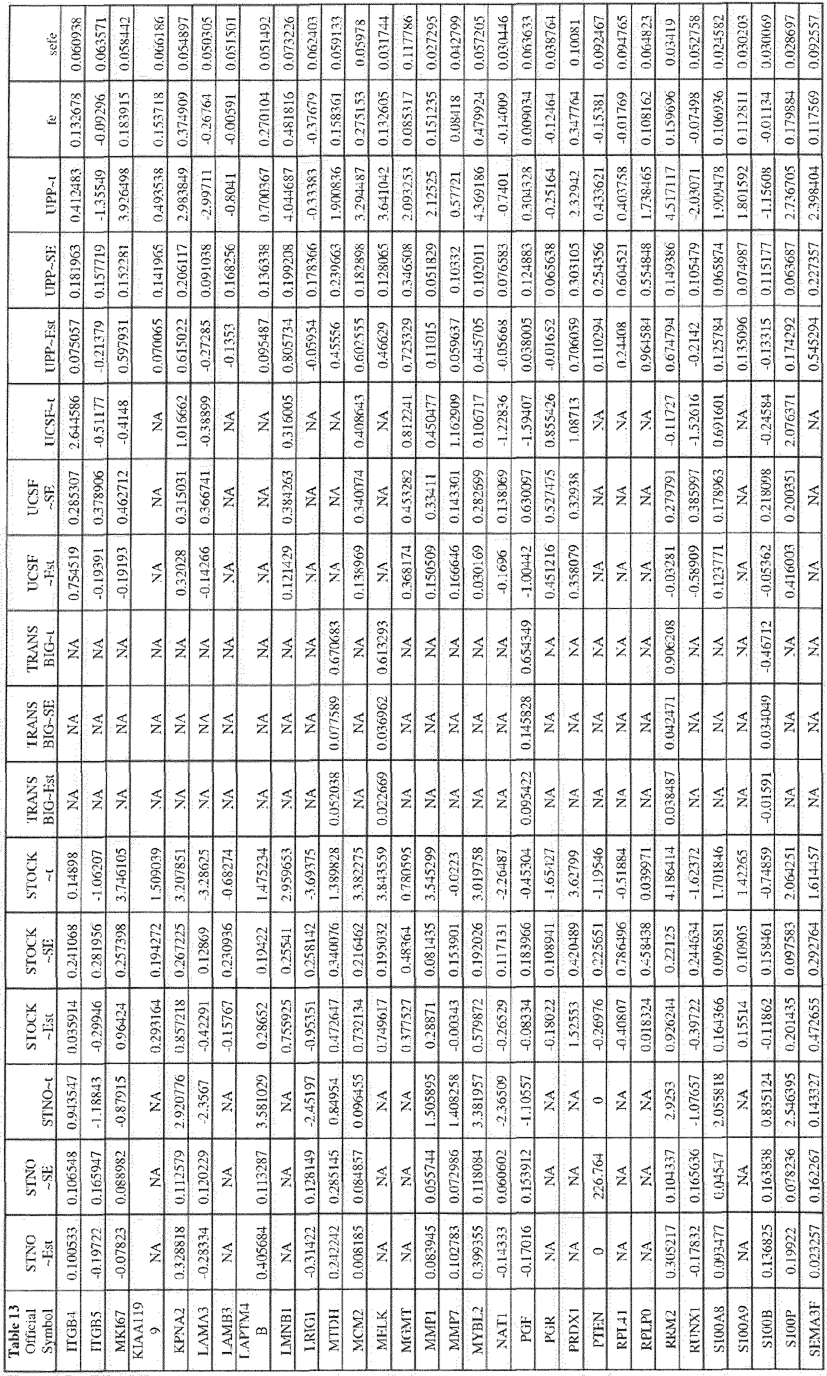

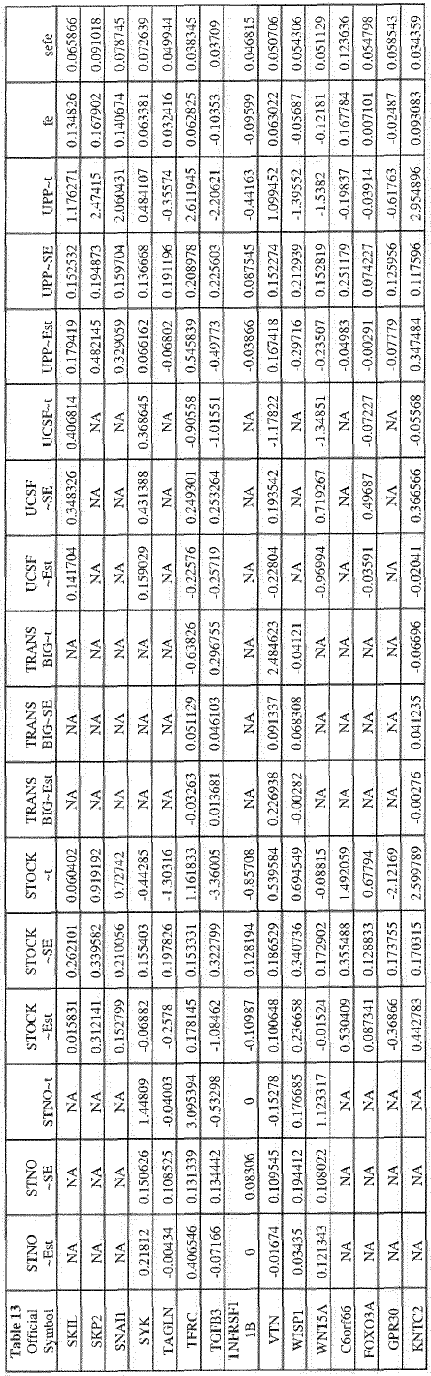

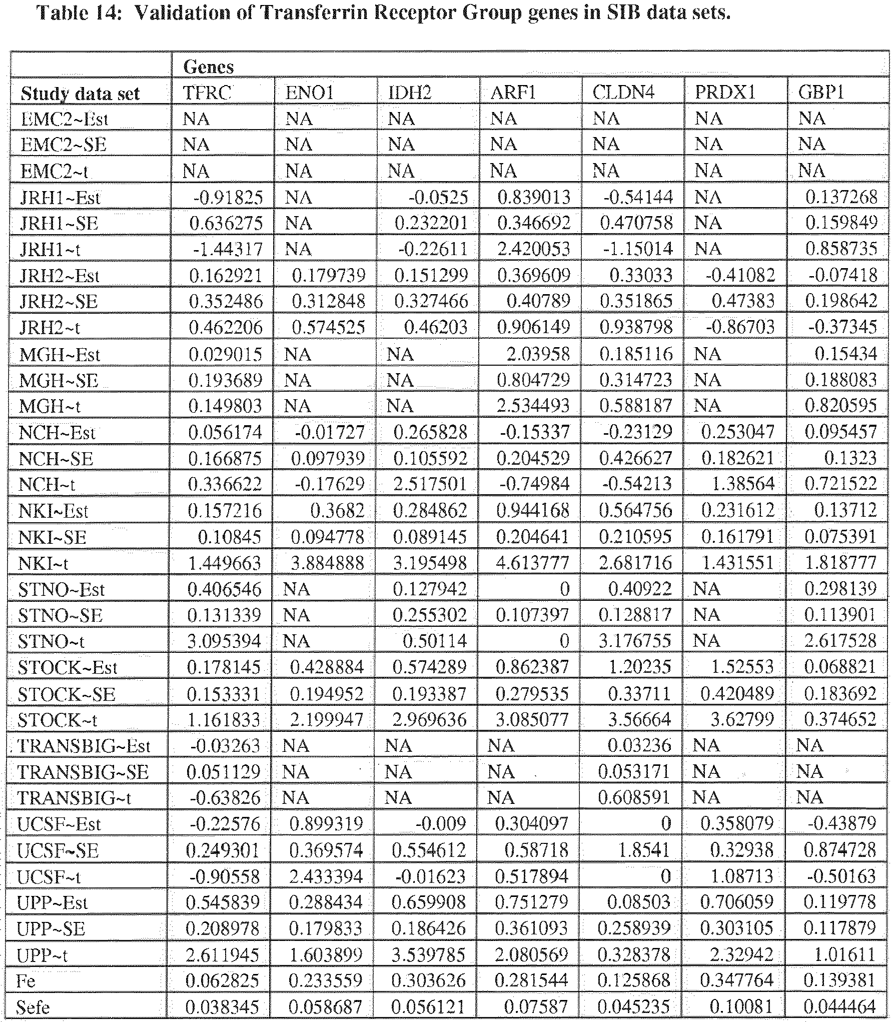

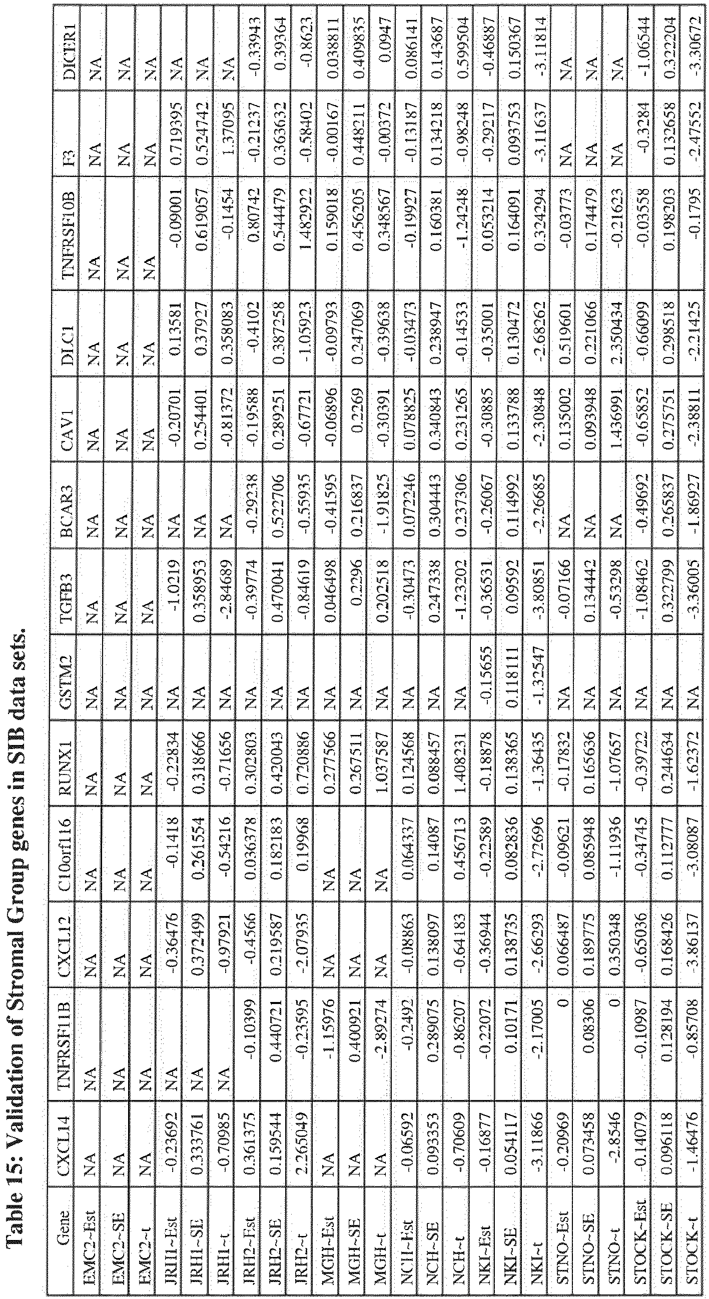

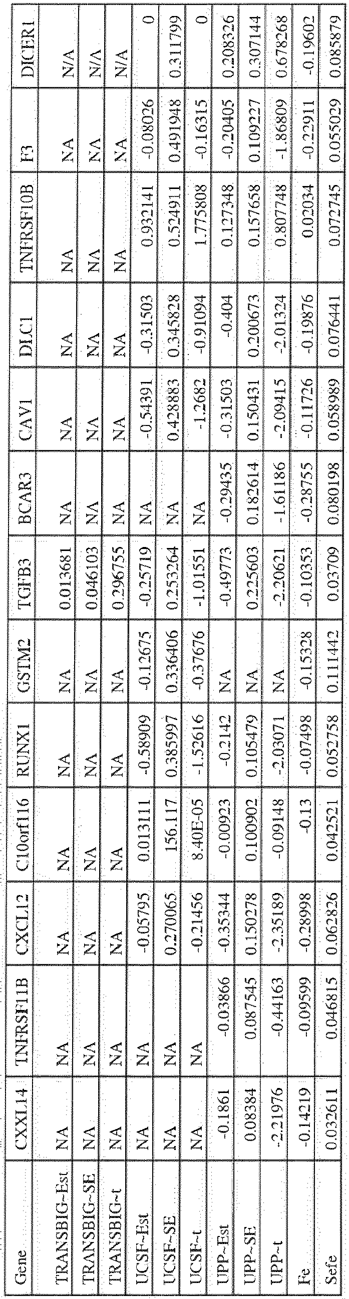

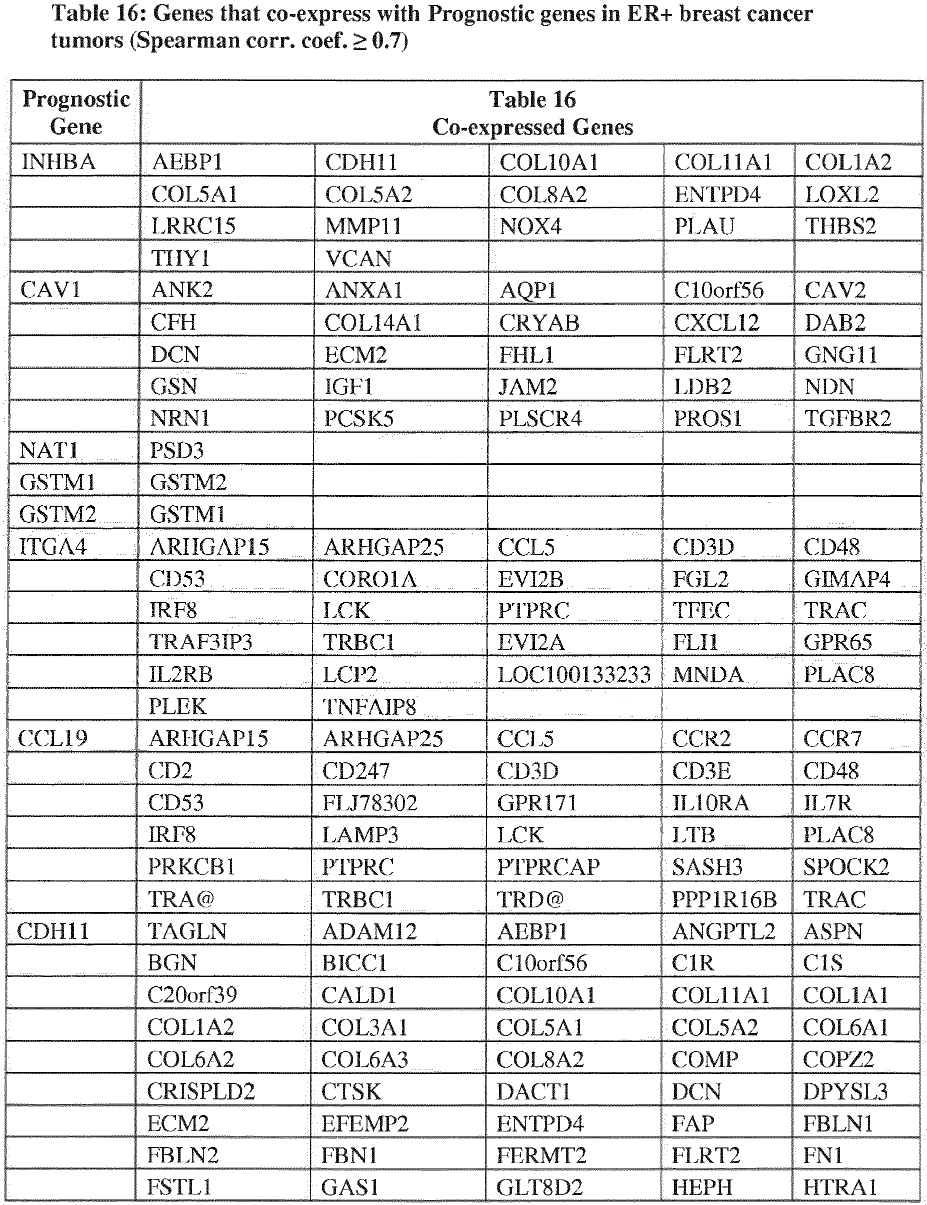

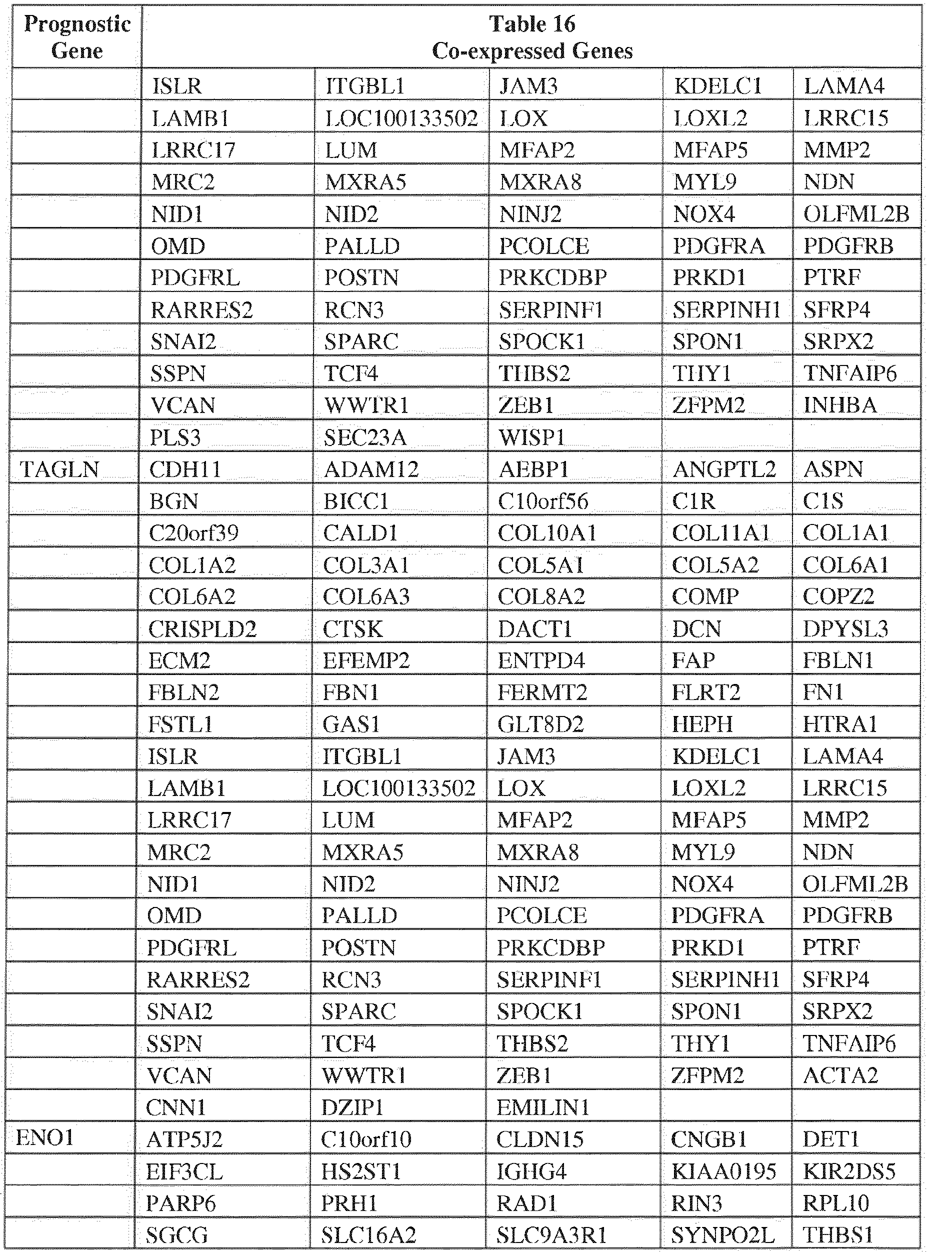

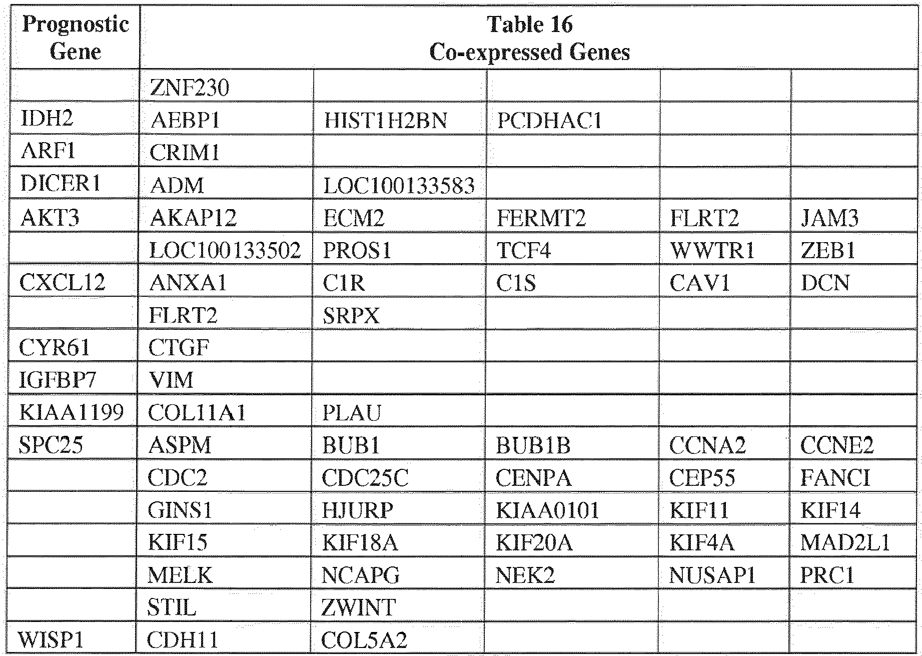

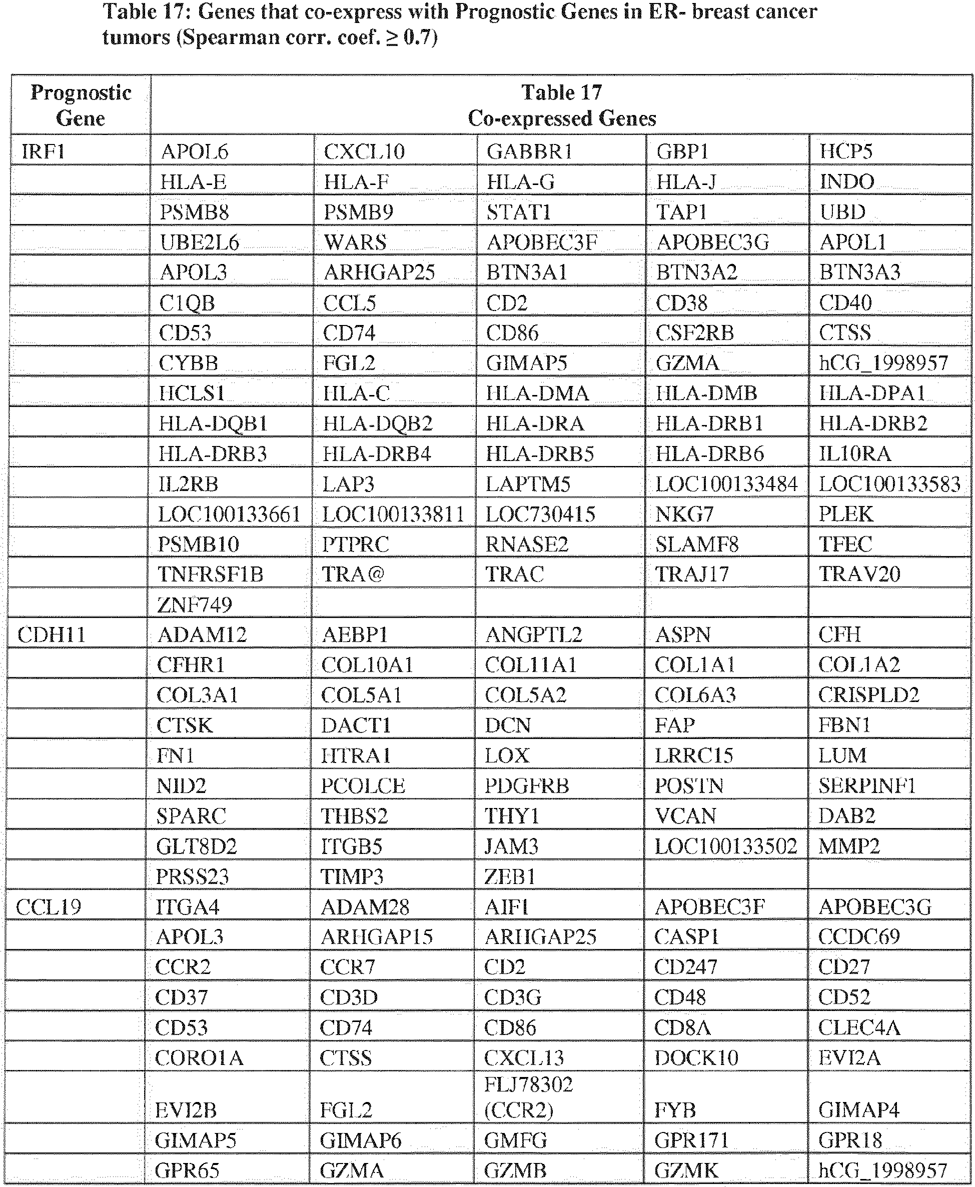

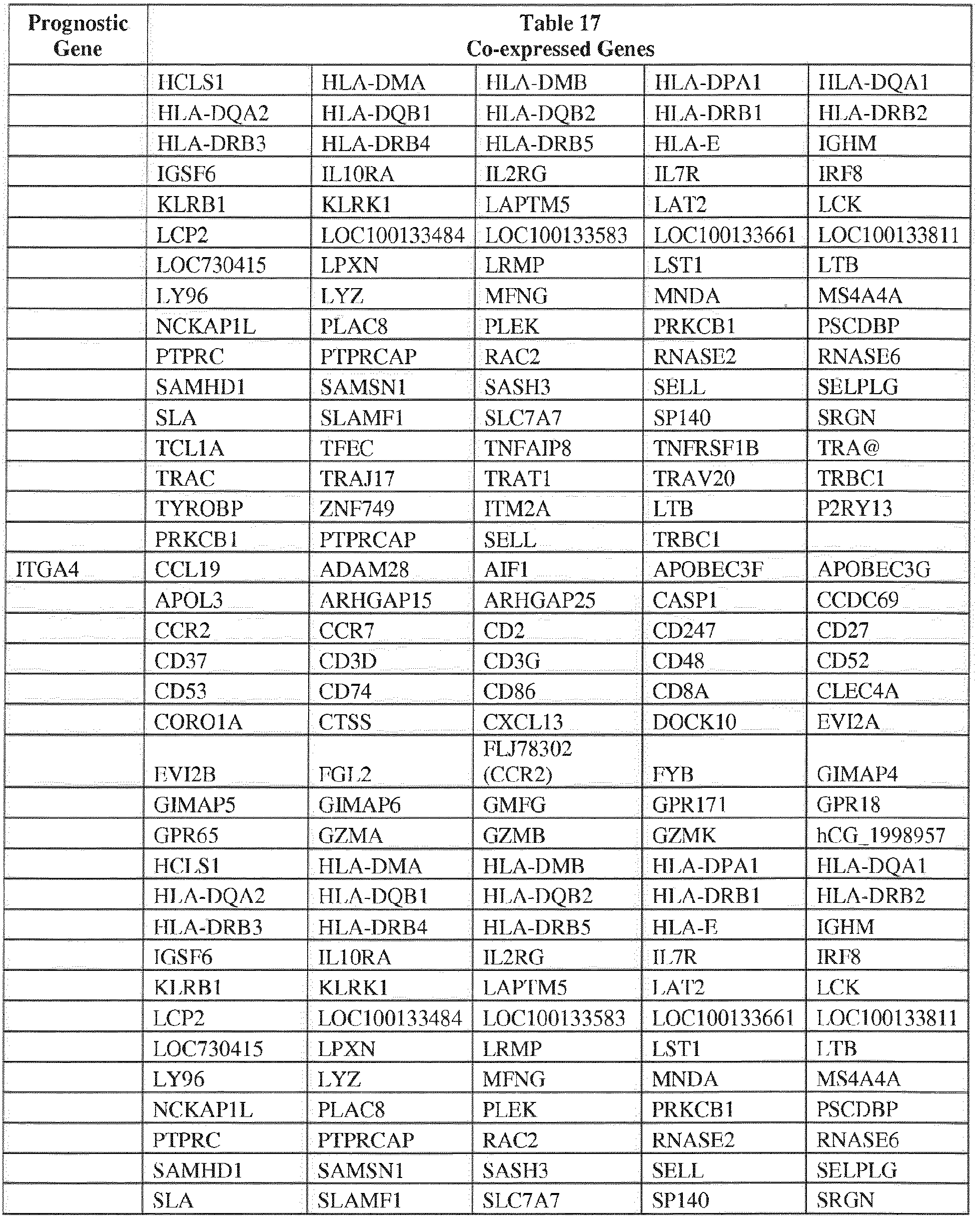

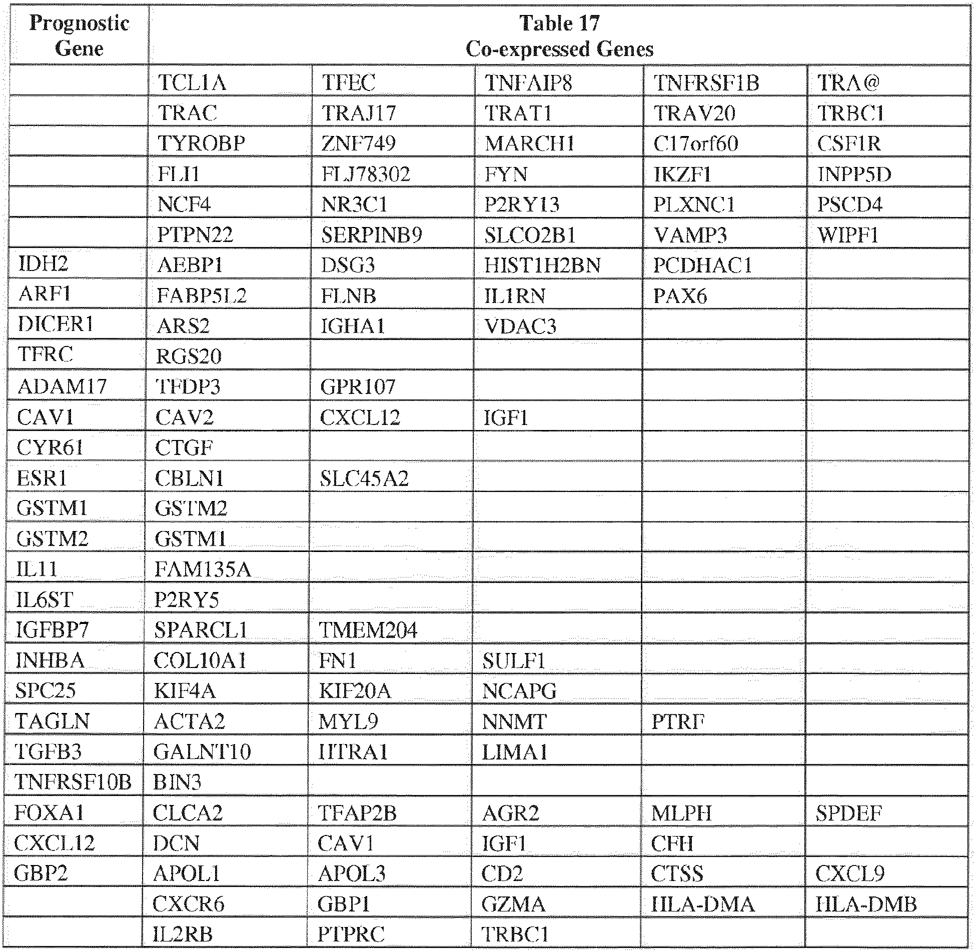

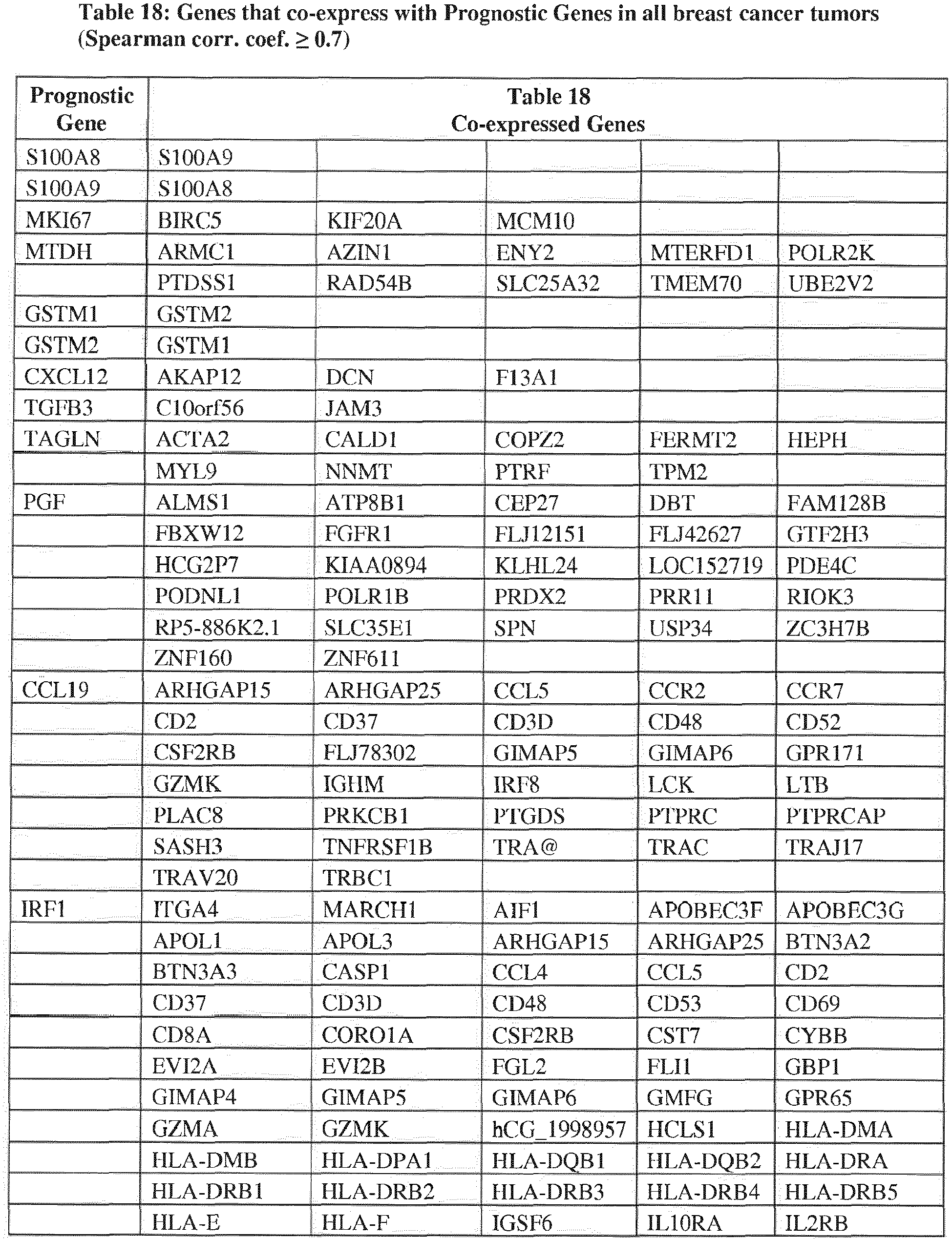

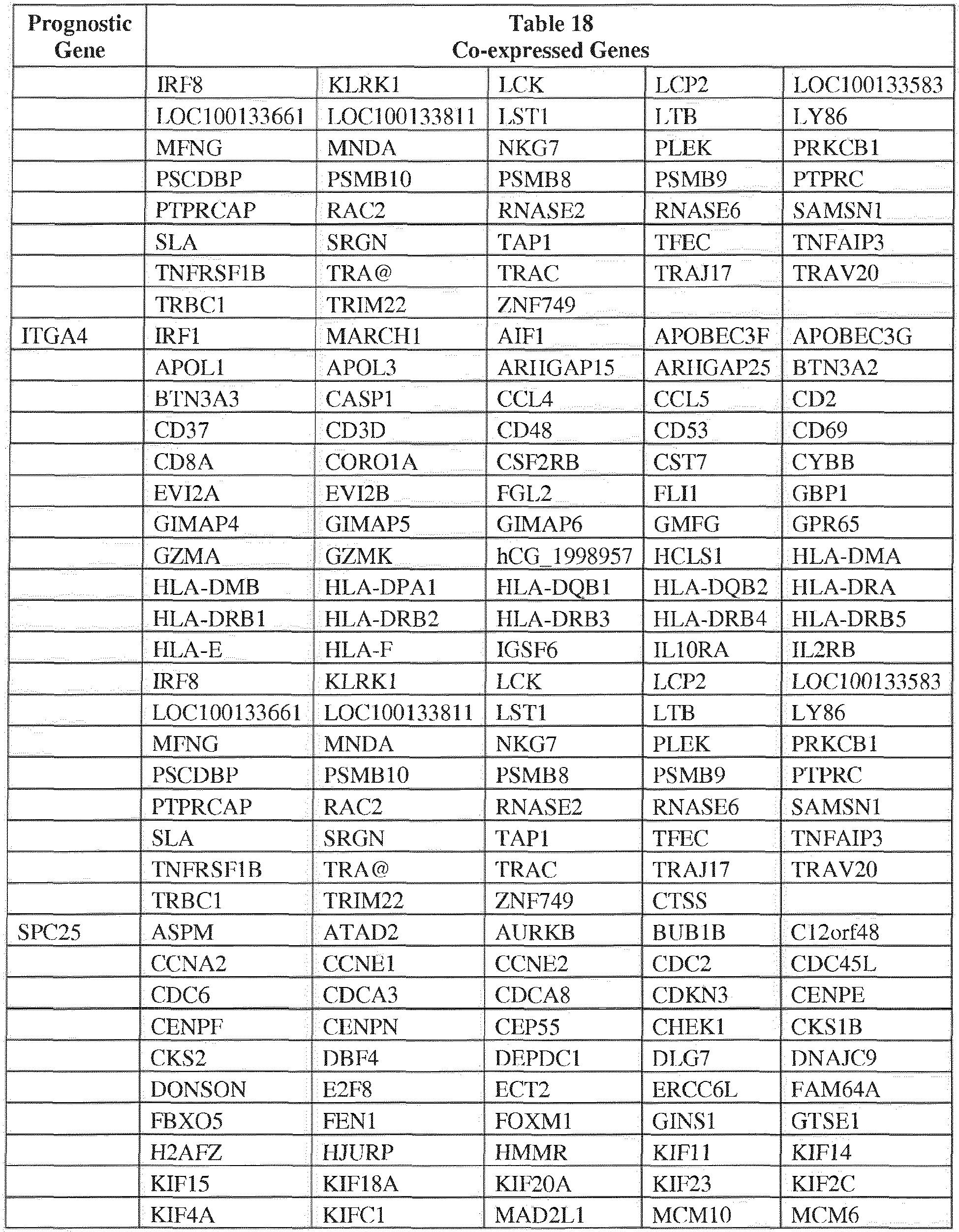

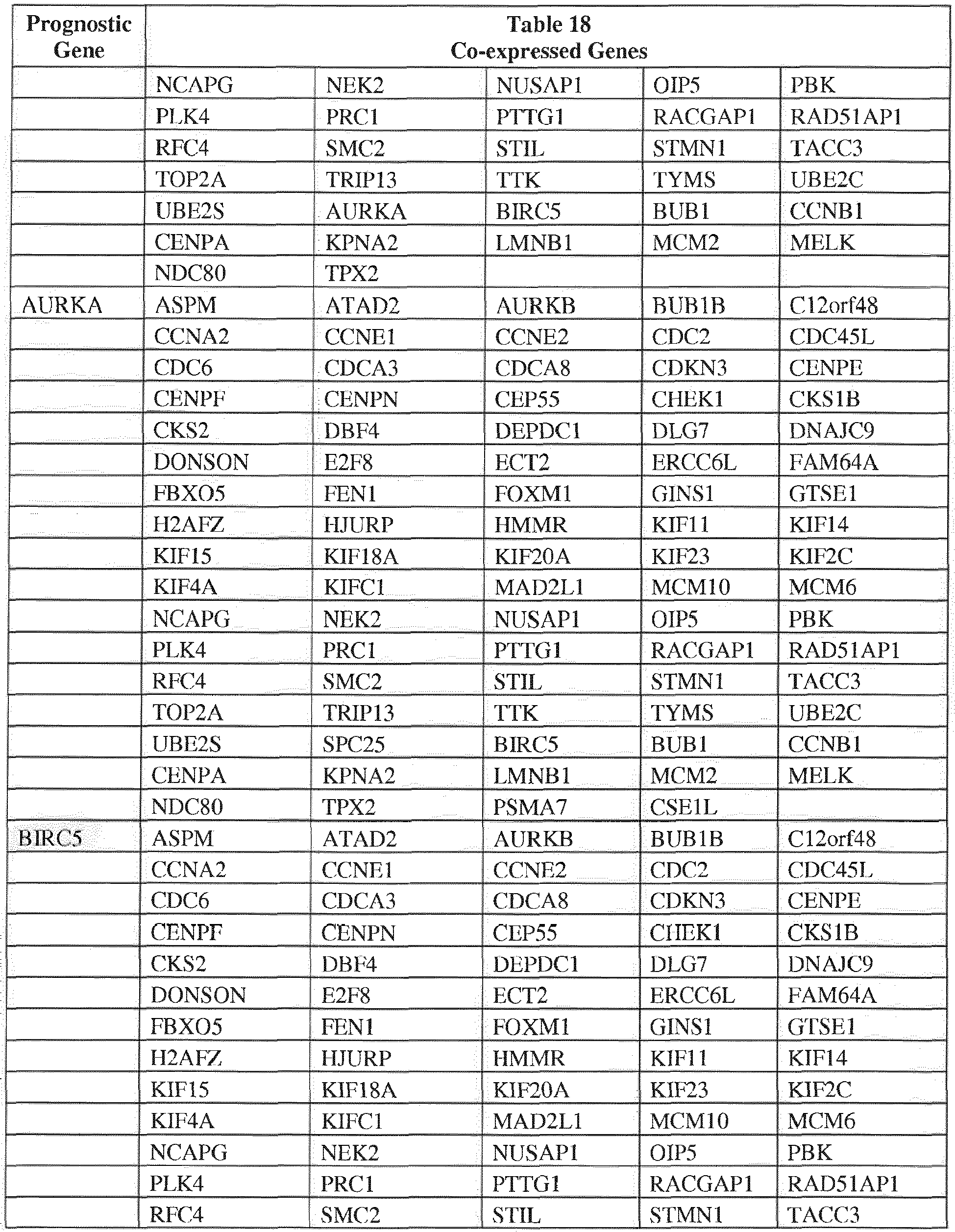

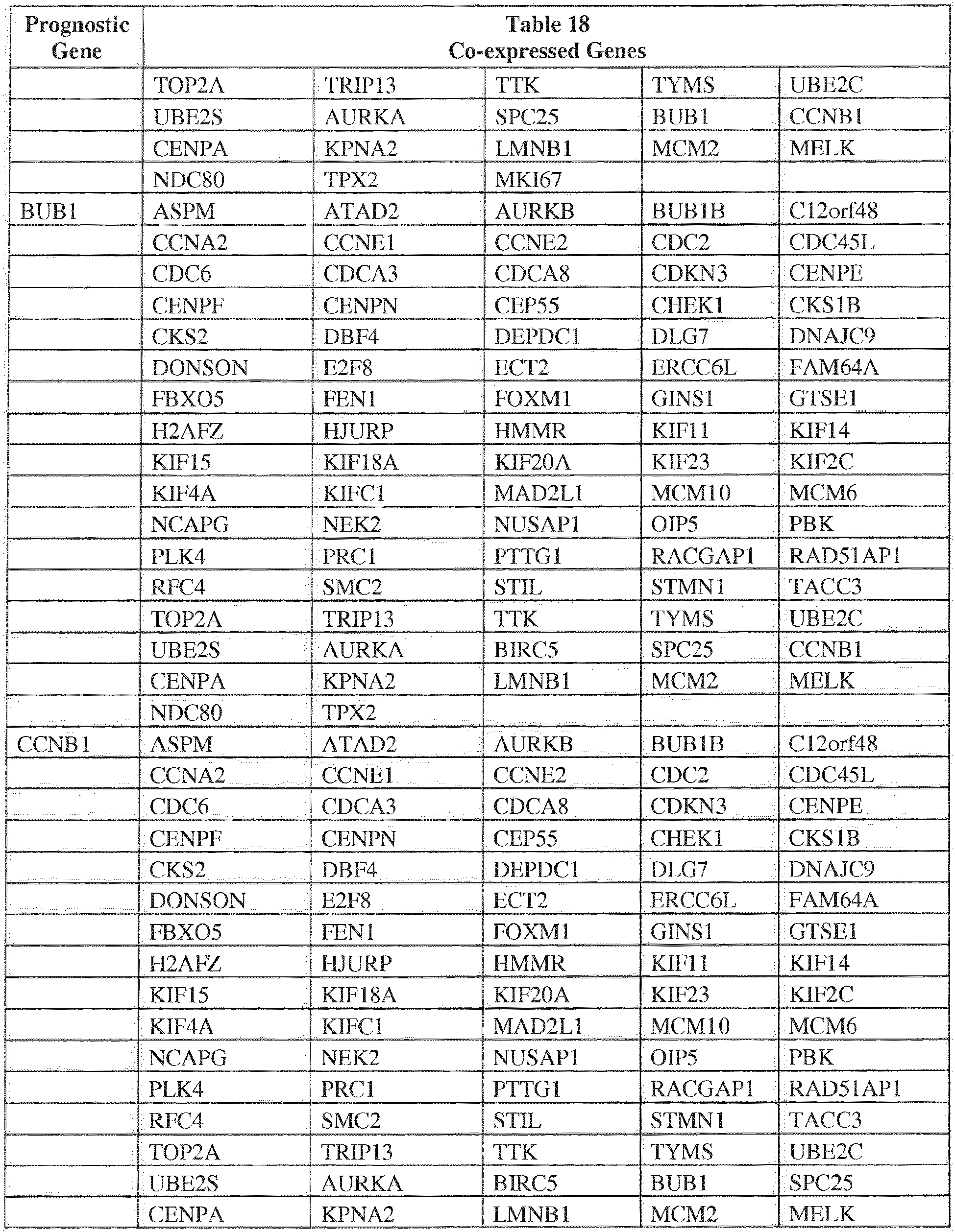

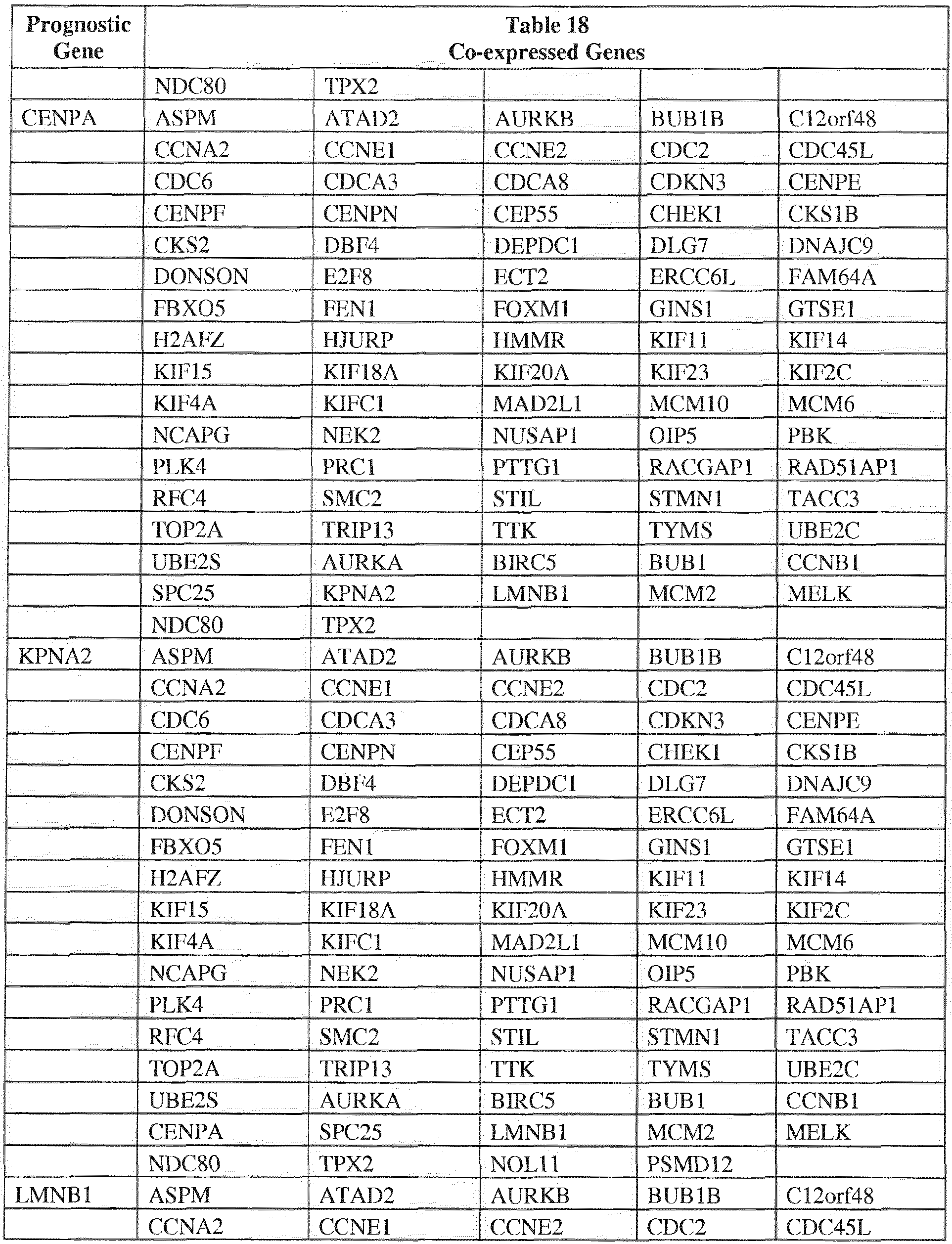

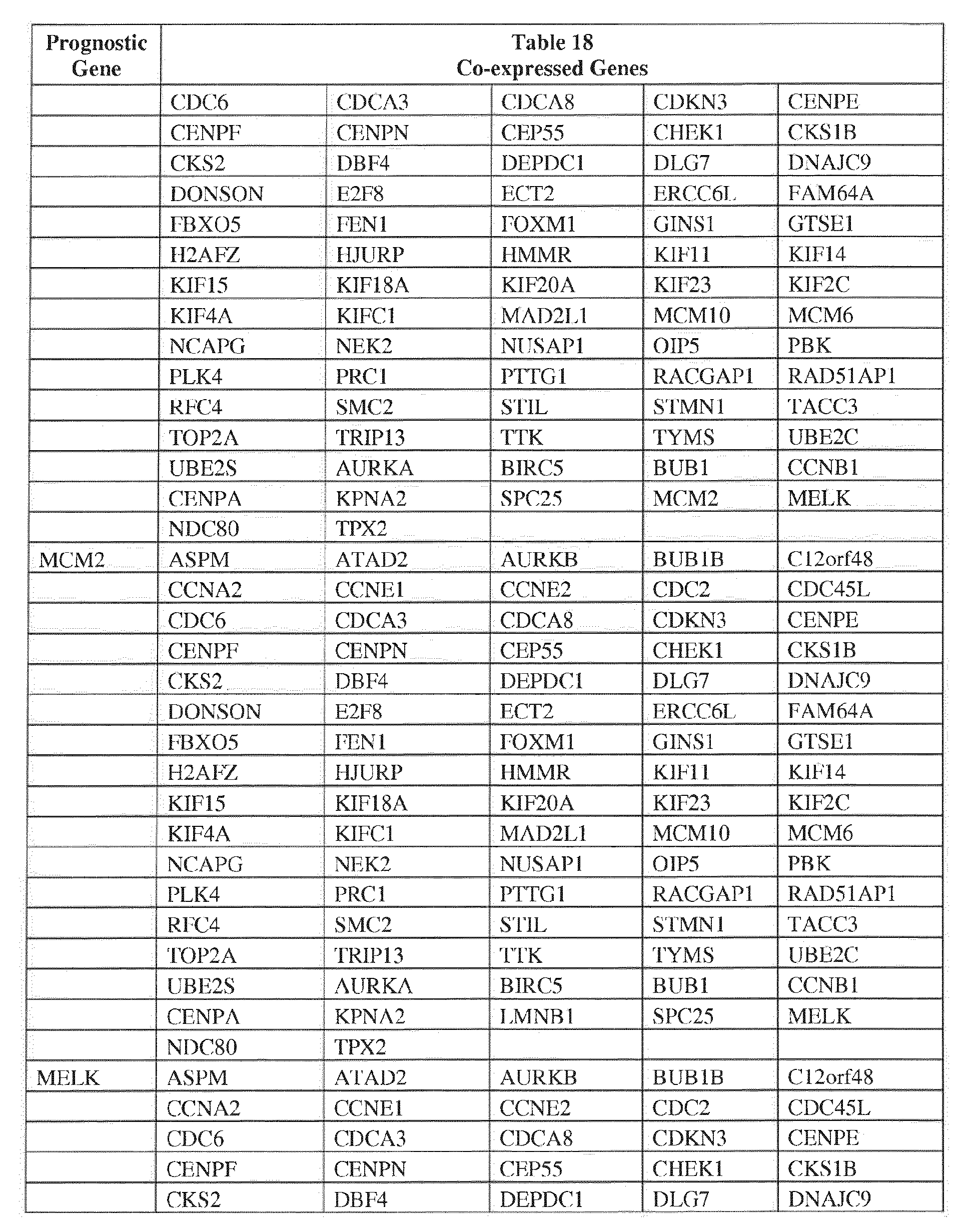

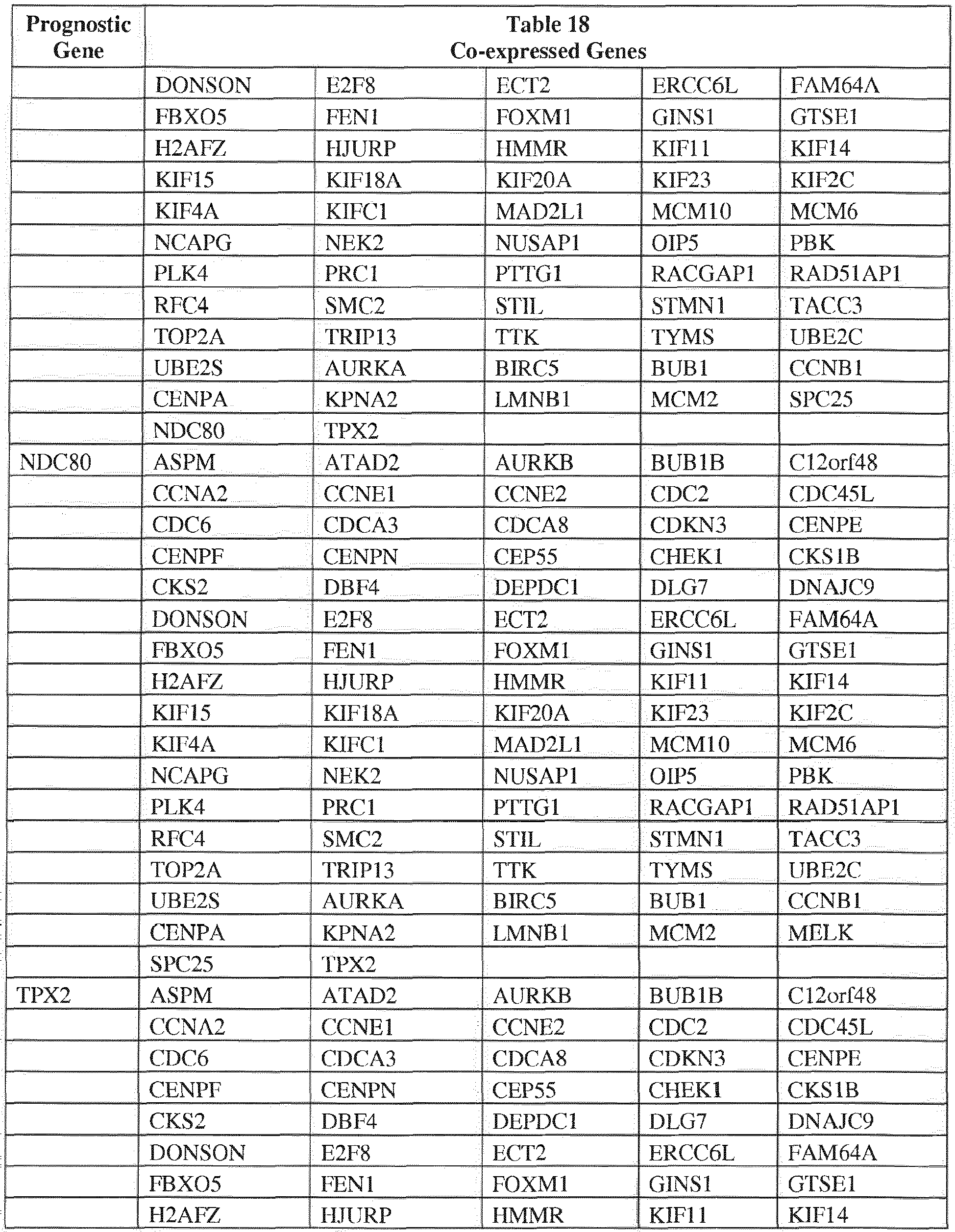

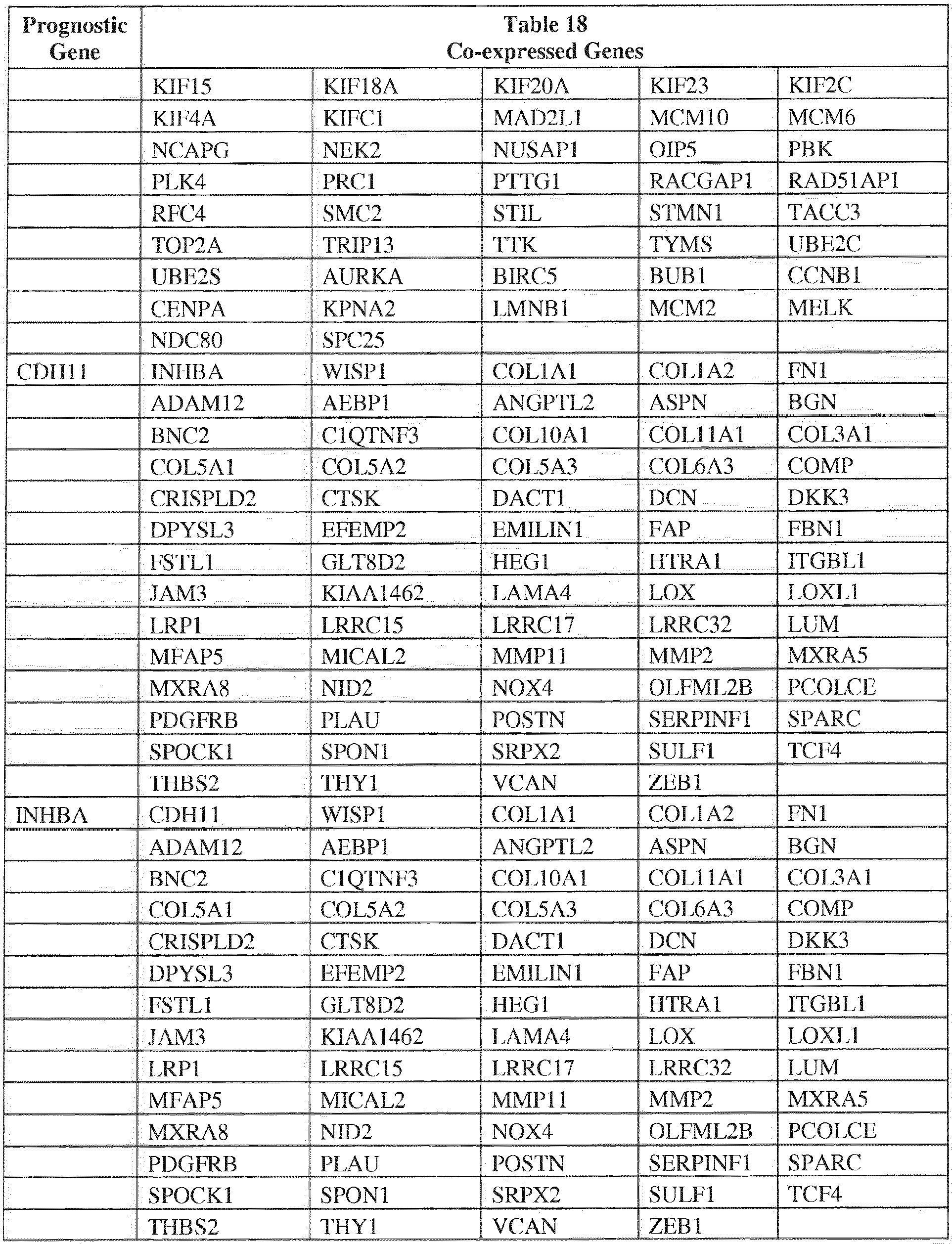

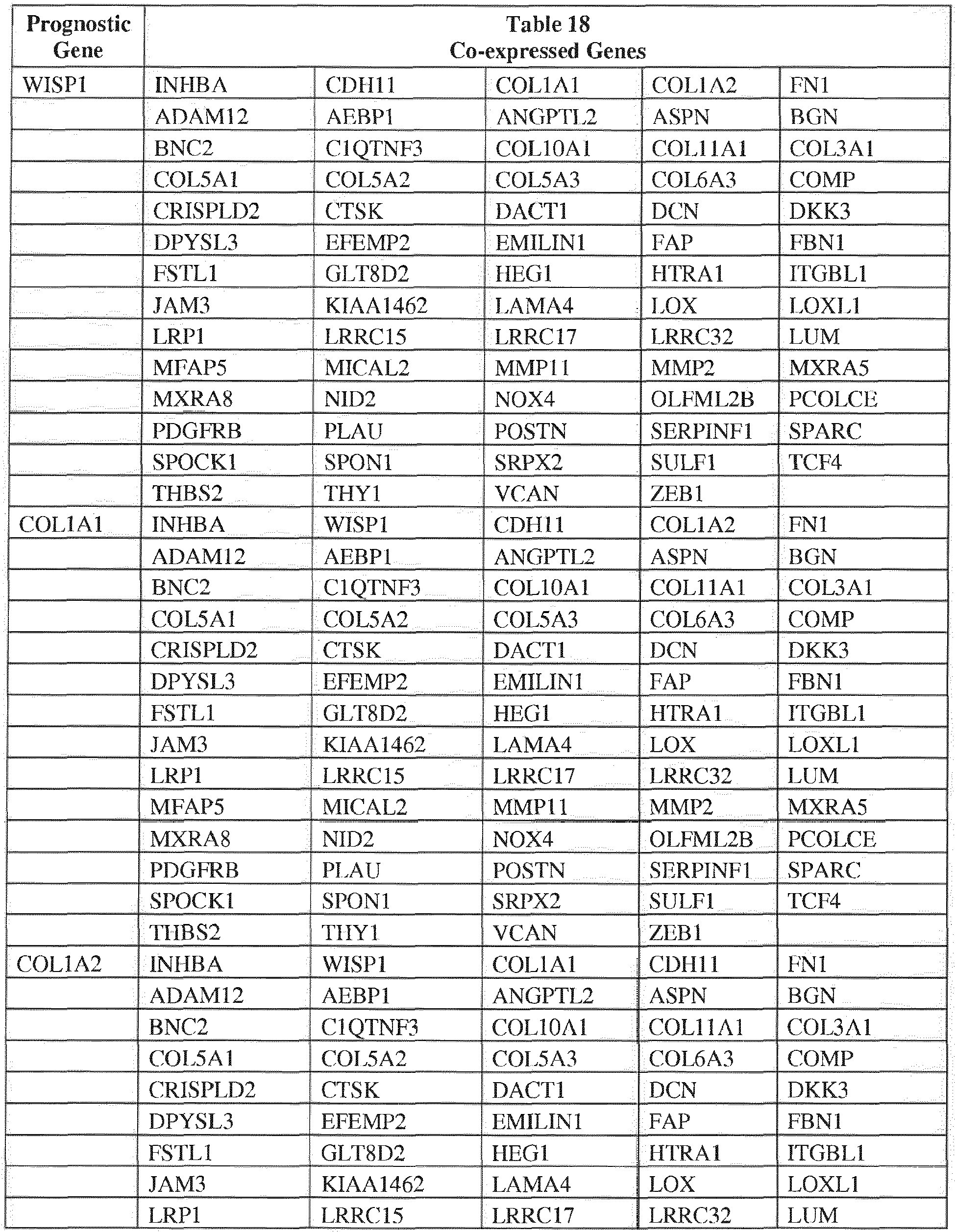

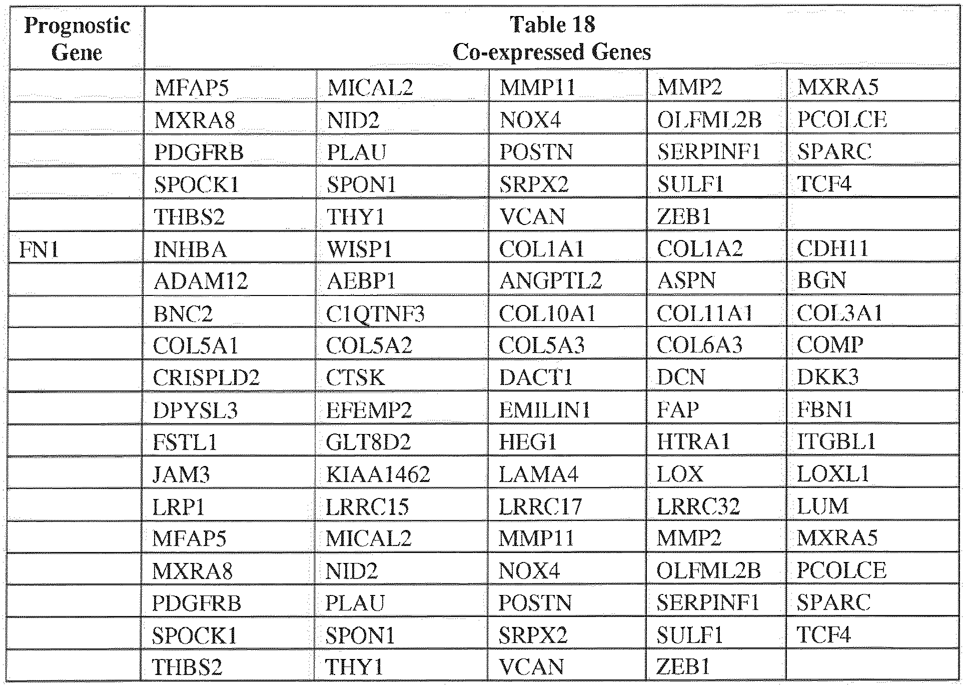

The present invention will now be described by way of reference to the following clauses:
1. A method for predicting the clinical outcome of a patient diagnosed with cancer comprising:
   (a) obtaining an expression level of an expression product of at least one prognostic gene from a tissue sample obtained from a tumor of the patient, wherein the at least one prognostic gene is selected from GSTM2, IL6ST, GSTM3, C8orf4, TNFRSF11B, NAT1, RUNX1, CSF1, ACTR2, LMNB1, TFRC, LAPTM4B, ENO1, CDC20, and IDH2, or a gene listed in Tables 1, 2, 7, or 8;
   (b) normalizing the expression level of the expression product of the at least one prognostics gene to obtain a normalized expression level; and
   (c) calculating a risk score based on the normalized expression value, wherein increased expression of a prognostic gene selected from GSTM2, IL6ST, GSTM3, C8orf4, TNFRSF11B, NAT1, RUNX1, and CSF1, or a prognostic gene listed in Tables 1 and 7, is positively correlated with good prognosis, and wherein increased expression of a prognostic gene selected from ACTR2, LMNB1, TFRC, LAPTM4B, ENO1, CDC20, and IDH2, or a prognostic gene in Tables 2 and 8, is negatively associated with good prognosis.
2. The method of clause 1, further comprising: generating a report based on the risk score.
3. The method of clause 1, wherein the patient is a human patient.
4. The method of clause 1, wherein the tumor is a breast cancer tumor.
5. The method of clause 1, wherein the tissue sample is a fixed paraffin-embedded tissue.
6. The method of clause 1, wherein the expression level is obtained using a PCR-based method.
7. The method of clause 1, wherein an expression level is obtained from at least two of the genes in any of the stromal, metabolic, immune, proliferation, or metabolic groups, or their gene products.
8. The method of clause 1, wherein an expression level is obtained from at least four genes in any two of the stromal, metabolic, immune, proliferation, or metabolic groups, or their gene products.
9. The method of clause 1, further comprising obtaining an expression level of at least one co-expressed gene from those listed in Table 18.
10. A method for predicting the clinical outcome of a patient diagnosed with estrogen receptor-negative (ER-) breast cancer comprising:
   (a) obtaining an expression level of an expression product of at least one prognostic gene listed in Tables 3, 4, 9 or 10 from a tissue sample obtained from a tumor of the patient, wherein the tumor is estrogen receptor negative;
   (b) normalizing the expression level of the expression product of the at least one prognostic gene to obtain a normalized expression level; and
   (c) calculating a risk score based on the normalized expression value, wherein increased expression of prognostic genes in Table 3 and Table 9 are positively correlated with good prognosis, and wherein increased expression of prognostic genes in Table 4 and Table 10 are negatively associated with good prognosis.
11. The method of clause 10, further comprising: generating a report based on the risk score.
12. The method of clause 10, wherein the patient is a human patient.
13. The method of clause 10, wherein the tumor is a breast cancer tumor is fixed paraffin-embedded tissue.
14. The method of clause 10, wherein the expression level is obtained using a PCR-based method.
15. The method of clause 10, wherein an expression level is obtained from at least two of the genes in any of the stromal, metabolic, immune, proliferation, or metabolic groups, or their gene products.
16. The method of clause 10, wherein an expression level is obtained from at least four genes in any two of the stromal, metabolic, immune, proliferation, or metabolic groups, or their gene products.
17. The method of clause 10, further comprising obtaining an expression level of at least one co-expressed gene from those listed in Table 17.
18. A computer program product for classifying a cancer patient according to prognosis, the computer program product for use in conjunction with a computer having a memory and a processor, the computer program product comprising a computer readable storage medium having a computer program encoded thereon, wherein said computer program product can be loaded into the one or more memory units of a computer and causes the one or more processor units of the computer to execute the steps of:
   (a) receiving a first data structure comprising the respective levels of an expression product of each of at least three different prognostic genes listed in any of Tables 1-12 in a tissue samples obtained from tumor in said patient;
   (b) normalizing said at least three expression values to obtain normalized expression values;
   (c) determining the similarity of the normalized expression values of each of said at least three prognostic genes to respective control levels of expression of the at least three prognostic genes obtained from a second data structure to obtain a patient similarity value, wherein the second data structure is based on levels of expression from a plurality of cancer tumors;
   (d) comparing said patient similarity value to a selected threshold value of similarity of said respective normalized expression values of each of said at least three prognostic genes to said respective control levels of expression of said at least three prognostic genes; and
   (e) classifying said patient as having a first prognosis if said patient similarity value exceeds said threshold similarity value, and a second prognosis if said patient similarity value does not exceed said threshold similarity value.

## Claims

1. A method for predicting the clinical outcome of a patient diagnosed with cancer comprising:
(a) obtaining an expression level of an expression product of a prognostic gene from a tissue sample obtained from a tumour of the patient, wherein the prognostic gene is UBE2C;
(b) normalizing the expression level of the expression product of the prognostics gene to obtain a normalized expression level;
(c) expressing the normalized expression level as an expression value compared to an expression level from a tumour reference set; and
(d) calculating a risk score based on the normalized expression value, wherein increased expression of UBE2C is negatively correlated with good prognosis.

2. A method as claimed in claim 1, further comprising: generating a report based on the risk score.

3. A method as claimed in claim 1, wherein the patient is a human patient.

4. A method as claimed in claim 1, wherein the tumour is a breast cancer tumour.

5. A method as claimed in claim 1, wherein the tissue sample is a fixed paraffin-embedded tissue.

6. A method as claimed in claim 1, wherein the expression level is obtained using a PCR-based method.

7. A method as claimed in claim 1, wherein an expression level is obtained from at least two genes in any of the stromal, metabolic, immune, proliferation, or metabolic groups, or their gene products.

8. A method as claimed in claim 1, wherein an expression level is obtained from at least four genes in any two of the stromal, metabolic, immune, proliferation, or metabolic groups, or their gene products.

9. A method as claimed in any preceding claim, further comprising obtaining an expression level of at least one co-expressed gene from those listed in Table 18.

10. A method as claimed in claim 9 wherein the co-expressed gene is BIRC5 (SURV), and wherein increased expression of UBE2C and BIRC5 (SURV) is negatively correlated with good prognosis.

11. A method for classifying a cancer patient according to prognosis, comprising the steps of:
(a) receiving a first data structure comprising the respective levels of an expression product of each of at least three different prognostic genes listed in any of Tables 1-12 in a tissue sample obtained from tumour in the patient, wherein one of the prognostic genes is UBE2C;
(b) normalizing the at least three expression values to obtain normalized expression values;
(c) determining the similarity of the normalized expression values of each of the at least three prognostic genes to respective control levels of expression of the at least three prognostic genes obtained from a second data structure to obtain a patient similarity value, wherein the second data structure is based on levels of expression from a plurality of cancer tumours;
(d) comparing the patient similarity value to a selected threshold value of similarity of the respective normalized expression values of each of the at least three prognostic genes to the respective control levels of expression of the at least three prognostic genes; and
(e) classifying the patient as having a first prognosis if the patient similarity value exceeds the threshold similarity value, and a second prognosis if the patient similarity value does not exceed the threshold similarity value.

12. A method as claimed in claim 11, wherein a second prognostic gene is BIRC5 (SURV).

13. A computer program comprising computer code means for performing steps (b) to (d) of a method as claimed in any of claims 1 to 12,
wherein the expression level of an expression product of a prognostic gene has been obtained from a tissue sample obtained from a tumour of the patient, wherein the prognostic gene is UBE2C,
wherein said program is run on a computer.

14. A computer program according to claim 13 wherein the expression levels of an expression product of at least two prognostic genes have been obtained from a tissue sample obtained from a tumour of the patient, wherein the at least two prognostic genes are UBE2C and BIRC5 (SURV).

15. A computer program as claimed in claim 14 embodied on a computer-readable medium.
